(19) Europäisches Patentamt
European Patent Office
Office européen des brevets

(11) EP 3 894 413 B1

(12) # EUROPEAN PATENT SPECIFICATION

(45) Date of publication and mention of the grant of the patent:
**15.11.2023 Bulletin 2023/46**

(21) Application number: **19897146.7**

(22) Date of filing: **06.12.2019**

(51) International Patent Classification (IPC):
***C07D 487/04*** (2006.01) ***A61K 31/519*** (2006.01)
***A61P 25/00*** (2006.01)

(52) Cooperative Patent Classification (CPC):
**A61P 25/00; A61P 9/00; C07D 487/04**

(86) International application number:
**PCT/US2019/064827**

(87) International publication number:
**WO 2020/123272 (18.06.2020 Gazette 2020/25)**

(54) **PYRAZOLOPYRIMIDINE PDE9 INHIBITORS**

PYRAZOLOPYRIMIDIN-PDE9-INHIBITOREN

INHIBITEURS PDE9 DE PYRAZOLOPYRIMIDINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.12.2018 US 201862778508 P**

(43) Date of publication of application:
**20.10.2021 Bulletin 2021/42**

(73) Proprietor: **Merck Sharp & Dohme LLC**
**Rahway, New Jersey 07065 (US)**

(72) Inventors:
- **ARASAPPAN, Ashok**
  **Kenilworth, New Jersey 07033 (US)**
- **COX, Jason, M.**
  **Kenilworth, New Jersey 07033 (US)**
- **DEBENHAM, John, S.**
  **Kenilworth, New Jersey 07033 (US)**
- **FENG, Zhe**
  **Kenilworth, New Jersey 07033 (US)**
- **GUO, Zhuyan**
  **Kenilworth, New Jersey 07033 (US)**
- **MENG, Dongfang**
  **Kenilworth, New Jersey 07033 (US)**

(74) Representative: **Merck Sharp & Dohme LLC**
**120 Moorgate**
**London EC2M 6UR (GB)**

(56) References cited:
**WO-A1-2016/196071 US-A1- 2018 134 712**

- **PETRIE et al.: "Synthesis and biological activity of 6-azacadeguomycin and certain 3,4,6-trisubstituted pyrazolo[3,4-d]pyrimidine ribonucleosides", Journal of Medicinal Chemistry, vol. 28, no. 8, 1985, XP002421202, DOI: 10.1021/jm00146a007**
- **BERMEJO et al.: "PAMPA-a drug absorption in vitro model 7. Comparing rat in situ, Caco-2, and PAMPA permeability of fluoroquinolones", European Journal of Pharmaceutical Sciences, vol. 21, 2004, pages 429-441, XP055657106,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 894 413 B1

## Description

### BACKGROUND OF THE INVENTION

The phosphodiesterases enzyme family hydrolyzes the cyclic nucleotides cyclic adenosine monophosphate (cAMP) and cyclic guanosine monophosphate (cGMP). cGMP and cAMP are central to the control and regulation of a multitude of cellular events, both physiological and pathophysiological. One pathway for affecting the levels of cyclic nucleotides, such as cAMP and cGMP, is to alter or regulate the enzymes that degrade these enzymes, known as 3', 5'-cyclic nucleotide specific phosphodiesterases (PDEs). The PDE superfamily includes twenty one genes that encode for eleven families of PDEs. These families are further subdivided based on catalytic domain homology and substrate specificity and include the: (1) cAM112 P specific, PDE4A-D, 7A and 7B, and 8A and 8B; (2) cGMP specific, PDE 5A, 6A-C, and 9A; and (3) those that are dual substrate, PDE 1A-C, 2A, 3A and 3B, 10A, and 11A. The homology between the families, ranging from 20% to 45% suggests that it may be possible to develop selective inhibitors for each of these subtypes.

The identification of the phosphodiesterase PDE9 has been reported and was distinguished from other PDEs on the basis of its amino acid sequence, functional properties, and tissue distribution. PDE9V is encoded by two genes (PDE9A and PDE9B) and is cGMP specific. At least 20 different splice variants have been discovered (PDE9A1-PD9A20) in human and in mouse. Structural study of PDE9A have been shown that its cDNA of the different splice variants share a high percentage of amino acid identity in the catalytic domain. However, despite its highest specificity for cGMP among all the PDEs, PDE9A lacks a GAF domain, whose binding of cGMP usually activates catalytic activity. Besides its expression in the kidney, spleen, and other peripheral organs, PDE9 is widespread through the brain in mice, rats and humans, and has been found to be present in a variety of human tissues, including the testes, small intestine, skeletal muscle, smooth muscle in the vasculature, heart, lung, and thymus.

Inhibition of PDE9 is believed to be useful in the treatment of cardiovascular and cerebrovascular diseases, such as hypertension and heart failure, and cognitive deficit associated with neurodegenerative and psychiatric disorders and a variety of conditions or disorders that would benefit from increasing levels of cGMP within neurons, including including Alzheimer's disease, schizophrenia, and depression.

US 2018/134712 A1 and WO2016/196071 A1 each disclose 6-alkyl dihydropyrazolopyrimidinone compounds which are useful as therapeutic agents for the treatment of central nervous system disorders associated with phosphodiesterase 2 (PDE2).

### SUMMARY OF THE INVENTION

The present invention is directed to amino and alkyl pyrazolopyrimidine compounds which may be useful as therapeutic agents for the treatment of disorders associated with phosphodiesterase 9 (PDE9). The present invention also relates to the use of such compounds for treating cardiovascular and cerebrovascular diseases, such as hypertension and heart failure, and neurological and psychiatric disorders, such as schizophrenia, psychosis or Huntington's disease, and those associated with striatal hypofunction or basal ganglia dysfunction.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

### DETAILED DESCRIPTION OF THE INVENTION

The present invention is directed to compounds of the formula I:

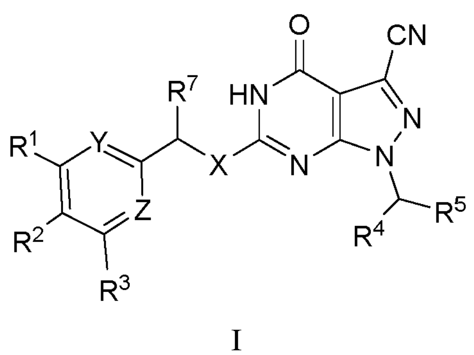

I

wherein:

X is -N(R6)- or -C($R^8R^9$)-;
Y is -N= or -CH=;
Z is -N= or -CH=;
$R^1$, $R^2$ and $R^3$ are independently selected from:

(1) hydrogen,
(2) halogen,
(3) hydroxyl,
(4) $C_{1-6}$alkyl, which is unsubstituted or substituted with substituents selected from: hydroxy and fluoro,
(5) -O-$C_{1-6}$alkyl, which is unsubstituted or substituted with substituents selected from fluoro,
(6) $C_{3-6}$cycloalkyl,
(7) $C_{2-6}$alkynyl, and
(8) -CN;

$R^4$ is selected from:

(1) hydrogen,
(2) $-CH_3$;
(3) $-CF_3$,
(4) $-CH_2OH$,
(5) $-CO_2H$, and
(6) $-CH_2CH_3$;

$R^5$ is a phenyl, pyridyl, pyrazinyl, pyrazolyl, pyrimidinyl, pyridazinyl, thiazolyl, cyclohexyl or tetrahydropyranyl ring, wherein the phenyl, pyridyl, pyrazinyl, pyrazolyl, pyrimidinyl, pyridazinyl, thiazolyl, cyclohexyl or tetrahydropyranyl ring is substituted with $R^{1a}$, $R^{1b}$ and $R^{1c}$, wherein $R^{1a}$, $R^{1b}$ and $R^{1c}$ are independently selected from:

(1) hydrogen,
(2) halogen,
(3) hydroxyl,
(4) $C_{1-6}$alkyl, which is unsubstituted or substituted with substituents selected from: hydroxy and fluoro,
(5) -O-$C_{1-6}$alkyl, which is unsubstituted or substituted with substituents selected from fluoro,
(6) $C_{3-6}$cycloalkyl,
(7) azetidine, morpholine, piperidine, or pyrrolidine, and
(8) -CN;

$R^6$ is selected from:

(1) hydrogen, and
(2) $C_{1-6}$alkyl;

R7 is selected from:

(1) hydrogen,
(2) $C_{1-6}$alkyl, which is unsubstituted or substituted with substituents selected from: hydroxy and fluoro,
(3) $C_{2-6}$alkenyl,
(4) $C_{3-6}$cycloalkyl, which is unsubstituted or substituted with substituents selected from: hydroxy and fluoro, and
(5) azetidine, morpholine, piperidine, or pyrrolidine;
or $R^6$ and $R^7$ and the -CH-N- to which they are attached are joined to form an azetidine or a pyrrolidine ring;

$R^8$ and $R^9$ are independently selected from:

(1) hydrogen, and
(2) $C_{1-6}$alkyl;

or a pharmaceutically acceptable salt thereof.

An embodiment of the present invention includes compounds of the formula Ia:

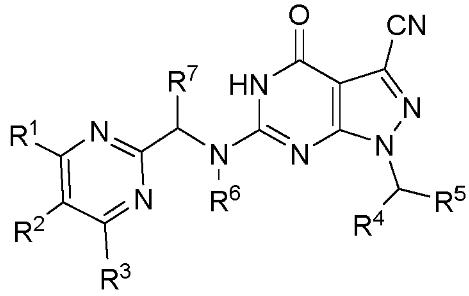

Ia

wherein $R^1$, $R^2$, $R^3$, $R^4$, $R^5$, $R^6$ and $R^7$ are defined herein; or a pharmaceutically acceptable salt thereof.

An embodiment of the present invention includes compounds of the formula Ib:

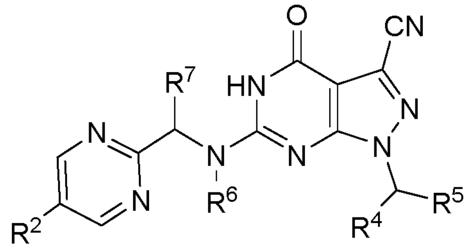

Ib

wherein $R^2$, $R^4$, $R^5$, $R^6$ and $R^7$ are defined herein; or a pharmaceutically acceptable salt thereof.

An embodiment of the present invention includes compounds of the formula Ic:

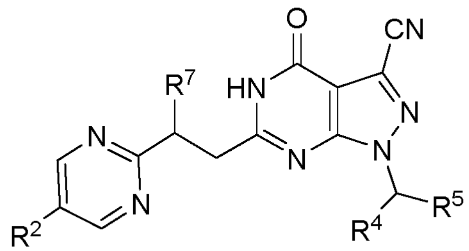

Ic

wherein $R^2$, $R^4$, $R^5$ and $R^7$ are defined herein; or a pharmaceutically acceptable salt thereof.

An embodiment of the present invention includes compounds wherein X is $-N(R^6)-$. An embodiment of the present invention includes compounds wherein X is $-C(R^8R^9)-$. An embodiment of the present invention includes compounds wherein X is $-CH_2-$.

An embodiment of the present invention includes compounds wherein $R^1$, $R^2$ and $R^3$ are independently selected from:

(1) hydrogen,
(2) halogen,
(3) hydroxyl,
(4) $C_{1-6}$alkyl, which is unsubstituted or substituted with substituents selected from: hydroxy and fluoro,
(5) $-O-C_{1-6}$alkyl, which is unsubstituted or substituted with substituents selected from fluoro,
(6) $C_{3-6}$cycloalkyl,

(7) $C_{2-4}$alkynyl, and
(8) -CN.

An embodiment of the present invention includes compounds wherein $R^1$, $R^2$ and $R^3$ are independently selected from:

(1) hydrogen,
(2) fluoro,
(3) chloro,
(4) hydroxyl,
(5) $C_{1-2}$alkyl, which is unsubstituted or substituted with one or more fluoro,
(6) -O-$C_{1-2}$alkyl, which is unsubstituted or substituted with one or more fluoro,
(7) $C_{3-5}$cycloalkyl, and
(8) $C_{2-3}$alkynyl.

An embodiment of the present invention includes compounds wherein $R^1$ is hydrogen and $R^3$ is hydrogen.
An embodiment of the present invention includes compounds wherein $R^1$ is hydrogen, $R^3$ is hydrogen and $R^2$ is selected from:

(1) hydrogen,
(2) fluoro,
(3) chloro,
(4) hydroxyl,
(5) $C_{1-2}$alkyl, which is unsubstituted or substituted with one or more fluoro,
(6) -O-$C_{1-2}$alkyl, which is unsubstituted or substituted with one or more fluoro, and
(7) $C_{3-5}$cycloalkyl, and
(8) $C_{2-3}$alkynyl.

An embodiment of the present invention includes compounds wherein $R^1$ is hydrogen, $R^3$ is hydrogen and $R^2$ is selected from:

(1) hydrogen,
(2) fluoro,
(3) chloro,
(4) bromo,
(5) hydroxyl,
(6) $-CH_3$,
(7) $-OCH_3$,
(8) $-CHF_2$,
(9) $-CF_3$,
(10) $-OCHF_2$,
(11) $-OCF_3$,
(12) $-CH_2CH_3$,
(13) $-CH(CH_3)_2$,
(14) $-C(CH_3)_3$,
(15) $-C\equiv CH$, and
(16) cyclopropyl.

An embodiment of the present invention includes compounds wherein $R^1$ is hydrogen, $R^3$ is hydrogen and $R^2$ is selected from:

(1) hydrogen,
(2) fluoro,
(3) $-CHF_2$,
(4) $-CF_3$,
(5) $-OCHF_2$, and
(6) $-OCF_3$.

An embodiment of the present invention includes compounds wherein $R^4$ is hydrogen. An embodiment of the present invention includes compounds wherein $R^4$ is $-CH_3$.

An embodiment of the present invention includes compounds wherein $R^5$ is a phenyl, pyridyl, pyrazinyl, pyrazolyl, pyrimidinyl, pyridazinyl or thiazolyl ring, wherein the phenyl, pyridyl, pyrazinyl, pyrazolyl, pyrimidinyl, pyridazinyl or thiazolyl ring is substituted with $R^{1a}$, $R^{1b}$ and $R^{1c}$, wherein $R^{1a}$, $R^{1b}$ and $R^{1c}$ are independently selected from:

(1) hydrogen,
(2) halogen,
(3) hydroxyl,
(4) $C_{1-6}$alkyl, which is unsubstituted or substituted with substituents selected from: hydroxy and fluoro,
(5) $-O-C_{1-6}$alkyl, which is unsubstituted or substituted with substituents selected from fluoro,
(6) $C_{3-6}$cycloalkyl, and
(7) -CN.

An embodiment of the present invention includes compounds wherein $R^5$ is a phenyl, pyridyl, pyrazinyl, pyrazolyl, pyrimidinyl, pyridazinyl or thiazolyl ring, wherein the phenyl, pyridyl, pyrazinyl, pyrazolyl, pyrimidinyl, pyridazinyl or thiazolyl ring is substituted with $R^{1a}$, $R^{1b}$ and $R^{1c}$, wherein $R^{1a}$, $R^{1b}$ and $R^{1c}$ are independently selected from:

(1) hydrogen,
(2) fluoro,
(3) $C_{1-2}$alkyl, which is unsubstituted or substituted with one or more fluoro,
(4) $-O-C_{1-2}$alkyl, which is unsubstituted or substituted with one or more fluoro,
(5) cyclopropyl, and
(6) -CN.

An embodiment of the present invention includes compounds wherein $R^5$ is a phenyl, pyridyl, pyrazinyl, pyrazolyl, pyrimidinyl, pyridazinyl or thiazolyl ring, wherein the phenyl, pyridyl, pyrazinyl, pyrazolyl, pyrimidinyl, pyridazinyl or thiazolyl ring is substituted with $R^{1a}$, $R^{1b}$ and $R^{1c}$, wherein $R^{1a}$, $R^{1b}$ and $R^{1c}$ are independently selected from:

(1) hydrogen,
(2) fluoro,
(3) $-CH_3$,
(4) $-CHF_2$,
(5) $-CF_3$,
(6) $-OCHF_2$,
(7) $-OCF_3$,
(8) $-OCHF_2$,
(9) $-OCF_3$,
(10) cyclopropyl, and
(11) -CN.

An embodiment of the present invention includes compounds wherein $R^5$ is a phenyl, pyridyl or tetrahydropyranyl ring. An embodiment of the present invention includes compounds wherein $R^5$ is a phenyl ring. An embodiment of the present invention includes compounds wherein $R^5$ is a pyridyl ring. An embodiment of the present invention includes compounds wherein $R^5$ is a tetrahydropyranyl ring.

An embodiment of the present invention includes compounds $R^{1b}$ is hydrogen and $R^{1c}$ is hydrogen, and $R^{1a}$ is selected from:

(1) hydrogen,
(2) fluoro,
(3) $-CH_3$,
(4) $-CHF_2$,
(5) $-CF_3$,
(6) $-OCHF_2$,
(7) $-OCF_3$,
(8) cyclopropyl, and
(9) -CN.

An embodiment of the present invention includes compounds wherein $R^5$ is selected from:

(1) 3-(trifluoromethyl)phenyl,
(2) tetrahydropyran-4-yl,
(3) 6-(cyclopropyl)pyridin-3-yl, and
(4) 6-(trifluoromethyl)pyridin-3-yl.

An embodiment of the present invention includes compounds wherein $R^6$ is hydrogen. An embodiment of the present invention includes compounds wherein $R^6$ is methyl. An embodiment of the present invention includes compounds wherein R6 is ethyl.

An embodiment of the present invention includes compounds wherein $R^7$ is hydrgen. An embodiment of the present invention includes compounds wherein $R^7$ is methyl. An embodiment of the present invention includes compounds wherein $R^7$ is ethyl. An embodiment of the present invention includes compounds wherein $R^7$ is isopropyl. An embodiment of the present invention includes compounds wherein $R^7$ is cyclopropyl. An embodiment of the present invention includes compounds wherein $R^7$ is cyclobutyl. An embodiment of the present invention includes compounds wherein $R^7$ is 3-butenyl.

An embodiment of the present invention includes compounds wherein $R^8$ and $R^9$ are each hydrogen.

An embodiment of the present invention includes a compound which is selected from:

1-(1-(6-cyclopropylpyridin-3-yl)ethyl)-6-(methyl(1-(pyrimidin-2-yl)propyl)amino)-4-oxo-4,5-dihydro-1H-pyrazolo[3,4-d]pyrimidine-3 -carbonitrile;
6-[methyl(1-pyrimidin-2-ylethyl)amino]-4-oxo-1-[1-[6-(trifluoromethyl)-3-pyridyl]ethyl]-5H-pyrazolo[3,4-d]pyrimidine-3-carbonitrile;
6-[methyl(1-pyrimidin-2-ylpropyl)amino]-4-oxo-1-[1-[6-(trifluoromethyl)-3-pyridyl]ethyl]-5H-pyrazolo[3,4-d]pyrimidine-3-carbonitrile;
6-[methyl(1-pyrimidin-2-ylpropyl)amino]-4-oxo-1-[1 -[4-(trifluoromethyl)phenyl] ethyl]-5H-pyrazolo[3,4-d]pyrimidine-3-carbonitrile;
6-[1-[5-(difluoromethyl)pyrimidin-2-yl]propyl-methyl-amino]-4-oxo-1-[1-[6-(trifluoromethyl)-3-pyridyl]ethyl]-5H-pyrazolo[3,4-d]pyrimidine-3-carbonitrile;
6-[methyl-(2-methyl-1-pyrimidin-2-yl-propyl)amino]-4-oxo-1-[1-[6-(trifluoromethyl)-3-pyridyl]ethyl]-5H-pyrazolo[3,4-d]pyrimidine-3-carbonitrile;
6-[[cyclopropyl(pyrimidin-2-yl)methyl]-methyl-amino]-4-oxo-1-[1-[6-(trifluoromethyl)-3-pyridyl]ethyl]-5H-pyrazolo[3,4-d]pyrimidine-3-carbonitrile;
6-[[cyclobutyl(pyrimidin-2-yl)methyl]-methyl-amino]-4-oxo-1-[1-[6-(trifluoromethyl)-3-pyridyl]ethyl]-5H-pyrazolo[3,4-d]pyrimidine-3-carbonitrile;
6-[1-(5-fluoro-2-pyridyl)propyl-methyl-amino]-4-oxo-1-[1-[6-(trifluoromethyl)-3-pyridyl]ethyl]-SH-pyrazolo[3,4-d]pyrimidine-3-carbonitrile;
6-[1-(5-fluoropyrimidin-2-yl)propyl-methyl-amino]-4-oxo-1-(1-tetrahydropyran-4-ylethyl)-5H-pyrazolo[3,4-d]pyrimidine-3-carbonitrile;
6-[methyl(1-pyrimidin-2-ylpent-4-enyl)amino]-4-oxo-1-[1-[6-(trifluoromethyl)-3-pyridyl]ethyl]-5H-pyrazolo[3,4-d]pyrimidine-3-carbonitrile;
4-oxo-6-(2-pyrimidin-2-ylazetidin-1-yl)-1-[1-[6-(trifluoromethyl)-3-pyridyl]ethyl]-5H-pyrazolo[3,4-d]pyrimidine-3 -carbonitrile;
6-[2-(4-fluorophenyl)azetidin-1-yl]-4-oxo-1-[1-[6-(trifluoromethyl)-3-pyridyl]ethyl]-5H-pyrazolo[3,4-d]pyrimidine-3-carbonitrile;
4-oxo-6-(2-pyrimidin-2-ylpyrrolidin-1-yl)-1-[1-[6-(trifluoromethyl)-3-pyridyl]ethyl]-5H-pyrazolo[3,4-d]pyrimidine-3-carbonitrile;
6-[2-(4-fluorophenyl)azetidin-1-yl]-4-oxo-1-[1-[6-(trifluoromethyl)-3-pyridyl]ethyl]-5H-pyrazolo[3,4-d]pyrimidine-3-carbonitrile;
6-[methyl-[1-[5-(trifluoromethoxy)pyrimidin-2-yl]propyl]amino]-4-oxo-1-[1-[6-(trifluoromethyl)-3-pyridyl]ethyl]-5H-pyrazolo[3,4-d]pyrimidine-3-carbonitrile;
1-[1-(6-cyclopropyl-3-pyridyl)ethyl]-6-[1-(5-fluoropyrimidin-2-yl)propyl-methyl-amino]-4-oxo-5H-pyrazolo[3,4-d]pyrimidine-3-carbonitrile;
1-[1-(4-cyclopropylphenyl)ethyl]-6-[1-(5-fluoropyrimidin-2-yl)propyl-methyl-amino]-4-oxo-5H-pyrazolo[3,4-d]pyrimidine-3-carbonitrile;
4-oxo-6-((1-(pyrimidin-2-yl)propyl)amino)-1-(1-(6-(trifluoromethyl)pyridin-3-yl)ethyl)-4,5-dihydro-1H-pyrazolo[3,4-d]pyrimidine-3-carbonitrile;
6-((1-(5-fluoropyrimidin-2-yl)propyl)(methyl)amino)-4-oxo-1-(1-(6-(trifluoromethyl)pyridin-3-yl)ethyl)-4,5-dihydro-1H-pyrazolo[3,4-d]pyrimidine-3-carbonitrile;
4-oxo-6-(2-(pyrimidin-2-yl)ethyl)-1-(1-(6-(trifluoromethyl)pyridin-3-yl)ethyl)-4,5-dihydro-1H-pyrazolo[3,4-d]pyrimidine-3-carbonitrile;
6-(2-(5-cyclopropylpyrimidin-2-yl)ethyl)-4-oxo-1-(1-(6-(trifluoromethyl)pyridin-3-yl)ethyl)-4,5-dihydro-1H-pyrazolo[3,4-d]pyrimidine-3 -carbonitrile;
4-oxo-6-(2-(pyrimidin-2-yl)propyl)-1-(1-(6-(trifluoromethyl)pyridin-3-yl)ethyl)-4,5-dihydro-1H-pyrazolo[3,4-d]pyrimidine-3-carbonitrile; and
4-oxo-6-(2-(pyrimidin-2-yl)butyl)-1-(1-(6-(trifluoromethyl)pyridin-3-yl)ethyl)-4,5-dihydro-1H-pyrazolo[3,4-d]pyrimidine-3-carbonitrile;
or a pharmaceutically acceptable salt thereof.

Certain embodiments of the present invention include a compound which is selected from the subject compounds of the Examples herein or a pharmaceutically acceptable salt thereof.

The compounds of the present invention may contain one or more asymmetric centers and can thus occur as racemates and racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within the ambit of this invention. The present invention is meant to comprehend all such isomeric forms of these compounds. Likewise, the present invention includes tautomeric forms of the compounds disclosed herein. Formula I shows the structure of the class of compounds without specific stereochemistry. At least some of the chemical names of compounds of the invention as set forth in this application may have been generated on an automated basis by use of commercially available chemical naming software programs, and have not been independently verified.

The independent syntheses of these diastereomers or their chromatographic separations may be achieved as known in the art by appropriate modification of the methodology disclosed herein. Their absolute stereochemistry may be determined by the x-ray crystallography of crystalline products or crystalline intermediates which are derivatized, if necessary, with a reagent containing an asymmetric center of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography. The coupling reaction is often the formation of salts using an enantiomerically pure acid or base. The diasteromeric derivatives may then be converted to the pure enantiomers by cleavage of the added chiral residue. The racemic mixture of the compounds can also be separated directly by chromatographic methods utilizing chiral stationary phases, which methods are well known in the art. Alternatively, any enantiomer of a compound may be obtained by stereoselective synthesis using optically pure starting materials or reagents of known configuration by methods well known in the art.

The compounds of the present invention may exist in different tautomeric forms, and all such forms are embraced within the scope of the invention. Also, for example, all keto-enol, lactam-lactim and amide-imidic acid forms of the compounds are included in the invention. Thus, for example, the compounds of the invention of the formula:

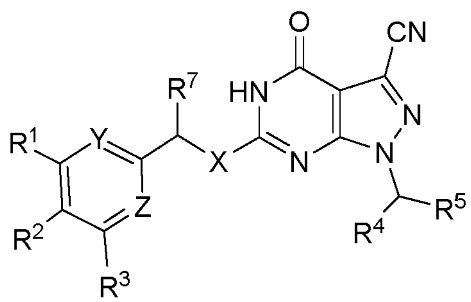

and their tautomers:

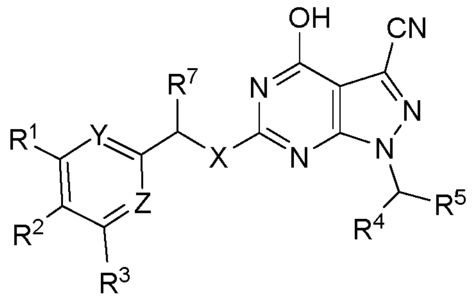

are both contemplated as being within the scope of the compounds of the invention, and the depiction of a particular tautomeric form embraces all other tautomeric forms.

As appreciated by those of skill in the art, halogen or halo as used herein are intended to include fluoro, chloro, bromo and iodo. The term $C_{1-6}$, as in $C_{1-6}$alkyl is defined to identify the group as having 1, 2, 3, 4, 5 or 6 carbons in a linear or branched arrangement, such that $C_{1-6}$alkyl specifically includes methyl, ethyl, n-propyl, iso-propyl, n-butyl, iso-butyl, tert-butyl, pentyl, and hexyl. Substituents (such as $R^1$, $R^2$, $R^3$, $R^{1a}$, $R^{1b}$, $R^{1c}$, $R^6$ and $R^7$) may be absent if the valency of the group to which they are attached does not permit such substitution. A group which is designated as being independently substituted with substituents may be independently substituted with multiple numbers of such substituents.

The present invention also includes all pharmaceutically acceptable isotopic variations of a compound of the Formula I in which one or more atoms is replaced by atoms having the same atomic number, but an atomic mass or mass number different from the atomic mass or mass number usually found in nature. Such compounds are identical to those disclosed herein, but for the fact that one or more atoms are replaced by an atom having an atomic mass or mass number different from the atomic mass or mass number usually found in nature. Examples of isotopes that can be incorporated into the compounds of the invention include isotopes of hydrogen such as $^{2}H$ and $^{3}H$, carbon such as $^{11}C$, $^{13}C$ and $^{14}C$, nitrogen such as $^{13}N$ and $^{15}N$, oxygen such as $^{15}O$, 170 and $^{18}O$, phosphorus such as $^{32}P$, sulfur such as $^{35}S$, fluorine such as $^{18}F$, iodine such as $^{123}I$ and $^{125}I$, and chlorine such as $^{36}Cl$. Certain isotopically-labelled compounds of Formula I, for example those incorporating a radioactive isotope, are useful in drug and/or substrate tissue distribution studies. The radioactive isotopes tritium, i.e. $^{3}H$, and carbon-14, i.e. $^{14}C$, are particularly useful for this purpose in view of their ease of incorporation and ready means of detection. Substitution with heavier isotopes such as deuterium, i.e. $^{2}H$, may afford certain therapeutic advantages resulting from greater metabolic stability, for example, increased in vivo half-life or reduced dosage requirements, and hence may be preferred in some circumstances. Substitution with positron emitting isotopes, such as $^{11}C$, $^{18}F$, $^{15}O$ and $^{13}N$, can be useful in Positron Emission Topography (PET) studies for examining substrate receptor occupancy. An embodiment of the present invention includes compounds that are substituted with a positron emitting isotope. An embodiment of the present invention includes compounds that are substituted with a $^{11}C$ isotope. An embodiment of the present invention includes compounds that are substituted with an $^{18}F$ isotope. In the compounds of the invention, the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds of the invention. For example, different isotopic forms of hydrogen (H) include protium ($^{1}H$) and deuterium ($^{2}H$). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched compounds of the invention can be prepared without undue experimentation by conventional techniques well known to those skilled in the art or by processes analogous to those described in the schemes and examples herein using appropriate isotopically-enriched reagents and/or intermediates.

Those skilled in the art will recognize those instances in which the compounds of the invention may form salts. In such instances, another embodiment provides pharmaceutically acceptable salts of the compounds of the invention. Thus, reference to a compound of the invention herein is understood to include reference to salts thereof, unless otherwise indicated. The term "pharmaceutically acceptable salts" refers to salts prepared from pharmaceutically acceptable non-toxic bases or acids including inorganic or organic bases and inorganic or organic acids. In addition, when a compound of the invention contains both a basic moiety, such as, but not limited to a pyridine or imidazole, and an acidic moiety, such as, but not limited to a carboxylic acid, zwitterions ("inner salts") may be formed and are included within the present invention. Salts derived from inorganic bases include aluminum, ammonium, calcium, copper, ferric, ferrous, lithium, magnesium, manganic salts, manganous, potassium, sodium, zinc, and the like. Particular embodiments include the ammonium, calcium, magnesium, potassium, and sodium salts. Salts in the solid form may exist in more than one crystal structure, and may also be in the form of hydrates or solvates. Salts derived from pharmaceutically acceptable organic non-toxic bases include salts of primary, secondary, and tertiary amines, substituted amines including naturally occurring

substituted amines, cyclic amines, and basic ion exchange resins, such as arginine, betaine, caffeine, choline, N,N'-dibenzylethylene-diamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like. When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like. Particular embodiments include the citric, hydrobromic, hydrochloric, maleic, phosphoric, sulfuric, trifluoroacetic, fumaric, and tartaric acids. It will be understood that, as used herein, references to the compounds of Formula I are meant to also include the pharmaceutically acceptable salts. Salts of the compounds of the invention may be formed by methods known to those of ordinary skill in the art, for example, by reacting a compound of the invention with an amount of acid or base, such as an equivalent amount, in a medium such as one in which the salt precipitates or in an aqueous medium followed by lyophilization.

Exemplifying the invention is the use of the compounds disclosed in the Examples and herein. Specific compounds within the present invention include a compound which is selected from the compounds disclosed in the following Examples and pharmaceutically acceptable salts thereof and individual enantiomers or diastereomers thereof.

The subject compounds may be useful in a method of treating a cardiovascular or cerebrovascular disease, or a neurological or psychiatric disorder associated with PDE9 dysfunction in a patient such as a mammal in need of such inhibition comprising the administration of an effective amount of the compound. In addition to primates, especially humans, a variety of other mammals may be treated according to the method of the present invention. The subject compounds may be useful in a method of inhibiting PDE9 activity in a patient such as a mammal in need of such inhibition comprising the administration of an effective amount of the compound. The subject compounds may also may be useful for treating a neurological or psychiatric disorder associated with striatal hypofunction or basal ganglia dysfunction in a mammalian patient in need thereof. The subject compounds may also may be useful for treating a cardiovascular and cerebrovascular disease, such as hypertension and heart failure. In addition to primates, especially humans, a variety of other mammals may be treated according to the method of the present invention.

The present invention is directed to a compound of the present invention or a pharmaceutically acceptable salt thereof for use in medicine. The present invention is further directed to a use of a compound of the present invention or a pharmaceutically acceptable salt thereof for the manufacture of a medicament for treating a neurological or psychiatric disorder associated with PDE9 dysfunction in a mammalian patient in need thereof. The present invention is further directed to a use of a compound of the present invention or a pharmaceutically acceptable salt thereof for the manufacture of a medicament for treating neurological and psychiatric disorders, such as schizophrenia, psychosis or Huntington's disease, and those associated with striatal hypofunction or basal ganglia dysfunction, and cardiovascular and cerebrovascular diseases, such as hypertension and heart failure, in a mammalian patient in need thereof.

As used herein, the terms "treatment" and "treating" refer to processes wherein there may be a slowing, interrupting, arresting, controlling, or stopping of the progression of the diseases or disorders described herein, but does not necessarily indicate a total elimination of all disease or disorder symptoms, as well as the prophylactic therapy to retard the progression or reduce the risk of the noted conditions, particularly in a patient who is predisposed to such disease or disorder, but does not yet experience or display symptoms of the disease state, inhibiting the disease state, i.e., arresting the development of the disease state or its clinical symptoms, or relieving the disease state, i.e., causing temporary or permanent regression of the disease state or its clinical symptoms.

The subject treated in the present methods is generally a mammal, in particular, a human being, male or female, in whom therapy is desired. The term "therapeutically effective amount" means the amount of the subject compound that will elicit the biological or medical response of a tissue, system, animal or human that is being sought by the researcher, veterinarian, medical doctor or other clinician. It is recognized that one skilled in the art may affect the neurological and psychiatric disorders by treating a patient presently afflicted with the disorders or by prophylactically treating a patient afflicted with such disorders with an effective amount of the compound of the present invention.

Inhibitors of PDE9, and in particular inhibitors of PDE9A, may provide therapeutic benefit to those individuals suffering from psychiatric and cognitive disorders. The conserved localization of PDE9 in cortex and hippocampus of rodents and humans, brain regions that play a key role in memory and learning, together with the previously described role for NO/cGMP/PKG signaling in synaptic plasticity and cognition has focused attention on a possible role for PDE9 in cognitive function and consequently as a therapeutic target for cognitive dysfunction in Alzheimer's disease and schizophrenia. The mechanism by which PDE9 inhibition improve cognitive function through the modulation of glutamate and/or cholinergic neuron signaling is potentially feasible given that both glutamate (NMDA) and cholinergic receptor activation enhance the formation of cGMP in brain and both neural substrates are involved in cognitive function.

As used herein, the term "selective PDE9 inhibitor" refers to an organic molecule that effectively inhibits an enzyme from the PDE9 family to a greater extent than enzymes from the PDE 1-8 or PDE10-11 families. In one embodiment, a selective PDE9 inhibitor is an organic molecule having a Ki for inhibition of PDE9 that is less than or about one-tenth that for a substance that is an inhibitor for another PDE enzyme. In other words, the organic molecule inhibits PDE9 activity to the same degree at a concentration of about one-tenth or less than the concentration required for any other PDE enzyme. Preferably, a selective PDE9 inhibitor is an organic molecule, having a Ki for inhibition of PDE9 that is less than or about one-hundredth that for a substance that is an inhibitor for another PDE enzyme. In other words, the organic molecule inhibits PDE9 activity to the same degree at a concentration of about one-hundredth or less than the concentration required for any other PDE enzyme. A "selective PDE9 inhibitor" can be identified, for example, by comparing the ability of an organic molecule to inhibit PDE9 activity to its ability to inhibit PDE enzymes from the other PDE families. For example, an organic molecule may be assayed for its ability to inhibit PDE9 activity, as well as PDE1A, PDE1B, PDE1C, PDE2A, PDE3A, PDE3B, PDE4A, PDE4B, PDE4C, PDE4D, PDE5A, PDE6A, PDE6B, PDE6C, PDE7A, PDE7B, PDE8A, PDE8B, PDE10A, and/or PDE11A.

Phosphodiesterase enzymes including PDE9 have been implicated in a wide range of biological functions. This has suggested a potential role for these enzymes in a variety of disease processes in humans or other species. The compounds of the present invention may have utility in treating a variety of neurological and psychiatric disorders, such as schizophrenia, psychosis or Huntington’s disease, and those associated with striatal hypofunction or basal ganglia dysfunction, and cardiovascular and cerebrovascular diseases, such as hypertension and heart failure.

In an embodiment, compounds of the present invention may provide a method for treating schizophrenia or psychosis comprising administering to a patient in need thereof an effective amount of a compound of the present invention. The Diagnostic and Statistical Manual of Mental Disorders (DSM-IV-TR) (2000, American Psychiatric Association, Washington DC) provides a diagnostic tool that includes paranoid, disorganized, catatonic or undifferentiated schizophrenia and substance-induced psychotic disorders. As used herein, the term "schizophrenia or psychosis" includes the diagnosis and classification of these mental disorders as described in DSM-IV-TR and the term is intended to include similar disorders described in other sources. Disorders and conditions encompassed herein include, but are not limited to, conditions or diseases such as schizophrenia or psychosis, including schizophrenia (paranoid, disorganized, catatonic, undifferentiated, or residual type), schizophreniform disorder, schizoaffective disorder, for example of the delusional type or the depressive type, delusional disorder, psychotic disorder, brief psychotic disorder, shared psychotic disorder, psychotic disorder due to a general medical condition and substance-induced or drug-induced (for example psychosis induced by alcohol, amphetamine, cannabis, cocaine, hallucinogens, inhalants, opioids, phencyclidine, ketamine and other dissociative anaesthetics, and other psychostimulants), psychosispsychotic disorder, psychosis associated with affective disorders, brief reactive psychosis, schizoaffective psychosis, "schizophrenia-spectrum" disorders such as schizoid or schizotypal personality disorders, personality disorder of the paranoid type, personality disorder of the schizoid type, illness associated with psychosis (such as major depression, manic depressive (bipolar) disorder, Alzheimer’s disease and post-traumatic stress syndrome), including both the positive and the negative symptoms of schizophrenia and other psychoses.

In another embodiment, the compounds of the present invention may provide a method for treating cognitive disorders or enhancing cognition comprising administering to a patient in need thereof an effective amount of a compound of the present invention. The DSM-IV-TR also provides a diagnostic tool that includes cognitive disorders including dementia, delirium, amnestic disorders and age-related cognitive decline. As used herein, the term "cognitive disorders" includes the diagnosis and classification of these disorders as described in DSM-IV-TR and the term is intended to include similar disorders described in other sources. Disorders and conditions encompassed herein include, but are not limited to, disorders that comprise as a symptom a deficiency in attention and/or cognition, such as dementia (associated with Alzheimer’s disease, ischemia, multi-infarct dementia, trauma, intracranial tumors, cerebral trauma, vascular problems or stroke, alcoholic dementia or other drug-related dementia, AIDS, HIV disease, Parkinson’s disease, Huntington’s disease, Pick’s disease, Creutzfeldt Jacob disease, perinatal hypoxia, other general medical conditions or substance abuse), Alzheimer’s disease, multi-infarct dementia, AIDS-related dementia, and Fronto temperal dementia, delirium, amnestic disorders or age related cognitive decline.

In another embodiment, compounds of the present invention may provide a method for treating anxiety disorders comprising administering to a patient in need thereof an effective amount of a compound of the present invention. The DSM-IV-TR also provides a diagnostic tool that includes anxiety disorders as generalized anxiety disorder, obsessive-compulsive disorder and panic attack. As used herein, the term "anxiety disorders" includes the diagnosis and classification of these mental disorders as described in DSM-IV-TR and the term is intended to include similar disorders described in other sources. Disorders and conditions encompassed herein include, but are not limited to, anxiety disorders such as, acute stress disorder, agoraphobia, generalized anxiety disorder, obsessive-compulsive disorder, panic attack, panic disorder, post-traumatic stress disorder, separation anxiety disorder, social phobia, specific phobia, substance-induced anxiety disorder and anxiety due to a general medical condition.

In another embodiment, compounds of the present invention may provide a method for treating substance-related disorders and addictive behaviors comprising administering to a patient in need thereof an effective amount of a compound of the present invention. The DSM-IV-TR also provides a diagnostic tool that includes persisting dementia,

persisting amnestic disorder, psychotic disorder or anxiety disorder induced by substance abuse, and tolerance of, dependence on or withdrawal from substances of abuse. As used herein, the term "substance-related disorders and addictive behaviors" includes the diagnosis and classification of these mental disorders as described in DSM-IV-TR and the term is intended to include similar disorders described in other sources. Disorders and conditions encompassed herein include, but are not limited to, substance-related disorders and addictive behaviors, such as substance-induced delirium, persisting dementia, persisting amnestic disorder, psychotic disorder or anxiety disorder, drug addiction, tolerance, and dependence or withdrawal from substances including alcohol, amphetamines, cannabis, cocaine, hallucinogens, inhalants, nicotine, opioids, phencyclidine, sedatives, hypnotics or anxiolytics.

In another embodiment, compounds of the present invention may provide a method for treating obesity or eating disorders associated with excessive food intake, and complications associated therewith, comprising administering to a patient in need thereof an effective amount of a compound of the present invention. At present, obesity is included in the tenth edition of the International Classification of Diseases and Related Health Problems (ICD-10) (1992 World Health Organization) as a general medical condition. The DSM-IV-TR also provides a diagnostic tool that includes obesity in the presence of psychological factors affecting medical condition. As used herein, the term "obesity or eating disorders associated with excessive food intake" includes the diagnosis and classification of these medical conditions and disorders described in ICD-10 and DSM-IV-TR and the term is intended to include similar disorders described in other sources. Disorders and conditions encompassed herein include, but are not limited to, obesity, bulimia nervosa and compulsive eating disorders.

In another embodiment, compounds of the present invention may provide a method for treating mood and depressive disorders comprising administering to a patient in need thereof an effective amount of a compound of the present invention. As used herein, the term "mood and depressive disorders" includes the diagnosis and classification of these medical conditions and disorders described in the DSM-IV-TR and the term is intended to include similar disorders described in other sources. Disorders and conditions encompassed herein include, but are not limited to, bipolar disorders, mood disorders including depressive disorders, major depressive episode of the mild, moderate or severe type, a manic or mixed mood episode, a hypomanic mood episode, a depressive episode with atypical features, a depressive episode with melancholic features, a depressive episode with catatonic features, a mood episode with postpartum onset, post-stroke depression; major depressive disorder, dysthymic disorder, minor depressive disorder, premenstrual dysphoric disorder, post-psychotic depressive disorder of schizophrenia, a major depressive disorder superimposed on a psychotic disorder such as delusional disorder or schizophrenia, a bipolar disorder, for example, bipolar I disorder, bipolar II disorder, cyclothymic disorder, depression including unipolar depression, seasonal depression and post-partum depression, premenstrual syndrome (PMS) and premenstrual dysphoric disorder (PDD), mood disorders due to a general medical condition, and substance-induced mood disorders.

In another embodiment, compounds of the present invention may provide a method for treating pain comprising administering to a patient in need thereof an effective amount of a compound of the present invention. Particular pain embodiments are bone and joint pain (osteoarthritis), repetitive motion pain, dental pain, cancer pain, myofascial pain (muscular injury, fibromyalgia), perioperative pain (general surgery, gynecological), chronic pain and neuropathic pain.

In other embodiments, compounds of the invention may provide methods for treating other types of cognitive, learning and mental related disorders including, but not limited to, learning disorders, such as a reading disorder, a mathematics disorder, or a disorder of written expression, attention-deficit/hyperactivity disorder, age-related cognitive decline, pervasive developmental disorder including autistic disorder, attention disorders such as attention-deficit hyperactivity disorder (ADHD) and conduct disorder; an NMDA receptor-related disorder, such as autism, depression, benign forgetfulness, childhood learning disorders and closed head injury; a neurodegenerative disorder or condition, such as neurodegeneration associated with cerebral trauma, stroke, cerebral infarct, epileptic seizure, neurotoxin poisoning, or hypoglycemia-induced neurodegeneration; multi-system atrophy; movement disorders, such as akinesias and akinetic-rigid syndromes (including, Parkinson's disease, drug-induced parkinsonism, post-encephalitic parkinsonism, progressive supranuclear palsy, multiple system atrophy, corticobasal degeneration, parkinsonism-ALS dementia complex and basal ganglia calcification), medication-induced parkinsonism (such as, neuroleptic-induced parkinsonism, neuroleptic malignant syndrome, neuroleptic-induced acute dystonia, neuroleptic-induced acute akathisia, neuroleptic-induced tardive dyskinesia and medication-induced postural tremor), Huntington's disease, dyskinesia associated with dopamine agonist therapy, Gilles de la Tourette's syndrome, epilepsy, muscular spasms and disorders associated with muscular spasticity or weakness including tremors; dyskinesias, including tremor (such as, rest tremor, postural tremor, intention tremor and essential tremor), restless leg syndrome, chorea (such as Sydenham's chorea, Huntington's disease, benign hereditary chorea, neuroacanthocytosis, symptomatic chorea, drug-induced chorea and hemiballism), myoclonus (including, generalised myoclonus and focal myoclonus), tics (including, simple tics, complex tics and symptomatic tics), dystonia (including, generalised, iodiopathic, drug-induced, symptomatic, paroxymal, and focal (such as blepharospasm, oromandibular, spasmodic, spasmodic torticollis, axial dystonia, hemiplegic and dystonic writer's cramp)); urinary incontinence; neuronal damage (including ocular damage, retinopathy or macular degeneration of the eye, tinnitus, hearing impairment and loss, and brain edema); emesis; and sleep disorders, including insomnia and narcolepsy.

In another embodiment, compounds of the present invention may provide a method for treating hypertension, such as essential hypertension (also known as primary or idiopathic hypertension) which is a form of hypertension for which no cause can be found, systemic hypertension, pulmonary hypertension (e.g. pulmonary arterial hypertension, pulmonary hypertension of the neonate), and heart failure (which includes both acute heart failure and chronic heart failure, the latter also known as congestive heart failure). The compounds could also be used to treat hypertension which is associated with any of several primary diseases, such as renal, pulmonary, endocrine, and vascular diseases, including treatment of patients with medical conditions such as heart failure and/or chronic kidney disease. Furthermore, the compounds of the present invention could be used in methods for treatment of, prevention of or reduction of risk for developing one or more disorders such as pulmonary hypertension, particularly pulmonary arterial hypertension, cardiovascular disease, edematous states, diabetes mellitus, diabetes insipidus, postoperative volume overload, endothelial dysfunction, diastolic dysfunction, systolic dysfunction, stable and unstable angina pectoris, thromboses, restenosis, myocardial infarction, stroke, cardiac insufficiency, pulmonary hypertonia, atherosclerosis, hepatic cirrhosis, ascitis, pre-eclampsia, cerebral edema, nephropathy, glomerulonephritis, nephrotic syndrome, acute kidney insufficiency, chronic kidney insufficiency (also referred to as chronic kidney disease, or more generally as renal impairment), acute tubular necrosis, hypercalcemia, idiopathic edema, Dent's disease, Meniere's disease, glaucoma, benign intracranial hypertension, and other conditions.

In a specific embodiment, compounds of the present invention may provide a method for treating thrombosis, atherosclerosis, restenosis, hypertension, angina pectoris, angiogenesis related disorders, arrhythmia, a cardiovascular or circulatory disease or condition, acute coronary syndrome, coronary artery disease, thrombosis, conditions of reduced blood vessel patency (for example post percutaneous transluminal coronary angioplasty), peripheral vascular disease, renal disease (especially that occurring with diabetes), angina (including stable, unstable and variant (Prinzmetal) angina), myocardial ischaemia, myocardial infarction, secondary prevention of myocardial infarction or stroke, urgent coronary revascularization, glomerulonephritis, thrombotic stroke, thromboembolytic stroke, peripheral artery disease, deep vein thrombosis, venous thromboembolism, cardiovascular disease associated with hormone replacement therapy, atherosclerosis, hypercholesterolemia, coronary heart disease, metabolic syndrome, acute coronary syndrome, disseminated intravascular coagulation syndrome, cerebral infarction, and conditions associated with cardiopulmonary bypass surgery, cardiac valve repair and replacement surgery, pericardial and aortic repair surgeries, such as bleeding, thrombotic vascular events (such as thrombosis or restenosis), vein graft failure, artery graft failure, atherosclerosis, angina pectoris, myocardial ischemia, acute coronary syndrome, myocardial infarction, heart failure, arrhythmia, hypertension, transient ischemic attack, cerebral function impairment, thromboembolic stroke, cerebral ischemia, cerebral infarction, thrombophlebitis, deep vein thrombosis and peripheral artery disease.

Of the disorders above, the treatment of schizophrenia, bipolar disorder, depression, including unipolar depression, seasonal depression and post-partum depression, premenstrual syndrome (PMS) and premenstrual dysphoric disorder (PDD), learning disorders, pervasive developmental disorders, including autistic disorder, attention disorders including Attention-Deficit/Hyperactivity Disorder, autism, tic disorders including Tourette's disorder, anxiety disorders including phobia and post traumatic stress disorder, cognitive disorders associated with dementia, AIDS dementia, Alzheimer's, Parkinson's, Huntington's disease, spasticity, myoclonus, muscle spasm, tinnitus and hearing impairment and loss are of particular importance.

The activity of the compounds in accordance with the present invention as PDE9 inhibitors may be readily determined without undue experimentation using a fluorescence polarization (FP) methodology that is well known in the art (Huang, W., et al., J. Biomol Screen, 2002, 7: 215). In particular, the compounds of the following examples had activity in reference assays by exhibiting the ability to inhibit the hydrolysis of the phosphosphate ester bond of a cyclic nucleotide.

In a typical experiment the PDE9 inhibitory activity of the compounds of the present invention was determined in accordance with the following experimental method.

Human PDE9 (PDE9A2, GenBank Accession No. NM_001001567), full length with N-terminal GST tag, was purchased from BPS Bioscience. The fluorescence polarization assay for cyclic nucleotide phosphodiesterases was performed using an IMAP® FP kit supplied by Molecular Devices, Sunnyvale, CA (product # R8139). IMAP® technology has been applied previously to phosphodiesterase assays (Huang, W., et al., J. Biomol Screen, 2002, 7: 215). Assays were performed at room temperature in 384-well microtiter plates with an incubation volume of 20.2 μL. Solutions of test compounds were prepared in DMSO and serially diluted with DMSO to yield 8 μL of each of 10 solutions differing by 3-fold in concentration, at 32 serial dilutions per plate. 100% inhibition is determined using a known PDE9 inhibitor, such as 1-(2-chlorophenyl)-6-[(2R)-3,3,3-trifluoro-2-methylpropyl] -1,5-dihydro-4H-pyrazolo [3,4-d] pyrimidine-4-one (BAY 73-6691) (Wunder et al, Mol. Pharmacol., 2005, 68(6): 1775-81), (6-[(3S,4S)-4-methyl-1-(pyrimidin-2-ylmethyl)pyrrolidin-3-yl]-1-(tetrahydro-2H-pyran-4-yl)-1,5-dihydro-4H-pyrazolo[3,4-d]pyrimidin-4-one (PF-04447943) (Wager et al., ACS Chemical Neuroscience, 2010, 1:435-449). 0% of inhibition is determined by using DMSO (1% final concentrations). A Labcyte Echo 555 (Labcyte, Sunnyvale, CA) is used to dispense 200 nL from each well of the titration plate to the 384 well assay plate. Human PDE9A2 membrane preps were diluted to 1 ng/ml. FAM-labeled cGMP substrate (Molecular

Devices, Sunnyvale, CA) was at a concentration of 100 nM (Km of PDE9 for cGMP is 70-170 nM) in the assay buffer (10 mM Tris HCl, pH 7.2, 10 mM $MgCl_2$, 0.05% $NaN_3$ 0.01% Tween-20, and 1 mM DTT). PDE9 enzyme mix and compounds were mixed and incubated at room temperature for 30 min. Following which, FAMcGMP substrate was added, shaken and incubated for an additional 60 min at room temperature. The final concentration of human PDE9 membrane preparations were 0.5 ng/ml. The final concentration of FAM-cGMP was 50 nM. After the incubation period, the enzymatic reaction was stopped by addition of binding solution (IMAP- FP, Molecular Devices, comprised of 80% Solution A, 20% Solution B and a 1:600 dilution of binding reagent) to each well. The plates were shaken then incubated at room temperature for 1 h prior to determining the fluorescence polarization (mP) using a Perkin Elmer EnVision™ plate reader (Waltham, MA).

Fluorescence polarization (mP) was calculated from the parallel (S) and perpendicular (P) fluorescence of each sample well and the analogous values for the median control well, containing only substrate (So and Po), using the following equation:

$$\text{Polarization (mP)} = 1000*(S/So\text{-}P/Po)/(S/So+P/Po).$$

Dose-inhibition profiles for each compound were characterized by fitting the mP data to a four-parameter equation given below. The apparent inhibition constant ($K_I$), the maximum inhibition at the low plateau relative to "100% Inhibition Control" (Imax; e.g. 1=> same as this control), the minimum inhibition at the high plateau relative to the "0% Inhibition Control" (Imin, e.g. 0=> same as the no drug control) and the Hill slope (nH) are determined by a non-linear least squares fitting of the mP values as a function of dose of the compound using an in-house software based on the procedures described by Mosser et al., JALA, 2003, 8: 54-63, using the following equation:

$$mP = \frac{(0\%mP - 100\%mP)(\text{Imax} - \text{Imin})}{1 + \left[\dfrac{[\text{Drug}]}{(10^{-pK_I}(1 + \dfrac{[Substrate]}{K_M})}\right]^{nH}} + 100\%mP + (0\%mP - 100\%mP)(1 - \text{Imax})$$

The median signal of the "0% inhibition controls" (0%mP) and the median signal of the "100% inhibition controls" (100%mP) are constants determined from the controls located in columns 1-2 and 23-24 of each assay plate. An apparent ($K_m$) for FAM-labeled cAMP of 150 nM was determined in separate experiments through simultaneous variation of substrate and selected drug concentrations.

Selectivity for PDE9, as compared to other PDE families, was assessed using the IMAP® technology. Human PDE10A2 enzyme was prepared from cytosolic fractions of transiently transfected HEK cells. All other PDE's were GST Tag human enzyme expressed in insect cells and were obtained from BPS Bioscience (San Diego, CA): PDE1A (Cat# 60010), PDE3A (Cat# 60030), PDE4A1A (Cat# 60040), PDE5A1 (Cat# 60050), PDE6C (Cat# 60060), PDE7A (Cat# 60070), PDE8A1 (Cat# 60080), PDE9A2 (Cat# 60090), PDE11A4 (Cat# 60110).

Assays for PDE 1 through 11 were performed in parallel at room temperature in 384-well microtiter plates with an incubation volume of 20.2 μL. Solutions of test compounds were prepared in DMSO and serially diluted with DMSO to yield 30 μL of each of ten solutions differing by 3-fold in concentration, at 32 serial dilutions per plate. 100% inhibition was determined by adding buffer in place of the enzyme and 0% inhibition is determined by using DMSO (1% final concentrations). A Labcyte POD 810 (Labcyte, Sunnyvale, CA) was used to dispense 200 nL from each well of the titration plate to make eleven copies of the assay plate for each titration, one copy for each PDE enzyme. A solution of each enzyme (dilution from aliquots, sufficient to produce 20% substrate conversion) and a separate solution of FAM-labeled cAMP or FAM-labeled cGMP from Molecular Devices ( Sunnyvale, CA, product # R7506 or cGMP#R7508), at a final concentration of 50 nM were made in the assay buffer (10 mM Tris HCl, pH 7.2, 10 mM $MgCl_2$, 0.05% $NaN_3$ 0.01% Tween-20, and 1 mM DTT). Note that the substrate for PDE2 is 50 nM FAM cAMP containing 1000 nM of cGMP. The enzyme and the substrate were then added to the assay plates in two consecutive additions of 10 μL and then shaken to mix. The reaction was allowed to proceed at room temperature for 60 minutes. A binding solution was then made from the kit components, comprised of 80% Solution A, 20% Solution B and binding reagent at a volume of 1/600 the total binding solution. The enzymatic reaction was stopped by addition of 60 μL of the binding solution to each well of the assay plate. The plates were sealed and shaken for 10 seconds. The plates were incubated at room temperature for one hour. The parallel and perpendicular fluorescence of each well of the plate was measured using a Perkin Elmer EnVision™ plate reader (Waltham, MA).

The apparent inhibition constants for the compounds against all 11 PDE's was determined from the parallel and perpendicular fluorescent readings as described for PDE FP assay using the following apparent $K_M$ values for each

enzyme and substrate combination: PDE1A (FAM cGMP) 70 nM, rhesus PD2A3 (FAM cAMP) 10,000 nM, PDE3A (FAM cAMP) 50 nM, PDE4A1A (FAM cAMP) 1500 nM, PDE5A1 (FAM cGMP) 400 nM, PDE6C (FAM cGMP) 700 nM, PDE7A (FAM cAMP) 150 nM, PDE8A1 (FAM cAMP) 50 nM, PDE10A2 (FAM cAMP) 150nM, PDE11A4 (FAM cAMP) 1000 nM. The intrinsic PDE10 inhibitory activity of a compound which may be used in accordance with the present invention may be determined by these assays.

The compounds of the following examples had activity in inhibiting the human PDE9 enzyme in the aforementioned assays, generally with a Ki of less than about 500 nM. Many of compounds within the present invention had activity in inhibiting the human PDE9 enzyme in the aforementioned assays with a Ki of less than about 100 nM, and wherein some of the compounds have a Ki of less than about 10 nM. Additional data are provided in the following Examples. Such a result is indicative of the intrinsic activity of the compounds in use as inhibitors of the PDE9 enzyme. In general, one of ordinary skill in the art would appreciate that a substance is considered to effectively inhibit PDE9 activity if it has a Ki of less than or about 500 nM, where more potent inhinbitors have a Ki of less than or about 100 nM. The present invention also includes compounds within the scope of the invention which possess activity as inhibitors of other phosphodiesterase enzymes.

The subject compounds may be further useful in a method for the prevention, treatment, control, amelioration, or reduction of risk of the diseases, disorders and conditions noted herein. The subject compounds may be further useful in a method for the prevention, treatment, control, amelioration, or reduction of risk of the aforementioned diseases, disorders and conditions in combination with other agents. The compounds of the present invention may be used in combination with one or more other drugs in the treatment, prevention, control, amelioration, or reduction of risk of diseases or conditions for which compounds of the present invention or the other drugs may have utility, where the combination of the drugs together are safer or more effective than either drug alone. Such other drug(s) may be administered, by a route and in an amount commonly used therefore, contemporaneously or sequentially with a compound of the present invention. When a compound of the present invention is used contemporaneously with one or more other drugs, a pharmaceutical composition in unit dosage form containing such other drugs and the compound of the present invention may be desirable. However, the combination therapy may also include therapies in which the compound of the present invention and one or more other drugs are administered on different overlapping schedules. It is also contemplated that when used in combination with one or more other active ingredients, the compounds of the present invention and the other active ingredients may be used in lower doses than when each is used singly. Accordingly, the pharmaceutical compositions of the present invention include those that contain one or more other active ingredients, in addition to a compound of the present invention. The above combinations include combinations of a compound of the present invention not only with one other active compound, but also with two or more other active compounds. Likewise, compounds of the present invention may be used in combination with other drugs that are used in the prevention, treatment, control, amelioration, or reduction of risk of the diseases or conditions for which compounds of the present invention are useful. Such other drugs may be administered, by a route and in an amount commonly used therefore, contemporaneously or sequentially with a compound of the present invention. Accordingly, the pharmaceutical compositions of the present invention include those that also contain one or more other active ingredients, in addition to a compound of the present invention. The weight ratio of the compound of the present invention to the second active ingredient may be varied and will depend upon the effective dose of each ingredient. Generally, an effective dose of each will be used. Thus, for example, when a compound of the present invention is combined with another agent, the weight ratio of the compound of the present invention to the other agent will generally range from about 1000: 1 to about 1:1000, such as about 200:1 to about 1:200. Combinations of a compound of the present invention and other active ingredients will generally also be within the aforementioned range, but in each case, an effective dose of each active ingredient should be used.

In such combinations the compound of the present invention and other active agents may be administered separately or in conjunction. In addition, the administration of one element may be prior to, concurrent to, or subsequent to the administration of other agent(s).

Accordingly, the subject compounds may be used alone or in combination with other agents which are known to be beneficial in the subject indications or other drugs that affect receptors or enzymes that either increase the efficacy, safety, convenience, or reduce unwanted side effects or toxicity of the compounds of the present invention. The subject compound and the other agent may be co-administered, either in concomitant therapy or in a fixed combination.

In one embodiment, the subject compound may be employed in combination with anti-Alzheimer's agents, beta-secretase inhibitors, gamma-secretase inhibitors, HMG-CoA reductase inhibitors, NSAID's including ibuprofen, vitamin E, and anti-amyloid antibodies.

In another embodiment, the subject compound may be employed in combination with sedatives, hypnotics, anxiolytics, antipsychotics, antianxiety agents, cyclopyrrolones, imidazopyridines, pyrazolopyrimidines, minor tranquilizers, melatonin agonists and antagonists, melatonergic agents, benzodiazepines, barbiturates, 5HT-2 antagonists, and the like, such as: adinazolam, allobarbital, alonimid, alprazolam, amisulpride, amitriptyline, amobarbital, amoxapine, aripiprazole, atypical antipsychotics, bentazepam, benzoctamine, brotizolam, bupropion, busprione, butabarbital, butalbital, capuride, carbocloral, chloral betaine, chloral hydrate, clomipramine, clonazepam, cloperidone, clorazepate, chlordiazepoxide, clorethate, chlorpromazine, clozapine, cyprazepam, desipramine, dexclamol, diazepam, dichloralphenazone, divalproex, diphenhydramine, doxepin, estazolam, ethchlorvynol, etomidate, fenobam, flunitrazepam, flupentixol, fluphenazine, flurazepam, fluvoxamine, fluoxetine, fosazepam, glutethimide, halazepam, haloperidol, hydroxyzine, imipramine, lithium, lorazepam, lormetazepam, maprotiline, mecloqualone, melatonin, mephobarbital, meprobamate, methaqualone, midaflur, midazolam, nefazodone, nisobamate, nitrazepam, nortriptyline, olanzapine, oxazepam, paraldehyde, paroxetine, pentobarbital, perlapine, perphenazine, phenelzine, phenobarbital, prazepam, promethazine, propofol, protriptyline, quazepam, quetiapine, reclazepam, risperidone, roletamide, secobarbital, sertraline, suproclone, temazepam, thioridazine, thiothixene, tracazolate, tranylcypromaine, trazodone, triazolam, trepipam, tricetamide, triclofos, trifluoperazine, trimetozine, trimipramine, uldazepam, venlafaxine, zaleplon, ziprasidone, zolazepam, zolpidem, and salts thereof, and combinations thereof, and the like, or the subject compound may be administered in conjunction with the use of physical methods such as with light therapy or electrical stimulation.

In another embodiment, the subject compound may be employed in combination with levodopa (with or without a selective extracerebral decarboxylase inhibitor such as carbidopa or benserazide), anticholinergics such as biperiden (optionally as its hydrochloride or lactate salt) and trihexyphenidyl (benzhexol) hydrochloride, COMT inhibitors such as entacapone, MOA-B inhibitors, antioxidants, A2a adenosine receptor antagonists, cholinergic agonists, NMDA receptor antagonists, serotonin receptor antagonists and dopamine receptor agonists such as alentemol, bromocriptine, fenoldopam, lisuride, naxagolide, pergolide and pramipexole. It will be appreciated that the dopamine agonist may be in the form of a pharmaceutically acceptable salt, for example, alentemol hydrobromide, bromocriptine mesylate, fenoldopam mesylate, naxagolide hydrochloride and pergolide mesylate. Lisuride and pramipexol are commonly used in a non-salt form.

In another embodiment, the subject compound may be employed in combination with a compound from the phenothiazine, thioxanthene, heterocyclic dibenzazepine, butyrophenone, diphenylbutylpiperidine and indolone classes of neuroleptic agent. Suitable examples of phenothiazines include chlorpromazine, mesoridazine, thioridazine, acetophenazine, fluphenazine, perphenazine and trifluoperazine. Suitable examples of thioxanthenes include chlorprothixene and thiothixene. An example of a dibenzazepine is clozapine. An example of a butyrophenone is haloperidol. An example of a diphenylbutylpiperidine is pimozide. An example of an indolone is molindolone. Other neuroleptic agents include loxapine, sulpiride and risperidone. It will be appreciated that the neuroleptic agents when used in combination with the subject compound may be in the form of a pharmaceutically acceptable salt, for example, chlorpromazine hydrochloride, mesoridazine besylate, thioridazine hydrochloride, acetophenazine maleate, fluphenazine hydrochloride, flurphenazine enathate, fluphenazine decanoate, trifluoperazine hydrochloride, thiothixene hydrochloride, haloperidol decanoate, loxapine succinate and molindone hydrochloride. Perphenazine, chlorprothixene, clozapine, haloperidol, pimozide and risperidone are commonly used in a non-salt form. Thus, the subject compound may be employed in combination with acetophenazine, alentemol, aripiprazole, amisulpride, benzhexol, bromocriptine, biperiden, chlorpromazine, chlorprothixene, clozapine, diazepam, fenoldopam, fluphenazine, haloperidol, levodopa, levodopa with benserazide, levodopa with carbidopa, lisuride, loxapine, mesoridazine, molindolone, naxagolide, olanzapine, pergolide, perphenazine, pimozide, pramipexole, quetiapine, risperidone, sulpiride, tetrabenazine, trihexyphenidyl, thioridazine, thiothixene, trifluoperazine or ziprasidone.

In another embodiment, the subject compound may be employed in combination with an anti-depressant or anti-anxiety agent, including norepinephrine reuptake inhibitors (including tertiary amine tricyclics and secondary amine tricyclics), selective serotonin reuptake inhibitors (SSRIs), monoamine oxidase inhibitors (MAOIs), reversible inhibitors of monoamine oxidase (RIMAs), serotonin and noradrenaline reuptake inhibitors (SNRIs), corticotropin releasing factor (CRF) antagonists, $\alpha$-adrenoreceptor antagonists, neurokinin-1 receptor antagonists, atypical antidepressants, benzodiazepines, 5-$HT_{1A}$ agonists or antagonists, especially 5-$HT_{1A}$ partial agonists, and corticotropin releasing factor (CRF) antagonists. Specific agents include: amitriptyline, clomipramine, doxepin, imipramine and trimipramine; amoxapine, desipramine, maprotiline, nortriptyline and protriptyline; fluoxetine, fluvoxamine, paroxetine and sertraline; isocarboxazid, phenelzine, tranylcypromine and selegiline; moclobemide: venlafaxine; duloxetine; aprepitant; bupropion, lithium, nefazodone, trazodone and viloxazine; alprazolam, chlordiazepoxide, clonazepam, chlorazepate, diazepam, halazepam, lorazepam, oxazepam and prazepam; buspirone, flesinoxan, gepirone and ipsapirone, and pharmaceutically acceptable salts thereof.

In one embodiment, the subject compound may be employed in combination with thiazide-like diuretics, e.g., hydrochlorothiazide (HCTZ or HCT); angiotensin converting enzyme inhibitors (e.g, alacepril, benazepril, captopril, ceronapril, cilazapril, delapril, enalapril, enalaprilat, fosinopril, imidapril, lisinopril, moveltipril, perindopril, quinapril, ramipril, spirapril, temocapril, or trandolapril); dual inhibitors of angiotensin converting enzyme (ACE) and neutral endopeptidase (NEP) such as omapatrilat, sampatrilat and fasidotril; angiotensin II receptor antagonists, also known as angiotensin receptor blockers or ARBs, which may be in free-base, free-acid, salt or pro-drug form, such as azilsartan, e.g., azilsartan medoxomil potassium (EDARBI®), candesartan, e.g., candesartan cilexetil (ATACAND®), eprosartan, e.g., eprosartan mesylate (TEVETAN®), irbesartan (AVAPRO®), losartan, e.g., losartan potassium (COZAAR®), olmesartan, e.g, olmesartan medoximil (BENICAR®), telmisartan (MICARDIS®), valsartan (DIOVAN®), and any of these drugs used in combination with a thiazide-like diuretic such as hydrochlorothiazide (e.g., HYZAAR@, DIOVAN HCT®, ATACAND HCT®), etc.); potassium sparing diuretics such as amiloride HCl, spironolactone, epleranone, triamterene, each with or without HCTZ; carbonic anhydrase inhibitors, such as acetazolamide; neutral endopeptidase inhibitors (e.g., thiorphan and phosphoramidon); aldosterone antagonists; aldosterone synthase inhibitors; renin inhibitors; pepstatin derivatives and fluoro- and chloro-derivatives of statone-containing peptides; enalkrein; aliskiren (2(S),4(S),5(S),7(S)-N-(2-carbamoyl-2-methylpropyl)-5-amino-4-hydroxy-2,7-diisopropyl-8-[4-methoxy-3-(3-methoxypropoxy)-phenyl]-octanamid hemifumarate)); endothelin receptor antagonists; vasodilators (e.g. nitroprusside); calcium channel blockers (e.g., amlodipine, nifedipine, verapamil, diltiazem, felodipine, gallopamil, niludipine, nimodipine, nicardipine, bepridil, nisoldipine); potassium channel activators (e.g., nicorandil, pinacidil, cromakalim, minoxidil, aprilkalim, loprazolam); sympatholitics; beta-adrenergic blocking drugs (e.g., acebutolol, atenolol, betaxolol, bisoprolol, carvedilol, metoprolol, metoprolol tartate, nadolol, propranolol, sotalol, timolol); alpha adrenergic blocking drugs (e.g., doxazocin, prazocin or alpha methyldopa); central alpha adrenergic agonists; peripheral vasodilators (e.g. hydralazine); nitrates or nitric oxide donating compounds, e.g. isosorbide mononitrate; lipid lowering agents, e.g., HMG-CoA reductase inhibitors such as simvastatin and lovastatin which are marketed as ZOCOR® and MEVACOR® in lactone pro-drug form and function as inhibitors after administration, and pharmaceutically acceptable salts of dihydroxy open ring acid HMG-CoA reductase inhibitors such as atorvastatin (particularly the calcium salt sold in LIPITOR®), rosuvastatin (particularly the calcium salt sold in CRESTOR®), pravastatin (particularly the sodium salt sold in PRAVACHOL®), and fluvastatin (particularly the sodium salt sold in LESCOL®); a cholesterol absorption inhibitor such as ezetimibe (ZETIA®), and ezetimibe in combination with any other lipid lowering agents such as the HMG-CoA reductase inhibitors noted above and particularly with simvastatin (VYTORIN®) or with atorvastatin calcium; niacin in immediate-release or controlled release forms, and particularly niacin in combination with a DP antagonist such as laropiprant and/or with an HMG-CoA reductase inhibitor; niacin receptor agonists such as acipimox and acifran, as well as niacin receptor partial agonists; metabolic altering agents including insulin sensitizing agents and related compounds for the treatment of diabetes such as biguanides (e.g., metformin), meglitinides (e.g., repaglinide, nateglinide), sulfonylureas (e.g., chlorpropamide, glimepiride, glipizide, glyburide, tolazamide, tolbutamide), thiazolidinediones also referred to as glitazones (e.g., pioglitazone, rosiglitazone), alpha glucosidase inhibitors (e.g., acarbose, miglitol), dipeptidyl peptidase inhibitors, (e.g., sitagliptin (JANUVIA®), alogliptin, vildagliptin, saxagliptin, linagliptin, dutogliptin, gemigliptin), SGLT2 inhibitors (e.g. sotagliflozin), ergot alkaloids (e.g., bromocriptine), combination medications such as JANUMET® (sitagliptin with metformin), and injectable diabetes medications such as exenatide and pramlintide acetate; phosphodiesterase-5 (PDE5) inhibitors such as sildenafil (Revatio, Viagra), tadalafil (Cialis, Adcirca) vardenafil HCl (Levitra); or with other drugs beneficial for the prevention or the treatment of the above-mentioned diseases including but not limited to diazoxide; and including the free-acid, free-base, and pharmaceutically acceptable salt forms, pro-drug forms (including but not limited to esters), and salts of pro-drugs of the above medicinal agents where chemically possible. Trademark names of pharmaceutical drugs noted above are provided for exemplification of the marketed form of the active agent(s); such pharmaceutical drugs could be used in a separate dosage form for concurrent or sequential administration with a compound of the present invention, or the active agent(s) therein could be used in a fixed dose drug combination including a compound of the present invention.

The compounds of the present invention may be administered by oral, parenteral (e.g., intramuscular, intraperitoneal, intravenous, ICV, intracisternal injection or infusion, subcutaneous injection, or implant), by inhalation spray, nasal, vaginal, rectal, sublingual, or topical routes of administration and may be formulated, alone or together, in suitable dosage unit formulations containing conventional non-toxic pharmaceutically acceptable carriers, adjuvants and vehicles appropriate for each route of administration. In addition to the treatment of warm-blooded animals such as mice, rats, horses, cattle, sheep, dogs, cats, monkeys, etc., the compounds of the invention are effective for use in humans. The terms "administration of" and or "administering a" compound should be understood to mean providing a compound of the invention or a prodrug of a compound of the invention to the individual in need of treatment.

The term "composition" as used herein is intended to encompass a product comprising specified ingredients in predetermined amounts or proportions, as well as any product which results, directly or indirectly, from combination of the specified ingredients in the specified amounts. Such term in relation to pharmaceutical composition, is intended to encompass a product comprising the active ingredient(s), and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or more of the ingredients. In general, pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active object compound is included in an amount sufficient to produce the desired effect upon the process or condition of diseases. Accordingly, the pharmaceutical compositions of the present invention encompass any composition made by mixing a compound of the present invention and a pharmaceutically acceptable carrier.

Pharmaceutical compositions intended for oral use may be prepared according to any method known to the

art for the manufacture of pharmaceutical compositions and such compositions may contain one or more agents selected from sweetening agents, flavoring agents, coloring agents and preserving agents in order to provide pharmaceutically elegant and palatable preparations. Tablets contain the active ingredient in admixture with non-toxic pharmaceutically acceptable excipients that are suitable for the manufacture of tablets. The tablets may be uncoated or they may be coated by known techniques to delay disintegration and absorption in the gastrointestinal tract and thereby provide a sustained action over a longer period. Compositions for oral use may also be presented as hard gelatin capsules wherein the active ingredients are mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil. Aqueous suspensions, oily suspensions, dispersible powders or granules, oil-in-water emulsions, and sterile injectable aqueous or oleagenous suspension may be prepared by standard methods known in the art. By "pharmaceutically acceptable" it is meant the carrier, diluent or excipient must be compatible with the other ingredients of the formulation and not deleterious to the recipient thereof.

The subject compounds may be further useful in a method for the prevention, treatment, control, amelioration, or reduction of risk of the diseases, disorders and conditions noted herein. The dosage of active ingredient in the compositions of this invention may be varied, however, it is necessary that the amount of the active ingredient be such that a suitable dosage form is obtained. The active ingredient may be administered to patients (animals and human) in need of such treatment in dosages that will provide optimal pharmaceutical efficacy. The selected dosage depends upon the desired therapeutic effect, on the route of administration, and on the duration of the treatment. The dose will vary from patient to patient depending upon the nature and severity of disease, the patient's weight, special diets then being followed by a patient, concurrent medication, and other factors which those skilled in the art will recognize. Generally, dosage levels of between 0.001 to 10 mg/kg. of body weight daily are administered to the patient, e.g., humans and elderly humans. The dosage range will generally be about 0.5 mg to 100 mg per patient per day which may be administered in single or multiple doses. In one embodiment, the dosage range will be about 0.5 mg to 50 mg per patient per day; in another embodiment about 0.5 mg to 20 mg per patient per day; and in yet another embodiment about 0.5 mg to 5 mg per patient per day. Pharmaceutical compositions of the present invention may be provided in a solid dosage formulation such as comprising about 0.5 mg to 100 mg active ingredient, or comprising about 0.5 mg to 50 mg active ingredient. The pharmaceutical composition may be provided in a solid dosage formulation comprising about 0.5 mg, 1 mg, 5 mg, 10 mg, 25 mg, 50 mg, or 100 mg active ingredient. For oral administration, the compositions may be provided in the form of tablets containing 0.5 to 100 milligrams of the active ingredient, such as 0.5, 1, 5, 10, 15, 20, 25, 50, 75, and 100 milligrams of the active ingredient for the symptomatic adjustment of the dosage to the patient to be treated. The compounds may be administered on a regimen of 1 to 4 times per day, such as once or twice per day.

Several methods for preparing the compounds of this invention are illustrated in the following Schemes and Examples. Starting materials and the requisite intermediates are in some cases commercially available, or can be prepared according to literature procedures or as illustrated herein. The compounds of this invention may be prepared by employing reactions as shown in the following schemes, in addition to other standard manipulations that are known in the literature or exemplified in the experimental procedures. Substituent numbering as shown in the schemes does not necessarily correlate to that used in the claims and often, for clarity, a single substituent is shown attached to the compound where multiple substituents are allowed under the definitions hereinabove. Reactions used to generate the compounds of this invention are prepared by employing reactions as shown in the schemes and examples herein, in addition to other standard manipulations such as ester hydrolysis, cleavage of protecting groups, etc., as may be known in the literature or exemplified in the experimental procedures. Starting materials are made according to procedures known in the art or as illustrated herein. The following abbreviations are used herein: Me: methyl; Et: ethyl; t-Bu: tert-butyl; Ar: aryl; Ph: phenyl; Bn: benzyl; Ac: acetyl; THF: tetrahydrofuran; Boc: tert-butyloxycarbonyl; DIEA or DIPEA: A, A-diisopropylethylamine; DPPA: diphenylphosphorylazide; EDC: *N*-(3-Dimethylaminopropyl)-N'-ethylcarbodiimide; EtOAc: ethyl acetate; HOBt: hydroxybenzotriazole hydrate; TEA: triethylamine; DMF: *N*,*N*-dimethylformamide; rt: room temperature; HPLC: high performance liquid chromatography; NMR: nuclear magnetic resonance; TLC: thin-layer chromatography; 18-crown-6: 1,4,7,10,13,16- hexaoxacyclooctadecane; CAN: acetonitrile; BAST: bis(2-methoxyethyl)aminosulfur trifluoride; DAST: diethylaminosulfurtrifluoride; DCE: dichloroethane; DCM: dichloromethane; DEA: diethylamine; DIAD: diethyl azodicarboxylate; DME: 1,2-dimethoxyethane; DMF: N,N-dimethylformamide; DMPU: 1,3-dimethyl-Tetrahydropyrimidin-2 (1H)-one; DMSO: dimethyl sulfoxide; Hex: hexane; HATU: 1-[bis(dimethylamino)methylene]-1H-1,2,3-triazolo[4,5-b]pyridinium-3-oxidhexafluorophosphate; LDA: lithium diisopropylamide; LiHMDS: lithium bis(trimethylsilyl)amide; m-CPBA: m-chloroperoxybenzoic acid; MTBE: methyl tert-butyl ether; NMM: N-methylmorpholine; NMP: N-methyl pyrrolidine; py: pyridine; TBAI: tetrabutylammonium iodide; TFA: trifluoroacetic acid; THF: tetrahydrofuran: TMEDA: tetramethylethylenediamine; ttbtpy: 4,4',4"-tri-tert-butyl-2,2',6',2"-terpyridine, VCD: vibrational circular dichroism.

The compounds of the present invention can be prepared in a variety of fashions. In some cases the final product may be further modified, for example, by manipulation of substituents. These manipulations may include, but are not limited to, reduction, oxidation, alkylation, acylation, and hydrolysis reactions which are commonly known to

those skilled in the art. In some cases the order of carrying out the foregoing reaction schemes may be varied to facilitate the reaction or to avoid unwanted reaction products. Because the schemes are an illustration, the invention should not be construed as being limited by the chemical reactions and conditions expressed. The preparation of the various starting materials used herein is well within the skill of a person versed in the art. The following examples are provided so that the invention might be more fully understood. These examples are illustrative only and should not be construed as limiting the invention in any way. Absolute stereochemistry of separate stereoisomers in the examples and intermediates are not determined unless stated otherwise in an example or explicitly in the nomenclature.

SCHEME 1

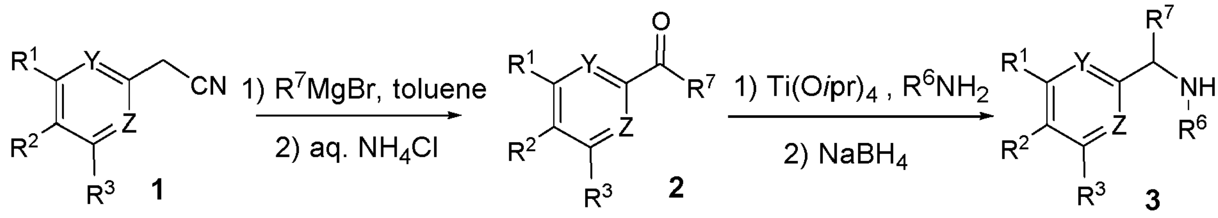


Amines of the general formula **3** are prepared as described in Scheme 1. Carbonitriles such as **1** are reacted with $R^7MgBr$ in a solvent such as toluene followed by quench with aq. $NH_4Cl$ to provide ketones such as **2.** The ketone **2** is then reacted with $Ti(Oipr)_4$, $R^6NH_2$ and $NaBH_4$ to afford amines of the general formula **3.**

SCHEME 2

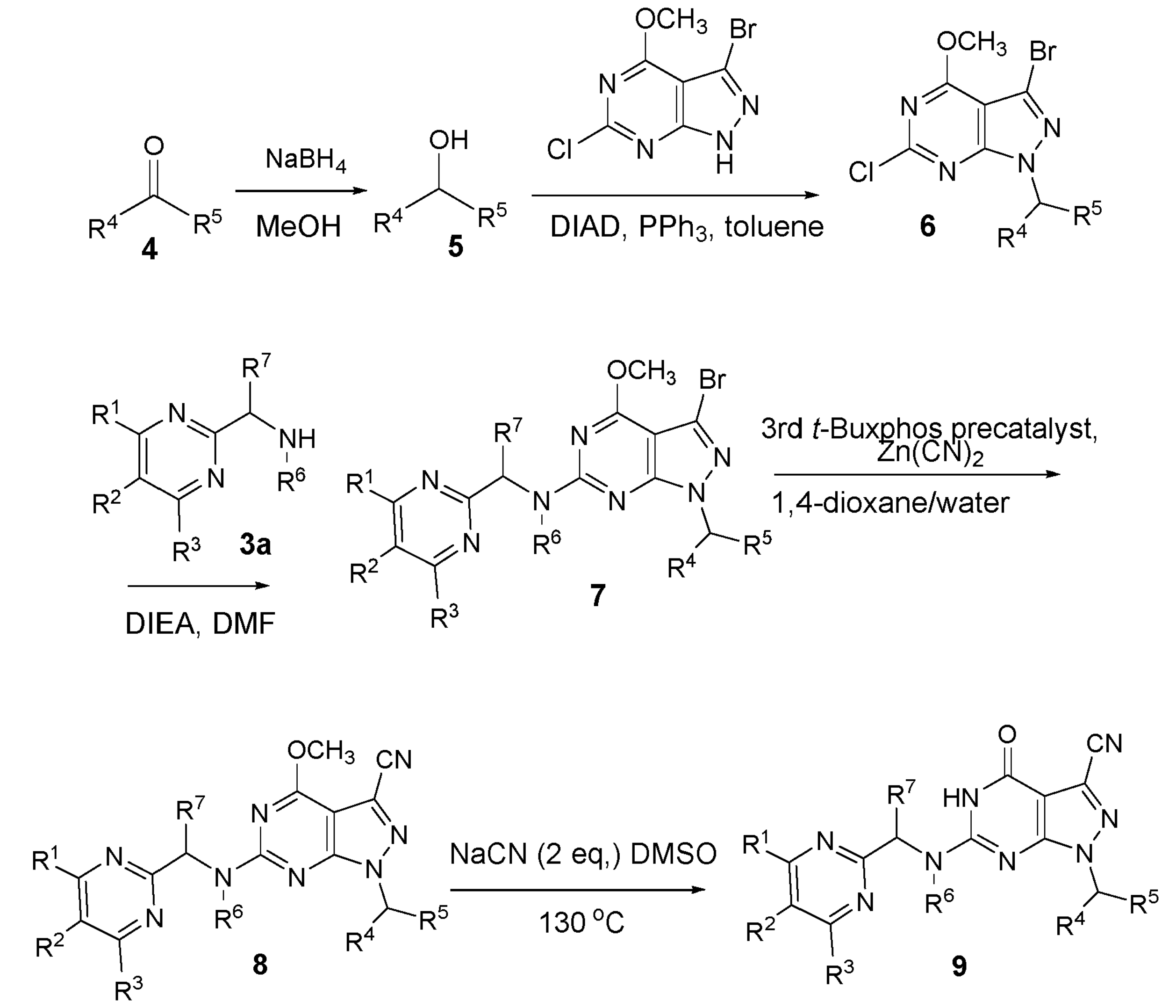


Scheme 2 illustrates the formation of oxo-pyrimidine-carbonitriles such as **9.** Ketones **4** is reduced to alcohol **5** using NaBH$_4$. The alcohol 5 is then condensed with pyrazolo[3,4-d]pyrimidine to afford 3-bromo-6-chloro-4-methoxy-1*H*-pyrazolo[3,4-*d*]pyrimidine such as **6,** which is then combined with amines such as 3a and DIEA in DMF to provide 3-bromo-4-methoxy-1*H*-pyrazolo [3,4-d]pyrimidine such as **7.** The 3-bromo-4-methoxy-1*H*-pyrazolo [3,4-d]pyrimidine **7** is then converted to pyrimidine-3-carbonitrile such as **8,** which is deprotected to afford compounds of general formua **9.**

## SCHEME 3

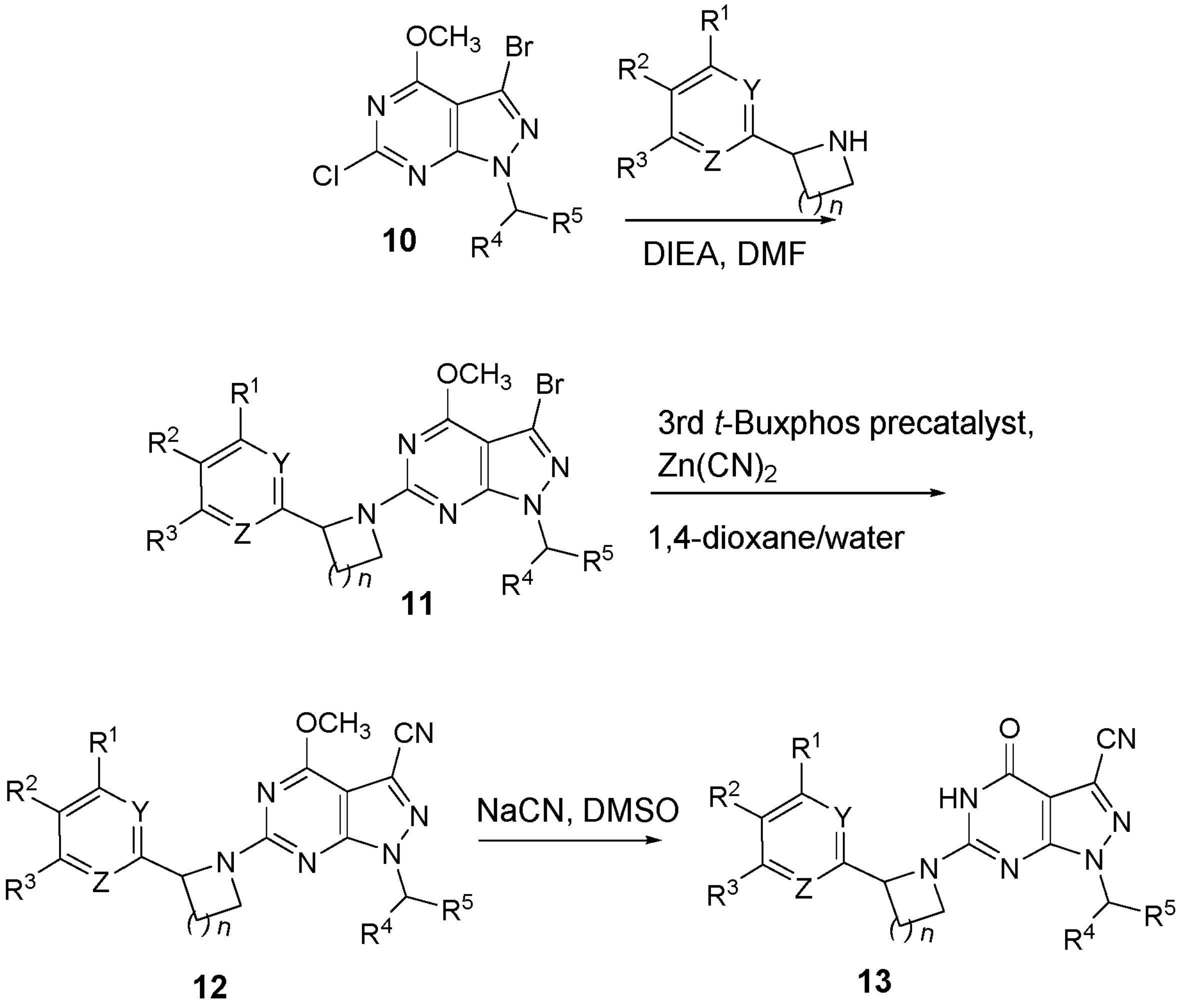


10

11

12

13

Scheme 3 illustrates the formation of compounds of general formula **13.** Reaction of 10 with an amine and DIEA in DMF provides adduct **11** which is then converted to carbonitril **12.** Deprotection of **12** affords compounds of general formula **13,** including 4-oxo-1-(1-(6-(trifluoromethyl)pyridin-3-yl)ethyl)-4,5-dihydro-1*H*-pyrazolo[3,4-*d*]pyrimidine-3-carbonitrile such as **13.**

## INTERMEDIATE 1

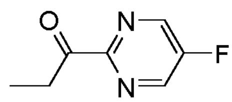

1-(5-Fluoropyrimidin-2-yl)propan-1-one

To a solution of 5-fluoropyrimidine-2-carbonitrile (1.00 g, 8.12 mmol) in THF (8 mL) was added ethylmagnesium bromide (2M in ether; 5.08 mL, 10.16 mmol) at -40 °C under $N_2$. The reaction mixture was stirred at -40 °C for 2 h. The reaction mixture was warmed to -25 °C before adding aq. HCl (4N; 6.09 mL, 24.37 mmol) and stirred for 30 min. The phases were separated, and the aqueous phase was extracted further with EtOAc (3 × 20 mL). The combined organic layers were washed with brine (3 × 10 mL), dried over anhydrous $Na_2SO_4$ and filtered. The filtrate was concentrated under vacuum. The residue was purified by a silica gel column chromatography, eluted with gradient 0%-20% EtOAc in petroleum ether. The fractions containing desired product were combined and concentrated under vacuum to afford 1-(5-fluoropyrimidin-2-yl)propan-1-one. LCMS (ES, *m*/*z*):155.2 $[M+H]^+$.

TABLE 1

| The following compounds were prepared according to the general procedure provided in the examples and procedures herein using known or prepared starting materials, as described in the reaction schemes and examples herein, such as described for Intermediate 1, using appropriate starting materials and purification conditions. | | |
|---|---|---|
| Intermediate # | Structure | Exact Mass [M+1]+ |
| 2 | 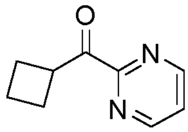 | 163.1 |
| 3 | 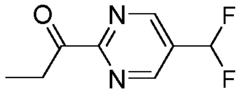 | 187.1 |
| 4 | 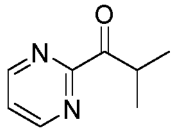 | 151.1 |
| 5 | 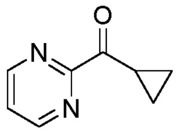 | 149.1 |
| 6 | 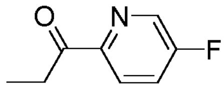 | 154.1 |

## INTERMEDIATE 7

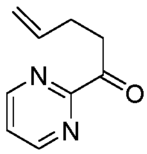

1 -(Pyrimidin-2-yl)pent-4-en-1 -one

To a solution of pyrimidine-2-carbonitrile (2.10 g, 19.98 mmol) in toluene (20 mL) was added (cyclopropylmethyl)magnesium bromide (2M in THF; 11.99 mL, 23.98 mmol) at -78 °C. The reaction solution was degassed with $N_2$ 3 times and stirred at -78 °C for 3 h. Then aq. $NH_4Cl$ (sat.; 100 mL) was added dropwise to quench the reaction mixture followed by additional stirring at -20 °C for 1 h. The resulting mixture was extracted with EtOAc (3 × 100 mL). The combined organic layers were washed with brine (3 × 10 mL), dried over anhydrous $Na_2SO_4$ and filtered. The filtrate was concentrated under vacuum. The residue was purified by a silica gel column chromatography, eluted with gradient 0-60% EtOAc in petroleum ether. The fractions containing desired product were combined and concentrated under vacuum to afford 1-(pyrimidin-2-yl)pent-4-en-1-one. LCMS (ES, *m*/*z*): 163.1 $[M+H]^+$.

## INTERMEDIATE 8

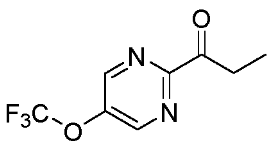

1-(5-(Trifluoromethoxy)pyrimidin-2-yl)propan-1-one

Step A: O-(2-bromopyrimidin-5-yl)-5-ethyl carbonodithioate. To a solution of 2-bromopyrimidin-5-ol (25.00 g, 143.00 mmol) in 5% sodium hydroxide (175 mL, 143 mmol) solution was added thiophosgene (11.50 mL, 143.00 mmol) in $CHCl_3$ (75 mL) over 3 minutes at 0 °C. The reaction mixture was then stirred at 0 °C for an additional 2 hours before being extracted using $CHCl_3$ (3 × 20 mL). The organic phase was washed with HCl (1N, 5 mL) and $H_2O$ (2 × 5 mL). All the organic phases were collected, dried over $Na_2SO_4$. The filtrate was then added to sodium ethanethiolate (13.22 g, 157.00 mmol) at 0 °C and stirred overnight. The suspension was then filtered through celite, and the filtrate was then concentrated under vacuum to get the crude as a red oil. The residue was purified by silica gel column chromatography, eluted with gradient 0-10% EtOAc in petroleum ether. The fractions containing desired product were combined and concentrated under vacuum to afford O-(2-bromopyrimidin-5-yl) S-ethyl carbonodithioate. LCMS (ES, *m*/*z*): 279.0 $[M+H]^+$.

Step B: 2-Bromo-5-(trifluoromethoxy)pyrimidine. To a solution of 1,3-dibromo-5,5-dimethylimidazolidine-2,4-dione (23.04 g, 81.00 mmol) in DCM (500 mL) at -78 °C was added dropwise pyridine hydrofluoride (70%, 200 mL, 17.91 mmol) solution. The whole mixture was then stirred at -78 °C for 30 minutes before adding O-(2-bromopyrimidin-5-yl) S-ethyl carbonodithioate (5.00 g, 17.91 mmol) in DCM (50 mL). The reaction mixture was kept at -5 °C and stirred for 2 hours. EtOAc (70 mL) was added to dilute the mixture before quenching it with cold aq. $NaHCO_3$ (500 mL) and $NaHSO_4$ (750 mL) until the red color disappeared. The pH was adjusted at 0 °C to 10 using aq. NaOH (5N, 400 mL). The reaction mixture was then extracted using EA (3 × 150 mL). All the organic phases were collected, dried over $Na_2SO_4$, and concentrated under vacuum. The residue was purified by silica gel column chromatography, eluted with gradient 0-10% EtOAc in petroleum ether. The fractions containing desired product were combined and concentrated under vacuum to afford 2-bromo-5-(trifluoromethoxy)pyrimidine.

Step C: 5-(Trifluoromethoxy)pyrimidine-2-carbonitrile. To a solution of 2-bromo-5-(trifluoromethoxy)pyrimidine (0.50 g, 2.06 mmol) in DMF (0.5 mL) were added dicyanozinc (0.50 g, 4.12 mmol), $Pd_2(dba)_3CHCl_3$ (85.00 mg, 0.082 mmol), and dppf (136 mg, 0.185 mmol). The reaction mixture was degassed with $N_2$ for 3 times and stirred at 120 °C for 2 hours. The resulting mixture was diluted with water (5 mL) and extracted with EA (3 × 3 mL). The combined organic layers were washed with water (3 × 2 mL), dried over $Na_2SO_4$, and concentrated under vacuum. The residue was purified by a silica gel column chromatography, eluted with gradient 0-10% EtOAc in petroleum ether. The fractions containing desired product were combined and concentrated under vacuum to afford 5-(trifluoromethoxy)pyrimidine-2-carbonitrile. $^1H$ NMR (400 MHz, $CDCl_3$) δ 8.79 (s, 2H). Step D: 1-(5-(Trifluoromethoxy)pyrimidin-2-yl)propan-1-one. The title compound was prepared as described for Intermediate 1 step A using 5-(trifluoromethoxy)pyrimidine-2-carbonitrile. LCMS (ES, *m*/*z*): 221.2 $[M+H]^+$.

INTERMEDIATE 9

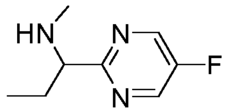

1-(5-Fluoropyrimidin-2-yl)-N-methylpropan-1-amine

To a solution of methylamine in MeOH (2M; 10.2 mL, 20.44 mmol) was added titanium(IV) isopropoxide (8.78 mL, 30.0 mmol) with ice bath cooling followed by the addition of 1-(5-fluoropyrimidin-2-yl)propan-1-one (3.50 g, 22.71 mmol) under $N_2$. The reaction mixture was stirred at room temperature for 5 h. $NaBH_4$ (0.86 g, 22.71 mmol) was added to the reaction mixture and the resulting mixture was stirred for a period of 2 h. The reaction was quenched by the addition of water (4.4 mL). The resulting mixture was concentrated under vacuum. The residue was purified by $C_{18}$ reverse phase column with gradient 1%-50% ACN in water as eluent. The fractions that contained the desired product were collected and concentrated under vacuum to afford 1-(5-fluoropyrimidin-2-yl)-*N*-methylpropan-1-amine. LCMS (ES, *m*/*z*): 170.2 $[M+H]^+$.

TABLE 2

The following compounds were prepared according to the general procedure provided in the examples and procedures herein using known or prepared starting materials, as described in the reaction schemes and examples herein using appropriate starting materials and purification conditions.

| Interemediate # | Structure | Exact Mass [M+1]$^+$ |
|---|---|---|
| 10 | 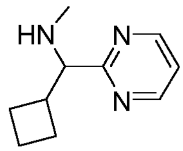 | 178.1 |
| 11 | 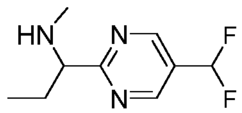 | 202.1 |
| 12 | 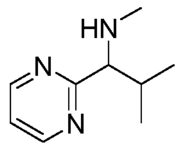 | 166.1 |
| 13 | 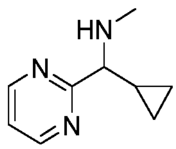 | 164.1 |
| 14 | 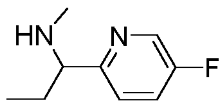 | 169.1 |
| | | |
| 15 | 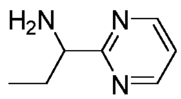 | 138.1 |
| 16 | 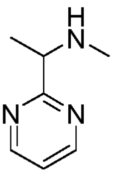 | 138.1 |
| 17 | 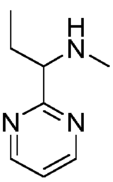 | 152.1 |
| 18 | 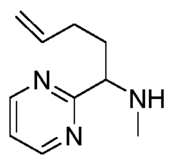 | 178.1 |

## INTERMEDIATE 19

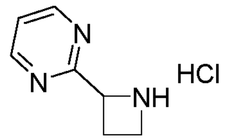

2-(Azetidin-2-yl)pyrimidine hydrochloride

Step A: 1-(Tert-butoxycarbonyl)azetidine-2-carboxylic acid. To a solution of azetidine-2-carboxylic acid (25.0 g, 247 mmol) in EtOH (500 mL) and water (250 mL) was added di-tert-butyl dicarbonate (71.20 g, 326 mmol). The reaction flask was cooled to 0 °C, prior to addition of NaOH (10.48 g, 262 mmol). The reaction mixture was warmed to room temperature and stirred overnight. The pH of the reaction mixture was adjusted to 3 by aq. HCl (2N). The resulting mixture was extracted with $Et_2O$ (3 × 500 mL). The combined organic layer was washed with brine (3 × 500 mL), dried over anhydrous $MgSO_4$ and filtered. The filtrate was concentrated under vacuum to afford 1-(*tert*-butoxycarbonyl)azetidine-2-carboxylic acid (49.8 g, 246 mmol) which was directly used for next step without further purification . ES, *m*/*z*): 202.1[M+H]$^+$.

Step B: Tert-butyl 2-carbamoylazetidine-1-carboxylate. To a solution of 1-(*tert*-butoxycarbonyl)azetidine-2-carboxylic acid (25.0 g, 124 mmol) in DME (250 mL) was added 4-methylmorpholine (12.57 g, 124 mmol) dropwise with ice bath cooling. The reaction mixture was stirred for 10 minutes at 0 °C. Isobutyl carbonochloridate (16.97 g, 124 mmol) was added and stirred for another 10 minutes followed by the addition of ammonia hydrate (174.0 g, 124 mmol). The reaction mixture was warmed to room temperature and stirred for 16 h overnight. EtOAc (50 mL) and water (75 mL) were added and the reaction mixture was stirred at room temperature for 15 minutes. The resulting mixture was extracted with EtOAc (3 × 500 mL). The combined organic layers were washed with brine (3 × 500 mL), dried over anhydrous $Na_2SO_4$ and filtered. The filtrate was concentrated under vacuum to afford tert-butyl 2-carbamoylazetidine-1-carboxylate which was directly used for next step without further purification . LCMS (ES, *m*/*z*): 201.1[M+H]$^+$.

Step C: Tert-butyl 2-carbamimidoylazetidine-1-carboxylate. To a solution of tert-butyl 2-carbamoylazetidine-1-carboxylate (10.0 g, 49.9 mmol) in DCM (230 mL) was added trimethyloxonium tetrafluoroborate (7.39 g, 49.9 mmol) under $N_2$. The reaction solution was degassed with $N_2$ 3 times and stirred at room temperature for 3 h. Ammonia (125 mL, 874 mmol) in MeOH solution was added to the reaction mixture and it was stirred at room temperature for 16 h under $N_2$. The resulting solution was concentrated under vacuum to afford tert-butyl 2-carbamimidoylazetidine-1-carboxylate which was directly used for next step without further purification. LCMS (ES, *m*/*z*): 200.1[M+H]$^+$.

Step D: Tert-butyl 2-(pyrimidin-2-yl)azetidine-1-carboxylate. To a solution of *tert*-butyl 2-carbamimidoylazetidine-1-carboxylate (4.90 g, 24.59 mmol) in DMA (10 mL) was added 1,1,3,3-tetramethoxypropane (12.11 g, 73.8 mmol) at room temperature. The reaction mixture was stirred at 90 °C for 16 h. The resulting mixture was concentrated under vacuum. The resulting solution was extracted with EtOAc (3 × 100 mL). The combined organic layer was washed with brine (3 × 100 mL), dried over anhydrous $Na_2SO_4$ and filtered. The filtrate was concentrated under vacuum. The residue was purified by silica gel column chromatography, eluted with gradient 0%-90% EtOAc in petroleum ether. The fractions containing desired product were combined and concentrated under vacuum to afford tert-butyl 2-(pyrimidin-2-yl)azetidine-1-carboxylate. LCMS (ES, *m*/*z*): 236.1[M+H]$^+$.

Step E: 2-(Azetidin-2-yl)pyrimidine hydrochloride. To a solution of tert-butyl 2-(pyrimidin-2-yl)azetidine-1-carboxylate (0.45 g, 1.91 mmol) in 1,4-dioxane (4 mL) was added HCl in 1,4-dioxane (4 M; 4 mL) at room temperature. The reaction mixture was stirred at room temperature for 3 h. The resulting mixture was concentrated under vacuum to afford 2-(azetidin-2-yl)pyrimidine hydrochloride (0.35 g) as a yellow solid which was directly used for next step without further purification. LCMS (ES, *m*/*z*): 136.1[M+H]$^+$.

The following compounds were prepared according to the general procedure provided in the examples and procedures herein using known or prepared starting materials, as described in the reaction schemes and examples herein using appropriate starting materials and purification conditions.

| Structure | Literature Reference |
|---|---|
| 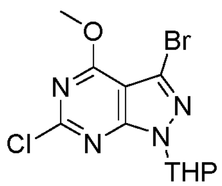 | PCT Int. Patent Appl., WO2016/116752 (28Jul2016) |

(continued)

| Structure | Literature Reference |
|---|---|
| 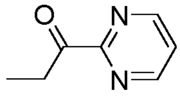 | PCT Int. Patent Appl., WO2014/120800 (07Aug2014) |
| 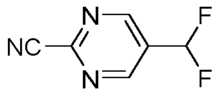 | U.S. Pat. Appl. Publ., US2015/0284341 (08Oct2015) |
| 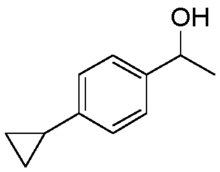 | Russian Journal of Organic Chemistry, 52(4), 503-512; 2016 |

EXAMPLES 1-4

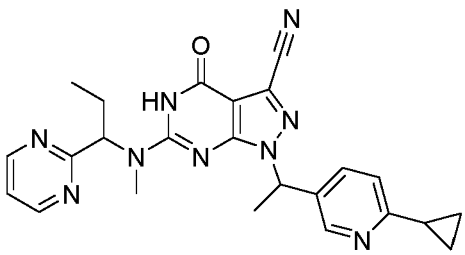

1-(1-(6-Cyclopropylpyridin-3-yl)ethyl)-6-(methyl(1-(pyrimidin-2-yl)propyl)amino)-4-oxo-4,5-dihydro-1*H*-pyrazolo[3,4-*d*]pyrimidine-3-carbonitrile

Step A: 3-Bromo-6-chloro-4-methoxy-1*H*-pyrazolo[3,4-*d*]pyrimidine. To a solution of 3-bromo-6-chloro-4-methoxy-1-(tetrahydro-2*H*-pyran-2-yl)-1*H*-pyrazolo[3,4-*d*]pyrimidine (0.20 g, 0.58 mmol) (PCT Int. Appl., 2016116752, 28 Jul 2016) in 1,4-dioxane (6 mL) was added HCl in 1,4-dioxane (4 M; 0.28 mL). The reaction mixture was stirred at 20 °C for 4 h, and concentrated in vacuum. The residue was purified by a silica gel column chromatography, eluted with gradient 0 - 11% EtOAc in petroleum ether. The fractions containing desired product were combined and concentrated under reduced pressure to afford 3-bromo-6-chloro-4-methoxy- 1*H*-pyrazolo[3,4-d]pyrimidine which was directly used for next step without further purification. LCMS (ES, m/z): 262.9, 264.9 $[M+H]^+$.

Step B: 1-(6-Cyclopropylpyridin-3-yl)ethanol. To a solution of 1-(6-cyclopropylpyridin-3-yl)ethanone (0.80 g, 4.96 mmol) in MeOH (8 mL) was slowly added $NaBH_4$ (0.20 g, 5.46 mmol). The reaction mixture was stirred at room temperature for 2 h. The reaction was quenched by water (20 mL), and extracted with EtOAc (3 × 20 mL). The combined organic layer was washed with brine (3 × 50 mL), dried over anhydrous $Na_2SO_4$ and filtered. The filtrate was concentrated under vacuum. The residue was purified by a silica gel column chromatography, eluted with gradient 20%-50% EtOAc in petroleum ether. The fractions containing desired product were combined and concentrated under reduced pressure to afford 1-(6-cyclopropylpyridin-3-yl) ethanol. LCMS (ES, *m/z):* 164.2$[M+H]^+$.

Step C: 3-Bromo-6-chloro-1-(1-(6-cyclopropylpyridin-3-yl)ethyl)-4-methoxy-1*H*-pyrazolo[3,4-d]pyrimidine. To a solution of 1-(6-cyclopropylpyridin-3-yl)ethanol (0.62 g, 3.80 mmol) in toluene (20 mL) were added 3-bromo-6-chloro-4-methoxy-1*H*-pyrazolo[3,4-*d*] pyrimidine (1.00 g, 3.80 mmol) and triphenylphosphine (2.49 g, 9.50 mmol). The reaction mixture was cooled to 0 °C, and DIAD (1.51 mL, 7.60 mmol) was added dropwise. The reaction mixture was then warmed to room temperature, and stirred at room temperature for 3 h. Water (50 mL) was added, and the mixture was extracted with EtOAc (3 × 20 mL). The combined organic layer was washed with brine (3 × 50 mL), dried over anhydrous $Na_2SO_4$ and filtered. The filtrate was concentrated under reduced pressure. The residue was purified by a silica gel column chromatography, eluted with gradient 5% - 20% EtOAc in petroleum ether. The fractions containing desired product

were combined and concentrated under vacuum to afford 3-bromo-6-chloro-1-(1-(6-cyclopropylpyridin-3-yl)ethyl)-4-methoxy-1*H*-pyrazolo[3,4- d]pyrimidine. LCMS (ES, *m*/*z*): 408.0, 410.0 [M+H]$^+$.

Step D: 3-Bromo-1-(1-(6-cyclopropylpyridin-3-yl)ethyl)-4-methoxy-*N*-methyl-*N*-(1-(pyrimidin-2- yl)propyl)-1*H*-pyrazolo[3,4-*d*]pyrimidin-6-amine. To a solution of 3-bromo-6-chloro-1-(1-(6-cyclopropylpyridin-3-yl)ethyl)-4-methoxy-1*H*-pyrazolo[3,4-d]pyrimidine (0.40 g, 0.98 mmol) in DMF (4 mL) were added *N*-methyl-1-(pyrimidin-2-yl)propan- 1-amine dihydrochloride (0.44 g, 1.96 mmol) and N-ethyl-N-isopropylpropan-2-amine (1.27 g, 9.79 mmol). The reaction mixture was stirred at 90 °C for 2 h. Water (20 mL) was added, and the reaction mixture was extracted with EtOAc (3 × 50 mL). The combined organic layers was washed with brine (3 × 100 mL), dried over anhydrous $Na_2SO_4$, and concentrated under reduced pressure. The residue was purified by a silica gel column chromatography, eluted with gradient 10% - 30% EtOAc in petroleum ether. The fractions containing desired product were combined and concentrated under reduced pressure to afford 3-bromo-1-(1-(6-cyclopropylpyridin-3-yl)ethyl) -4-methoxy-N-methyl-N-(1-(pyrimidin-2-yl)propyl)-1*H*-pyrazolo[3,4-*d*]pyrimidin-6-amine. LCMS (ES, *m*/*z*): 523.2, 525.2 [M+H]$^+$.

Step E: 1-(1-(6-Cyclopropylpyridin-3-yl)ethyl)-4-methoxy-6-(methyl(1-(pyrimidin-2-yl)propyl)amino)-1*H*-pyrazolo[3,4-*d*]pyrimidine-3-carbonitrile. To a solution of 3-bromo-1-(1-(6-cyclopropylpyridin-3-yl)ethyl)-4-methoxy-N-methyl -N-(1-(pyrimidin-2- yl)propyl)-1*H*-pyrazolo[3,4-d]pyrimidin-6-amine (0.25 g, 0.48 mmol) in 1, 4-dioxane (3 mL) and water (0.6 mL) were added t-Bu-Brettphos Pd $G_3$ (65.60 mg, 0.10 mmol) and dicyanozinc (0.11 g, 0.96 mmol). The reaction mixture was degassed with $N_2$ for 3 times and stirred at 55 °C for 2 days under $N_2$. After cooling to room temperature, the resulting mixture was diluted with aq. $NaHCO_3$ (sat.; 20 mL) and extracted with EtOAc (3 × 20 mL). The combined organic layers were washed with sat. $FeSO_4$ (3 × 20 mL), dried over anhydrous $Na_2SO_4$ and filtered. The filtrate was concentrated under reduced pressure. The residue was purified by a silica gel column chromatography, eluted with gradient 10%-30% EtOAc in petroleum ether. The fractions containing desired product were combined and concentrated under reduced pressure to afford 1-(1-(6-cyclopropylpyridin-3-yl)ethyl)-4-methoxy-6-(methyl(1-(pyrimidin -2-yl)propyl)amino)-1*H*-pyrazolo[3,4-*d*]pyrimidine-3-carbonitrile. LCMS (ES, *m*/*z*): 470.4 [M+H]$^+$.

Step F: 1-(1-(6-cyclopropylpyridin-3-yl)ethyl)-6-(methyl(1-(pyrimidin-2-yl)propyl)amino)- 4-oxo-4,5-dihydro-1*H*-pyrazolo[3,4-*d*]pyrimidine-3-carbonitrile. To a solution of 1-(1-(6-cyclopropylpyridin-3-yl)ethyl)-4-methoxy-6-(methyl(1- (pyrimidin-2-yl)propyl)amino)- 1H-pyrazolo[3,4-d]pyrimidine-3-carbonitrile (0.20 g, 0.43 mmol) in DMSO (2 mL) was added cyanosodium (41.7 mg, 0.85 mmol), and the mixture was stirred at 130 °C for 1 h. The resulting mixture was filtered and the filtrate was purified by Prep-HPLC with the following conditions: column: X Select CSH Prep C18 OBD Column, 19 × 250 mm, 5 μm; Mobile Phase A: water with 0.05% TFA, Mobile Phase B: ACN; Flow rate: 25 mL/min; Gradient: 19% B to 30% B in 12 min; 254/210 nm; $Rt_1$: 10.63 min (mixture A); $Rt_2$: 11.22 min (mixture B). The desired fractions were combined and concentrated under reduced pressure to afford the title compounds as two mixtures of isomers. From mixture A Examples 1 & 2 were separated by Prep-Chiral HPLC: Column: CHIRALPAK IE, 2 × 25 cm, 5 μm; Mobile Phase A: Hex, Mobile Phase B: EtOH; Flow rate: 16 mL/min; Gradient: 50% B to 50% B in 25 min; 220/254 nm; $RT_1$:13.29 min; $RT_2$:16.76 min. The faster-eluting isomer was obtained at 13.29 min. The collected fractions were combined and concentrated under reduced pressure to afford Example 1: $^1$H NMR (300 MHz, $CDCl_3$) δ 10.76 (*brs,* 1H), 8.75 (d, *J*= 4.8 Hz, 2H), 8.43 (d, *J*= 2.1 Hz, 1H), 7.57 (dd, *J*= 8.1 Hz, 2.1 Hz, 1H), 7.27-7.23 (m, 1H), 7.06 (d, *J* = 8.1 Hz, 1H), 5.82 (q, *J* = 6.9 Hz, 1H), 5.73-5.60 (m, 1H), 3.15 (s, 3H), 2.44-2.31 (m, 1H), 2.13-1.95 (m, 2H), 1.88 (d, *J*= 7.2 Hz, 3H), 1.06-0.99 (m, 7H). LCMS (ES, *m*/*z*): 456.2[M+H]$^+$. The slower-eluting isomer was obtained at 16.76 min. The collected fractions were combined and concentrated under reduced pressure to afford Example 2: $^1$H NMR (300 MHz, $CDCl_3$) δ10.52 (*brs,* 1H), 8.75 (d, *J*= 4.8 Hz, 2H), 8.43 (d, *J* = 2.1 Hz, 1H), 7.59 (dd, *J*= 8.1 Hz, 2.1 Hz, 1H), 7.28-7.24 (m, 1H), 7.06 (d, *J* = 8.1 Hz, 1H), 5.83 (q, *J* = 7.2 Hz, 1H), 5.73-5.60 (m, 1H), 3.15 (s, 3H), 2.43-2.30 (m, 1H), 2.15-1.95 (m, 2H), 1.88 (d, *J*= 7.2 Hz, 3H), 1.00-0.99 (m, 7H). LCMS (ES, *m*/*z*): 456.2 [M+H]$^+$. From mixture B examples 3 & 4 were separated by prep-chiral HPLC: column: CHIRALPAK IE, 2 × 25 cm, 5 μm; Mobile Phase A: Hex with 0.2% IPA, Mobile Phase B: EtOH; Flow rate: 18 mL/min; Gradient: 35% B to 35% B in 36 min; 220/254 nm; $Rt_1$:19.88 min; $Rt_2$:23.60 min. The faster-eluting isomer was obtained at 19.88 min. The collected fractions were combined and concentrated under reduced pressure to afford Example 3: $^1$H NMR (300 MHz, $CDCl_3$) δ10.63 (*brs,* 1H), 8.74 (d, *J*= 4.8 Hz, 2H), 8.39 (d, *J*= 2.1 Hz, 1H), 7.57 (dd, *J* = 6.9 Hz, 2.7 Hz, 1H), 7.27-7.22 (m, 1H), 7.04 (d, *J* = 8.1 Hz, 1H), 5.84 (q, *J* = 7.2 Hz, 1H), 5.60-5.48 (m, 1H), 3.23 (s, 3H), 2.40-2.28 (m, 1H), 2.16-2.04 (m, 2H), 1.88 (d, *J*= 7.2 Hz, 3H), 1.06-0.92 (m, 7H). LCMS (ES, *m*/*z*): 456.2 [M+H]$^+$. The slower-eluting isomer was obtained at 23.60 min. The collected fractions were combined and concentrated under reduced pressure to afford Example 4: $^1$H NMR (300 MHz, $CDCl_3$) δ10.59 (*brs,* 1H), 8.73 (d, *J*= 4.8 Hz, 2H), 8.37 (d, *J*= 1.8 Hz, 1H), 7.55 (dd, *J*= 8.4 Hz, 2.1 Hz, 1H), 7.27-7.22 (m, 1H), 7.02 (d, *J*= 8.1 Hz, 1H), 5.83 (q, *J*= 7.2 Hz, 1H), 5.60-5.48 (m, 1H), 3.22 (s, 3H), 2.40-2.28 (m, 1H), 2.16-1.93 (m, 2H), 1.88 (d, *J*= 7.2 Hz, 3H), 1.06-0.88 (m, 7H). LCMS (ES, *m*/*z*): 456.2[M+H]$^+$.

TABLE 3

The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation.

| Ex | Structure | Name | LCMS $[M+1]^+$ | Prep-HPLC Column; Peak # | Prep CHIRAL HPLC Column; Peak # |
|---|---|---|---|---|---|
| 5 | 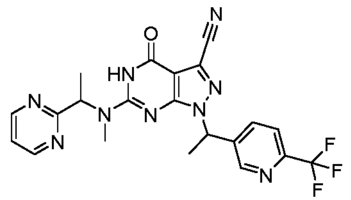 | 6-[methyl(1-pyrimidin-2-ylethyl)amino] -4-oxo-1-[1-[6-(trifluoromethy l)-3-pyridyl] ethyl] - 5H-pyrazolo[3,4-d] pyrimidine-3-carbonitrile | 470.3 | (X Bridge C18 OBD 19 × 250 mm; A:water(20 mmol/L $NH_4HCO_3$), Mobile Phase B: ACN; Flow rate: 20 mL/min; Gradient: 35% B to 65% B in 9 min); 1 | (CHIRAL ART Cellulose-SB S-5um, 2 x 25 cm, 5 μm, 20 % EtOH in Hex); 1 |
| 6 | 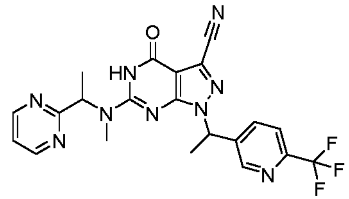 | 6-[methyl(1-pyrimidin-2-ylethyl)amino] -4-oxo-1-[1-[6-(trifluoromethy l)-3-pyridyl] ethyl]- 5H-pyrazolo[3,4-d] pyrimidine-3-carbonitrile | 470.3 | (X Bridge C 18 OBD 19 x 250 mm; A:water(20 mmol/L $NH_4HCO_3$), Mobile Phase B: ACN; Flow rate: 20 mL/min; Gradient: 35% B to 65% B in 9 min); 1 | (CHIRAL ART Cellulose-SB S-5um, 2 x 25 cm, 5 μm, 20 % EtOH in Hex); 2 |
| 7 | 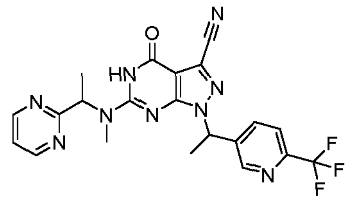 | 6-[methyl(1-pyrimidin-2-ylethyl)amino] -4-oxo-1-[1-[6-(trifluoromethy l)-3-pyridyl] ethyl]- 5H-pyrazolo[3,4-d] pyrimidine-3-carbonitrile | 470.3 | (X Bridge C18 OBD 19 x 250 mm; A:water(20 mmol/L $NH_4HCO_3$), Mobile Phase B: ACN; Flow rate: 20 mL/min; Gradient: 35% B to 65% B in 9 min); 2 | (CHIRALPA K IC, 2 x 25 cm, 5 μm, 35% EtOH in Hex); 1 |
| 8 | 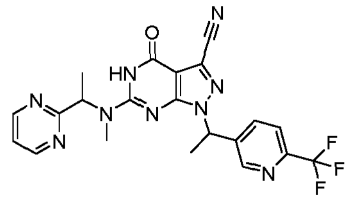 | 6-[methyl(1-pyrimidin-2-ylethyl)amino] -4-oxo-1-[1-[6-(trifluoromethy l)-3-pyridyl]ethyl]-5H-pyrazolo[3,4-d] pyrimidine-3-carbonitrile | 470.2 | (X Bridge C18 OBD 19 x 250 mm; A:water(20 mmol/L $NH_4HCO_3$), Mobile Phase B: ACN; Flow rate: 20 mL/min; Gradient: 35% B to 65% B in 9 min); 2 | (CHIRALPA KIC, 2 x 25 cm, 5 μm, 35% EtOH in Hex); 2 |

(continued)

The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation.

| Ex | Structure | Name | LCMS $[M+1]^+$ | Prep-HPLC Column; Peak # | Prep CHIRAL HPLC Column; Peak # |
|---|---|---|---|---|---|
| 9 | 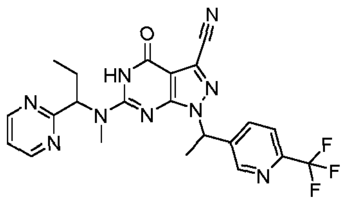 | 6-[methyl(1-pyrimidin-2-ylpropyl)amino ]-4-oxo-1-[1-[6-(trifluoromethy l)-3-pyridyl] ethyl]- 5H-pyrazolo[3,4-d] pyrimidine-3-carbonitrile | 484.3 | (X Bridge C18 OBD 19 × 250 mm; A:water(20 mmol/L $NH_4HCO_3$), Mobile Phase B: ACN; Flow rate: 20 mL/min; Gradient: 20% B to 80% B in 9 min); 1 | (CHIRAL ART Cellulose-SB S-5um, 2 x 25 cm,5 μm, 20% EtOH in Hex); 1 |
| 10 | 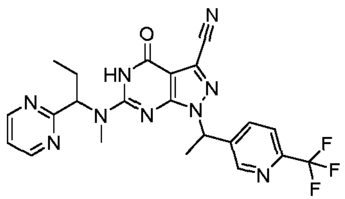 | 6-[methyl(1-pyrimidin-2-ylpropyl)amino ]-4-oxo-1-[1-[6-(trifluoromethyl)-3-pyridyl] ethyl]- 5H-pyrazolo-[3,4-d] pyrimidine-3-carbonitrile | 484.3 | (X Bridge C18 OBD 19 x 250 mm; A:water(20 mmol/L $NH_4HCO_3$), Mobile Phase B: ACN; Flow rate: 20 mL/min; Gradient: 20% B to 80% B in 9 min); 1 | (CHIRAL ART Cellulose-SB S-5um, 2 x 25 cm,5 μm, 20% EtOH in Hex); 2 |
| 11 | 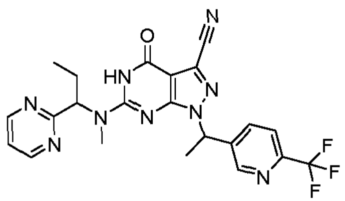 | 6-[methyl(1-pyrimidin-2-ylpropyl)amino ]-4-oxo-1-[1-[6-(trifluoromethyl)-3-pyridyl] ethyl] - 5H-pyrazolo-[3,4-d] pyrimidine-3-carbonitrile | 484.3 | (X Bridge C18 OBD 19 x 250 mm; A:water(20 mmol/L $NH_4HCO_3$), Mobile Phase B: ACN; Flow rate: 20 mL/min; Gradient: 20% B to 80% B in 9 min); 2 | (Chiralpak IC, 2 x 25 cm, 5 μm, 50% EtOH in Hex); 1 |
| 12 | 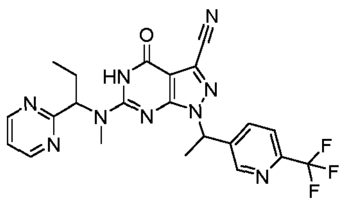 | 6-[methyl(1-pyrimidin-2-ylpropyl)amino ]-4-oxo-1-[1-[6-(trifluoromethyl)-3-pyridyl] ethyl]- 5H-pyrazolo[3,4-d] pyrimidine-3-carbonitrile | 484.3 | (X Bridge C 18 OBD 19 x 250 mm; A:water(20 mmol/L $NH_4HCO_3$), Mobile Phase B: ACN; Flow rate: 20 mL/min; Gradient: 20% B to 80% B in 9 min); 2 | (ChiralpakIC, 2 x 25 cm, 5 μm, 50% EtOH in Hex); 2 |

(continued)

The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation.

| Ex | Structure | Name | LCMS [M+1]+ | Prep-HPLC Column; Peak # | Prep CHIRAL HPLC Column; Peak # |
|---|---|---|---|---|---|
| 13 | 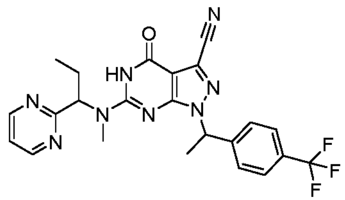 | 6-[methyl(1-pyrimidin-2-ylpropyl)amino ]-4-oxo-1-[1-[4-(trifluoromethyl) phenyl] ethyl]-5H-pyrazolo[3,4-d] pyrimidine-3-carbonitrile | 483.3 | | (CHIRALPA K AD-H, 2.0 cm I.D.x 25cm L, 15% IPA in Hex); 1 |
| 14 | 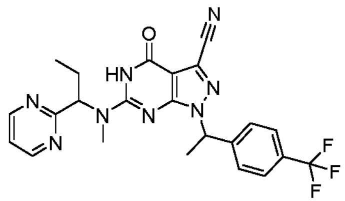 | 6-[methyl(1-pyrimidin-2-ylpropyl)amino ]-4-oxo-1-[1-[4-(trifluoromethyl) phenyl] ethyl]-5H-pyrazolo[3,4-d] pyrimidine-3-carbonitrile | 483.3 | | (CHIRALPA K AD-H, 2.0cm I.D. x 25cm L, 15% IPA in Hex); 2 |
| 15 | 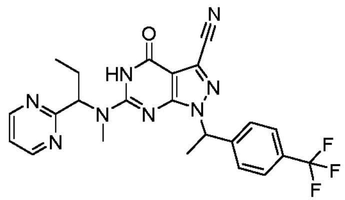 | 6-[methyl(1-pyrimidin-2-ylpropyl)amino ]-4-oxo-1-[1-[4-(trifluoromethyl) phenyl] ethyl]-5H-pyrazolo[3,4-d] pyrimidine-3-carbonitrile | 483.2 | | (CHIRALPA K AD-H, 2.0cm I.D. x 25cm L, 15% IPA in Hex); 3 |
| 16 | 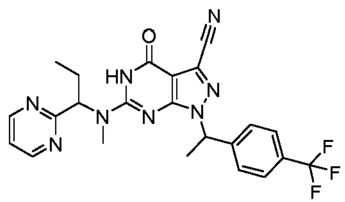 | 6-[methyl(1-pyrimidin-2-ylpropyl)amino ]-4-oxo-1-[1-[4-(trifluoromethyl) phenyl] ethyl]-5H-pyrazolo[3,4-d] pyrimidine-3-carbonitrile | 483.3 | | (CHIRALPA K AD-H, 2.0 cm I.D. x 25cm L, 15% IPA in Hex); 4 |

(continued)

The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation.

| Ex | Structure | Name | LCMS [M+1]+ | Prep-HPLC Column; Peak # | Prep CHIRAL HPLC Column; Peak # |
|---|---|---|---|---|---|
| 17 | 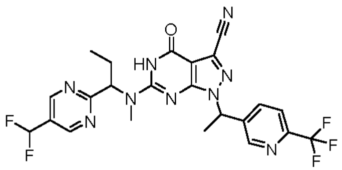 | 6-[1-[5-(difluoromethy l) pyrimidin-2-yl] propylmethyl-amino]-4-oxo-1-[1-[6-(trifluoromethy l)-3-pyridyl]-ethyl]-5H-pyrazolo[3,4-d] pyrimidine-3-carbonitrile | 534.2 | | From final reaction mixture the first eluted peak on Chiralpak IC (2 x 25cm, 5 μm; 50% IPA in Hex:DCM=1: 1) is further separated by Chiralpak ID-2 (2 x 25cm, 5 μm; 15% EtOH in Hex); 1 |
| 18 | 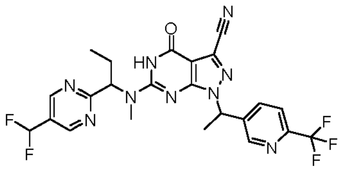 | 6-[1-[5-(difluoromethy l) pyrimidin-2-yl] propylmethyl-amino]-4-oxo-1-[1-[6-(trifluoromethy 1)-3-pyridyl]ethyl]-5H-pyrazolo[3,4-d] pyrimidine-3-carbonitrile | 534.2 | | From final reaction mixture the first eluted peak on Chiralpak IC (2 x 25cm, 5 μm; 50% IPA in Hex:DCM=1: 1) is further separated by Chiralpak ID-2 (2 x 25cm, 5 μm; 15% EtOH in Hex); 2 |
| 19 | 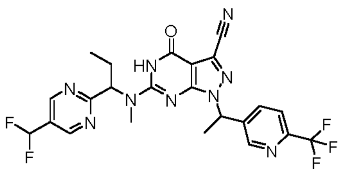 | 6-[1-[5-(difluoromethy l) pyrimidin-2-yl] propylmethyl-amino]-4-oxo-1-[1-[6-(trifluoromethy l)-3-pyridyl]-ethyl]-5H-pyrazolo[3,4-d] pyrimidine-3-carbonitrile | 534.2 | | (Chiralpak IC, 2 x 25cm, 5 μm; 50% IPA in Hex:DCM=1: 1); 2 |

(continued)

The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation.

| Ex | Structure | Name | LCMS $[M+1]^+$ | Prep-HPLC Column; Peak # | Prep CHIRAL HPLC Column; Peak # |
|---|---|---|---|---|---|
| 20 | 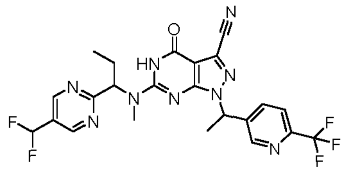 | 6-[1-[5-(difluoromethy l) pyrimidin-2-yl] propylmethyl-amino]-4-oxo- 1-[1-[6-(trifluoromethy 1)-3-pyridyl]-ethyl]-5H-pyrazolo[3,4-d] pyrimidine-3-carbonitrile | 534.2 | | (Chiralpak IC, 2 x 25cm, 5 μm; 50% IPA in Hex:DCM=l: 1); 3 |
| 21 | 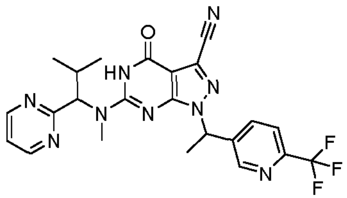 | 6-[methyl-(2-methyl-1-pyrimidin-2-yl-propyl) amino]-4-oxo- 1-[1-[6-(trifluoromethy l)-3-pyridyl]-ethyl]-5H-pyrazolo[3,4-d] pyrimidine-3-carbonitrile | 498.2 | (X Bridge C 18 OBD 19 x 250 mm; A:water(20 mmol/L $NH_4HCO_3$), Mobile Phase B: ACN; Flow rate: 20 mL/min; Gradient: 55% B to 65% B in 6.5 min);1 | (CHIRALPA K IC, 2 x 25cm,5 μm; 50% EtOH in Hex (8 mmol/L $NH_3$·MeOH)); 1 |
| 22 | 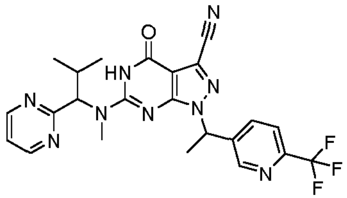 | 6-[methyl-(2-methyl-1-pyrimidin-2-yl-propyl) amino]-4-oxo- 1-[1-[6-(trifluoromethy l)-3-pyridyl]-ethyl]-5H-pyrazolo[3,4-d] pyrimidine-3-carbonitrile | 498.2 | (X Bridge C 18 OBD 19 x 250 mm; A:water(20 mmol/L $NH_4HCO_3$), Mobile Phase B: ACN; Flow rate: 20 mL/min; Gradient: 55% B to 65% B in 6.5 min);1 | (CHIRALPA K IC, 2 x 25cm,5 μm; 50% EtOH in Hex (8 mmol/L NH3.MeOH)); 2 |
| 23 | 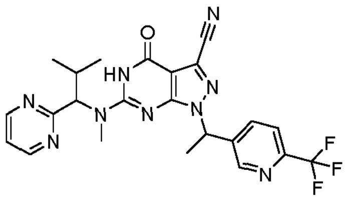 | 6-[methyl-(2-methyl-1-pyrimidin-2-yl-propyl) amino]-4-oxo- 1-[1-[6-(trifluoromethy l)-3-pyridyl]-ethyl]-5H-pyrazolo[3,4-d] pyrimidine-3-carbonitrile | 498.3 | (X Bridge C 18 OBD 19 x 250 mm; A:water(20 mmol/L $NH_4HCO_3$), Mobile Phase B: ACN; Flow rate: 20 mL/min; Gradient: 55% B to 65% B in 6.5 min);2 | (CHIRALPA K IC, 2 x 25cm,5 μm; 50% EtOH in Hex (8 mmol/L NH3.MeOH)); 1 |

(continued)

The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation.

| Ex | Structure | Name | LCMS $[M+1]^+$ | Prep-HPLC Column; Peak # | Prep CHIRAL HPLC Column; Peak # |
|---|---|---|---|---|---|
| 24 | 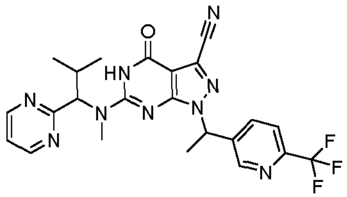 | 6-[methyl-(2-methyl-1-pyrimidin-2-yl-propyl) amino]-4-oxo-1-[1-[6-(trifluoromethy l)-3-pyridyl]-ethyl]-5H-pyrazolo[3,4-d] pyrimidine-3-carbonitrile | 498.3 | (X Bridge C 18 OBD 19 x 250 mm; A:water(20 mmol/L $NH_4HCO_3$), Mobile Phase B: ACN; Flow rate: 20 mL/min; Gradient: 55% B to 65% B in 6.5 min);2 | (CHIRALPA K IC, 2 x 25cm,5 µm; 50% EtOH in Hex (8 mmol/L NH3.MeOH)); 2 |
| 25 | 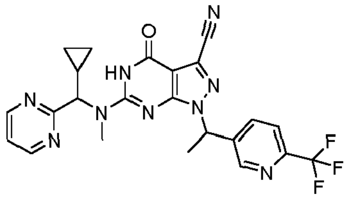 | 6-[[cyclopropyl(pyrimid in-2-yl)methyl]-methyl-amino]-4-oxo-1-[1-[6-(trifluoromethyl)-3-pyridyl] ethyl]- 5H-pyrazolo[3,4-d] pyrimidine-3-carbonitrile | 496.3 | (X Bridge C 18 OBD 19 × 250 mm; A:water(20 mmol/L $NH_4HCO_3$), Mobile Phase B: ACN; Flow rate: 20 mL/min; Gradient: 26% B to 65% B in 8 min); 1 | CHIRALPAK ID-03, 2.0 cm I.D x 25cm L (5 µm); 15% EtOH in Hex); 1 |
| 26 | 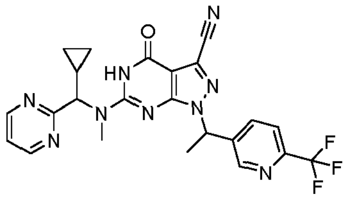 | 6-[[cyclopropyl(pyrimid in-2-yl)methyl]-methyl-amino]-4-oxo-1-[1-[6-(trifluoromethy 1)-3-pyridyl]ethyl]-5H-pyrazolo[3,4-d] pyrimidine-3-carbonitrile | 496.3 | (X Bridge C 18 OBD 19 x 250 mm; A: water(20 mmol/L $NH_4HCO_3$), Mobile Phase B: ACN; Flow rate: 20 mL/min; Gradient: 26% B to 65% B in 8 min); 1 | CHIRALPAK ID-03, 2.0 cm I.D × 25cm L(5 µm); 15% EtOH in Hex); 2 |
| 27 | 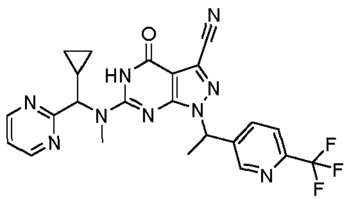 | 6-[[cyclopropyl(pyrimid in-2-yl)methyl]-methyl-amino]-4-oxo-1-[1-[6-(trifluoromethy l)-3-pyridyl] ethyl] - 5H-pyrazolo[3,4-d] pyrimidine-3-carbonitrile | 496.3 | (X Bridge C 18 OBD 19 x 250 mm; A:water(20 mmol/L $NH_4HCO_3$), Mobile Phase B: ACN; Flow rate: 20 mL/min; Gradient: 26% B to 65% B in 8 min);2 | (CHIRALPA K IC, 2 x 25cm, 5 µm; 30% EtOH in Hex (0.1%DEA)); 1 |

(continued)

The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation.

| Ex | Structure | Name | LCMS $[M+1]^+$ | Prep-HPLC Column; Peak # | Prep CHIRAL HPLC Column; Peak # |
|---|---|---|---|---|---|
| 28 | 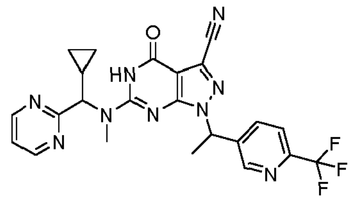 | 6-[[cyclopropyl(pyrimid in-2-yl)methyl]-methyl-amino]-4-oxo-1-[1-[6-(trifluoromethy l)-3-pyridyl] ethyl]- 5H-pyrazolo[3,4-d] pyrimidine-3-carbonitrile | 496.3 | (X Bridge C 18 OBD 19 x 250 mm; A: water(20 mmol/L $NH_4HCO_3$), Mobile Phase B: ACN; Flow rate: 20 mL/min; Gradient: 26% B to 65% B in 8 min);2 | (CHIRALPA K IC, 2 x 25cm, 5 μm; 30%: EtOH in Hex (0.1%DEA)); 2 |
| 29 | 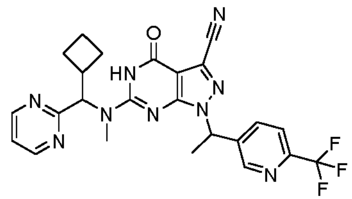 | 6-[[cyclobutyl(pyrimidi n-2-yl)methyl]-methyl-amino]-4-oxo-1-[1-[6-(trifluoromethy l)-3-pyridyl] ethyl]- 5H-pyrazolo[3,4-d] pyrimidine-3-carbonitrile | 510.4 | (XSelect CSH Prep C18 OBD Column, 19 x 250mm, 5 μm; Mobile Phase A: water (0.05% TFA), Mobile Phase B: ACN; Flow rate: 25 mL/min; Gradient: 53% B to 55% B in 14 min); 1 | (CHIRALPA K IE, 2 x 25cm, 5 μm; 30% EtOH in Hex (0.1%DEA)); 1 |
| 30 | 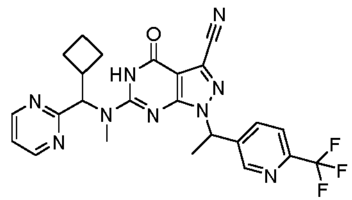 | 6-[[cyclobutyl(pyrimidi n-2-yl)methyl]-methyl-amino]-4-oxo-1-[1-[6-(trifluoromethy l)-3-pyridyl] ethyl]- 5H-pyrazolo[3,4-d] pyrimidine-3-carbonitrile | 510.4 | (XSelect CSH Prep C18 OBD Column, 19 x 250mm, 5 μm; Mobile Phase A: water (0.05% TFA), Mobile Phase B: ACN; Flow rate: 25 mL/min; Gradient: 53% B to 55% B in 14 min);1 | (CHIRALPA K IE, 2 x 25cm, 5 μm; 30%: EtOH in Hex (0.1%DEA)); 2 |
| 31 | 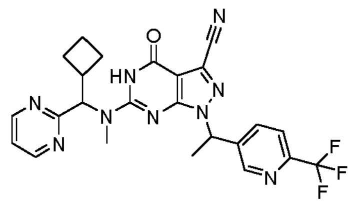 | 6-[[cyclobutyl(pyrimidi n-2-yl)methyl]-methyl-amino]-4-oxo-1-[1-[6-(trifluoromethy l)-3-pyridyl]ethyl]-5H-pyrazolo[3,4-d] pyrimidine-3-carbonitrile | 510.4 | (X Select CSH Prep C18 OBD Column, 19 x 250mm, 5 μm; Mobile Phase A: water (0.05% TFA), Mobile Phase B: ACN; Flow rate: 25 mL/min; Gradient: 53% B to 55% B in 14 min);2 | (CHIRALPA K IC, 2 x 25cm, 5 μm; 30% EtOH in Hex (0.1%DEA)); 1 |

(continued)

| The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation. | | | | | |
|---|---|---|---|---|---|
| Ex | Structure | Name | LCMS $[M+1]^+$ | Prep-HPLC Column; Peak # | Prep CHIRAL HPLC Column; Peak # |
| 32 | 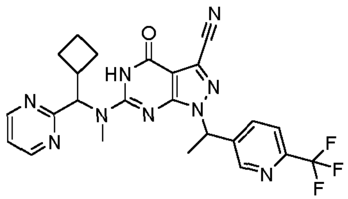 | 6-[[cyclobutyl(pyrimidi n-2-yl)methyl]-methyl-amino]-4-oxo-1-[1-[6-(trifluoromethy l)-3-pyridyl]ethyl]-5H-pyrazolo[3,4-d] pyrimidine-3-carbonitrile | 510.4 | (XSelect CSH Prep C18 OBD Column, 19 x 250mm, 5 µm; Mobile Phase A: water (0.05% TFA), Mobile Phase B: ACN; Flow rate: 25 mL/min; Gradient: 53% B to 55% B in 14 min); 2 | (CHIRALPA K IC, 2 x 25cm, 5 µm; 30% EtOH in Hex (0.1%DEA)); 2 |
| 33 | 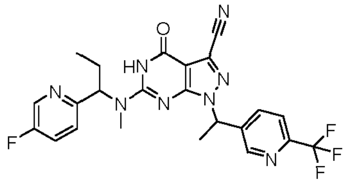 | 6-[1-(5-fluoro-2-pyridyl) propylmethyl-amino]-4-oxo-1-[1-[6-(trifluoromethy 1)-3-pyridyl] ethyl]- 5H-pyrazolo[3,4-d] pyrimidine-3-carbonitrile | 501.2 | | From final reaction mixture the first eluted peak on CHIRALPAK IC (2 x 25 cm,5 µm; 50% (EtOH:IPA=3 5:15) in Hex) is further separated by CHIRALART Amylose-SA (2 x 25cm,5 µm; KSA99S05-2520WX13534; 20% IPA in $CO_2$); 1 |
| 34 | 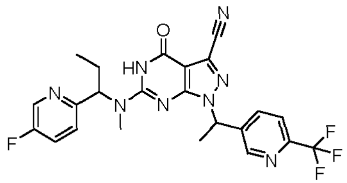 | 6-[1-(5-fluoro-2-pyridyl) propylmethyl-amino]-4-oxo-1-[1-[6-(trifluoromethy l)-3-pyridyl]ethyl]-5H-pyrazolo[3,4-d] pyrimidine-3-carbonitrile | 501.2 | | From final reaction mixture the first eluted peak on CHIRALPAK IC (2 x 25 cm,5 µm; 50% (EtOH: IPA=35:15) in Hex) is further separated by CHIRALART Amylose-SA (2 x 25cm,5 µm; KSA99S05-2520WX13534; 20% IPA in $CO_2$); 2 |
| 35 | 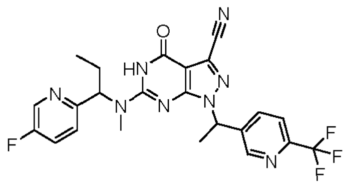 | 6-[1-(5-fluoro-2-pyridyl) propylmethyl-amino]-4-oxo-1-[1-[6-(trifluoromethy l)-3-pyridyl]ethyl]-5H-pyrazolo[3,4-d] pyrimidine-3-carbonitrile | 501.2 | | (CHIRALPA K IC, 2 x 25cm,5 µm; 50% (EtOH: IPA=3 5:15) in Hex); 2 |

(continued)

The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation.

| Ex | Structure | Name | LCMS [M+1]+ | Prep-HPLC Column; Peak # | Prep CHIRAL HPLC Column; Peak # |
|---|---|---|---|---|---|
| 36 | 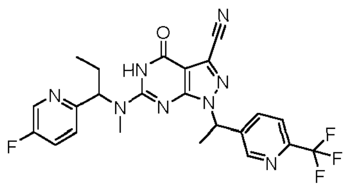 | 6-[1-(5-fluoro-2-pyridyl)-propyl-methyl-amino]-4-oxo-1-[1-[6-(trifluoromethyl)-3-pyridyl] ethyl]- 5H-pyrazolo[3,4-d] pyrimidine-3-carbonitrile | 501.2 | | (CHIRALPA K IC, 2 x 25 cm, 5 μm; 50% (EtOH: IPA=3 5:15) in Hex); 3 |
| 37 | 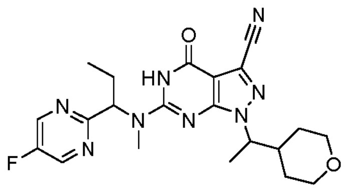 | 6-[1-(5-fluoropyrimidi n-2-yl)propylmethyl-amino]-4-oxo-1-(1-tetrahydropyra n-4-ylethyl)-5H-pyrazolo [3,4-d]pyrimidine-3-carbonitrile | 441.3 | (X Bridge C 18 OBD Prep Column 100 Å, 10 μm, 19 mm x 250 mm; Mobile Phase A: water (20 mmol/L $NH_4HCO_3$), Mobile Phase B: ACN; Flow rate: 25 mL/min; Gradient: 39% B to 45% B in 9 min); 1 | (CHIRALPA K IC, 2 x 25 cm, 5 μm; 8% EtOH in Hex : DCM = 3 : 1 (0.1%DEA)); 1 |
| 38 | 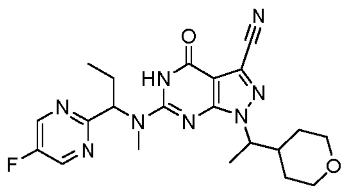 | 6-[1-(5-fluoropyrimidi n-2-yl)propylmethyl-amino]-4-oxo-1-(1-tetrahydropyra n-4-ylethyl)-5H-pyrazolo [3,4-d]pyrimidine-3-carbonitrile | 441.4 | (X Bridge C 18 OBD Prep Column 100 Å, 10 μm, 19 mm x 250 mm; Mobile Phase A: water (20 mmol/L $NH_4HCO_3$), Mobile Phase B: ACN; Flow rate: 25 mL/min; Gradient: 39% B to 45% B in 9 min); 1 | (CHIRALPA K IC, 2 x 25 cm, 5 μm; 8% EtOH Hex: DCM = 3 : 1 (0.1%DEA)); 2 |

(continued)

The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation.

| Ex | Structure | Name | LCMS $[M+1]^+$ | Prep-HPLC Column; Peak # | Prep CHIRAL HPLC Column; Peak # |
|---|---|---|---|---|---|
| 39 | 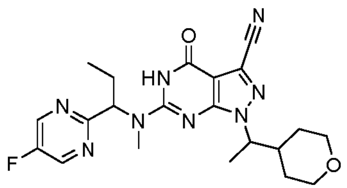 | 6-[1-(5-fluoropyrimidi n-2-yl)propylmethyl-amino]-4-oxo-1-(1-tetrahydropyra n-4-ylethyl)-5H-pyrazolo [3,4-d]pyrimidine-3-carbonitrile | 441.4 | (X Bridge C 18 OBD Prep Column 100 Å, 10 μm, 19 mm x 250 mm; Mobile Phase A: water (20 mmol/L $NH_4HCO_3$), Mobile Phase B: ACN; Flow rate: 25 mL/min; Gradient: 39% B to 45% B in 9 min); 2 | (CHIRALPA K IC, 2 x 25 cm, 5 μm; 8% EtOH Hex: DCM = 3 : 1 (0.1%DEA)); 1 |
| 40 | 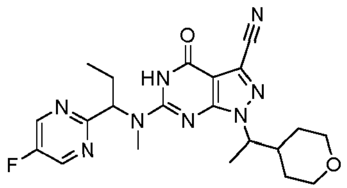 | 6-[1-(5-fluoropyrimidi n-2-yl)propylmethyl-amino]-4-oxo-1-(1-tetrahydropyra n-4-ylethyl)-5H-pyrazolo [3,4-d]pyrimidine-3-carbonitrile | 441.3 | (X Bridge C 18 OBD Prep Column 100 Å, 10 μm, 19 mm x 250 mm; Mobile Phase A: water (20 mmol/L $NH_4HCO_3$), Mobile Phase B: ACN; Flow rate: 25 mL/min; Gradient: 39% B to 45% B in 9 min); 2 | (CHIRALPA K IC, 2 x 25 cm, 5 μm; 8% EtOH Hex : DCM = 3 : 1 (0.1%DEA)); 2 |
| 41 | 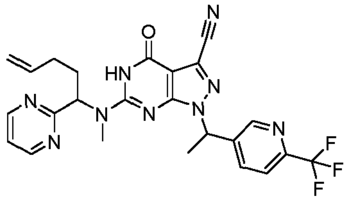 | 6-[methyl(1-pyrimidin-2-ylpent-4-enyl)amino]-4-oxo-1-[1-[6-(trifluoromethy l)-3-pyridyl]ethyl]-5H-pyrazolo[3,4-d] pyrimidine-3-carbonitrile | 510.2 | (X Bridge C 18 OBD Prep Column 100 Å, 10 μm, 19 mm x 250 mm; Mobile Phase A: water (20 mmol/L $NH_4HCO_3$), Mobile Phase B: ACN; Flow rate: 25 mL/min; Gradient: 50% B to 80% B in 6.5 min); 1 | (CHIRALPA K IG, 2 x 25 cm, 5 μm, 20% EtOH in Hex); 1 |

(continued)

| The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation. | | | | | |
|---|---|---|---|---|---|
| Ex | Structure | Name | LCMS $[M+1]^+$ | Prep-HPLC Column; Peak # | Prep CHIRAL HPLC Column; Peak # |
| 42 | 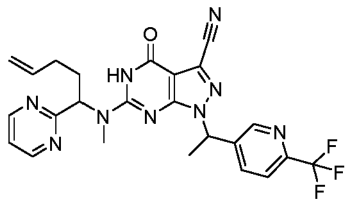 | 6-[methyl(1-pyrimidin-2-ylpent-4-enyl)amino]-4-oxo-1-[1-[6-(trifluoromethy l)-3-pyridyl]ethyl]-5H-pyrazolo[3,4-d] pyrimidine-3-carbonitrile | 510.2 | (X Bridge C 18 OBD Prep Column 100 Å, 10 μm, 19 mm x 250 mm; Mobile Phase A: water (20 mmol/L $NH_4HCO_3$), Mobile Phase B: ACN; Flow rate: 25 mL/min; Gradient: 50% B to 80% B in 6.5 min); 1 | (CHIRALPA K IG, 2 x 25 cm, 5 μm, 20% EtOH in Hex); 2 |
| 43 | 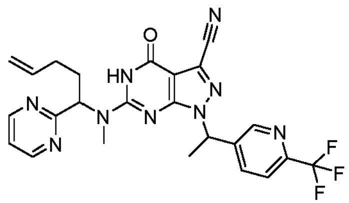 | 6-[methyl(1-pyrimidin-2-ylpent-4-enyl)amino]-4-oxo-1-[1-[6-(trifluoromethy l)-3-pyridyl] ethyl]- 5H-pyrazolo[3,4-d] pyrimidine-3-carbonitrile | 510.2 | (X Bridge C 18 OBD Prep Column 100 Å, 10 μm, 19 mm x 250 mm; Mobile Phase A: water (20 mmol/L $NH_4HCO_3$), Mobile Phase B: ACN; Flow rate: 25 mL/min; Gradient: 50% B to 80% B in 6.5 min); 2 | (CHIRALPA K IC, 2 x 25 cm, 5 μm, 50% EtOH in Hex); 1 |
| 44 | 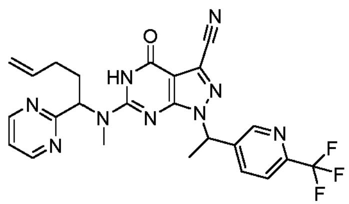 | 6-[methyl(1-pyrimidin-2-ylpent-4-enyl)amino]-4-oxo-1-[1-[6-(trifluoromethy l)-3-pyridyl]ethyl]-5H-pyrazolo[3,4-d] pyrimidine-3-carbonitrile | 510.2 | (X Bridge C 18 OBD Prep Column 100 Å, 10 μm, 19 mm x 250 mm; Mobile Phase A: water (20 mmol/L $NH_4HCO_3$), Mobile Phase B: ACN; Flow rate: 25 mL/min; Gradient: 50% B to 80% B in 6.5 min); 2 | (CHIRALPA K IC, 2 x 25 cm, 5 μm, 50% EtOH in Hex); 1 |

(continued)

| The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation. | | | | | |
|---|---|---|---|---|---|
| Ex | Structure | Name | LCMS $[M+1]^+$ | Prep-HPLC Column; Peak # | Prep CHIRAL HPLC Column; Peak # |
| 45 | 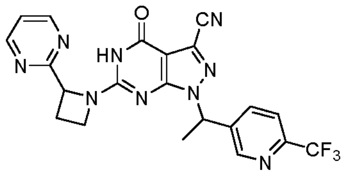 | 4-oxo-6-(2-pyrimidin-2-ylazetidin-1-yl)-1-[1-[6-(trifluoromethy 1)-3-pyridyl]ethyl]-5H-pyrazolo[3,4-d] pyrimidine-3-carbonitrile | 468.1 | (Sunfire Prep $C_{18}$ OBD Column, 10 μm, 19 × 250mm; Mobile Phase A: water (0.05% TFA), Mobile Phase B: ACN, Gradient: 30% B to 50% B in 12 min); 1 | (CHIRALPA K IC, 2 × 25 cm, 5 μm; 50% EtOH in Hex: DCM = 3 : 1); 1 |
| 46 | 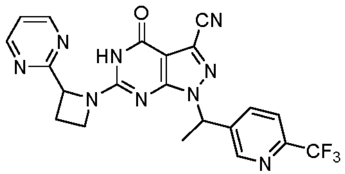 | 4-oxo-6-(2-pyrimidin-2-ylazetidin-1-yl)-1-[1-[6-(trifluoromethy l)-3-pyridyl] ethyl]- 5H-pyrazolo[3,4-d] pyrimidine-3-carbonitrile | 468.1 | (Sunfire Prep $C_{18}$ OBD Column, 10 μm, 19 x 250mm; Mobile Phase A: water (0.05% TFA), Mobile Phase B: ACN, Gradient: 30% B to 50% B in 12 min); 1 | (CHIRALPA K IC, 2 x 25 cm, 5 μm; 50% EtOH in Hex: DCM = 3 : 1); 2 |
| 47 | 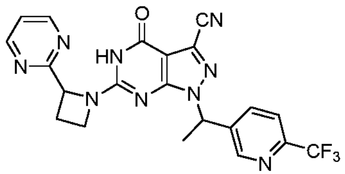 | 4-oxo-6-(2-pyrimidin-2-ylazetidin-1-yl)-1-[1-[6-(trifluoromethy l)-3-pyridyl] ethyl]- 5H-pyrazolo[3,4-d] pyrimidine-3-carbonitrile | 468.1 | (Sunfire Prep $C_{18}$ OBD Column, 10 μm, 19 x 250mm; Mobile Phase A: water (0.05% TFA), Mobile Phase B: ACN, Gradient: 30% B to 50% B in 12 min); 2 | (CHIRALPA K IC, 2 x 25 cm, 5 μm; 50% EtOH in Hex: DCM = 3 : 1); 1 |
| 48 | 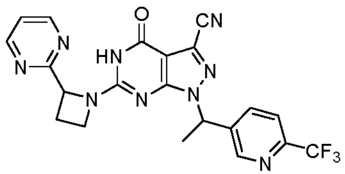 | 4-oxo-6-(2-pyrimidin-2-ylazetidin-1-yl)-1-[1-[6-(trifluoromethy l)-3-pyridyl] ethyl]- 5H-pyrazolo[3,4-d] pyrimidine-3-carbonitrile | 468.1 | (Sunfire Prep $C_{18}$ OBD Column, 10 μm, 19 x 250mm; Mobile Phase A: water (0.05% TFA), Mobile Phase B: ACN, Gradient: 30% B to 50% B in 12 min); 2 | (CHIRALPA K IC, 2 x 25 cm, 5 μm; 50% EtOH in Hex: DCM = 3 : 1); 2 |

(continued)

| The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation. | | | | | |
|---|---|---|---|---|---|
| Ex | Structure | Name | LCMS $[M+1]^+$ | Prep-HPLC Column; Peak # | Prep CHIRAL HPLC Column; Peak # |
| 49 | 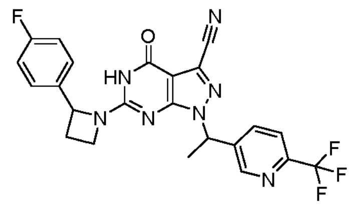 | 6-[2-(4-fluorophenyl)a zetidin-1-yl]-4-oxo-1-[1-[6-(trifluorometHy l)-3-pyridyl]ethyl]-5H-pyrazolo[3,4-d] pyrimidine-3-carbonitrile | 484.2 | (X Bridge C 18 OBD Prep Column 100 Å, 10 μm, 19 mm x 250 mm; Mobile Phase A: water (20 mmol/L $NH_4HCO_3$), Mobile Phase B: ACN; Flow rate: 20 mL/min; Gradient: 20% B to 80% B in 9 min); 1 | (CHIRALPA K IE, 2 x 25 cm, 5 μm, 35% EtOH in Hex); 1 |
| 50 | 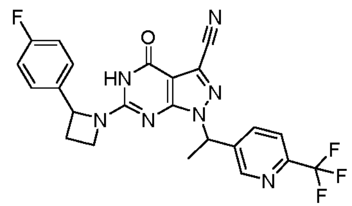 | 6-[2-(4-fluorophenyl)a zetidin-1-yl]-4-oxo-1-[1-[6-(trifluoromethy l)-3-pyridyl]ethyl]-5H-pyrazolo[3,4-d] pyrimidine-3-carbonitrile | 484.2 | (X Bridge C 18 OBD Prep Column 100Å, 10 μm, 19 mm x 250 mm; Mobile Phase A: water (20 mmol/L $NH_4HCO_3$), Mobile Phase B: ACN; Flow rate: 20 mL/min; Gradient: 20% B to 80% B in 9 min); 1 | (CHIRALPA K IE, 2 x 25 cm, 5 μm, 35% EtOH in Hex); 2 |
| 51 | 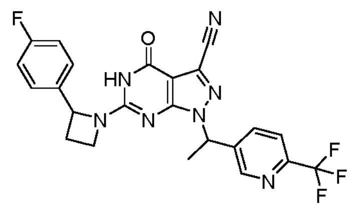 | 6-[2-(4-fluorophenyl)a zetidin-1-yl]-4-oxo-1-[1-[6-(trifluoromethy l)-3-pyridyl]ethyl]-5H-pyrazolo[3,4-d] pyrimidine-3-carbonitrile | 484.2 | (X Bridge C 18 OBD Prep Column 100Å, 10 μm, 19 mm x 250 mm; Mobile Phase A: water (20 mmol/L $NH_4HCO_3$), Mobile Phase B: ACN; Flow rate: 20 mL/min; Gradient: 20% B to 80% B in 9 min); 2 | (CHIRALPA K IC, 2 x 25 cm, 5 μm, 50% EtOH in Hex); 1 |

(continued)

| The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation. | | | | | |
|---|---|---|---|---|---|
| Ex | Structure | Name | LCMS $[M+1]^+$ | Prep-HPLC Column; Peak # | Prep CHIRAL HPLC Column; Peak # |
| 52 | 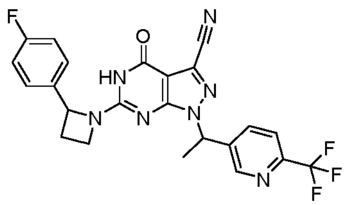 | 6-[2-(4-fluorophenyl)a zetidin-1-yl]-4-oxo-1-[1-[6-(trifluoromethy l)-3-pyridyl]ethyl]-5H-pyrazolo[3,4-d] pyrimidine-3-carbonitrile | 484.2 | (X Bridge C 18 OBD Prep Column 100Å, 10 µm, 19 mm x 250 mm; Mobile Phase A: water (20 mmol/L $NH_4HCO_3$), Mobile Phase B: ACN; Flow rate: 20 mL/min; Gradient: 20% B to 80% B in 9 min); 2 | (CHIRALPA K IC, 2 x 25 cm, 5 µm, 50% EtOH in Hex); 2 |
| 53 | 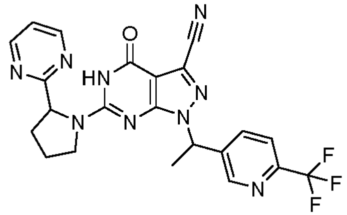 | 4-oxo-6-(2-pyrimidin-2-ylpyrrolidin-1-yl)-1-[1-[6-(trifluoromethy l)-3-pyridyl]ethyl]-5H-pyrazolo[3,4-d] pyrimidine-3-carbonitrile | 482.2 | (X Bridge C 18 OBD Prep Column 100Å, 10 µm, 19 mm x 250 mm; Mobile Phase A: water (20 mmol/L $NH_4HCO_3$), Mobile Phase B: ACN; Flow rate: 20 mL/min; Gradient: 39% B to 40% B in 9 min); 1 | (CHIRALPA K IE, 2 x 25 cm, 5 µm, 50% EtOH in Hex); 1 |
| 54 | 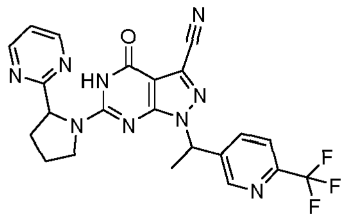 | 4-oxo-6-(2-pyrimidin-2-ylpyrrolidin-1-yl)-1-[1-[6-(trifluoromethy l)-3-pyridyl]ethyl]-5H-pyrazolo[3,4-d] pyrimidine-3-carbonitrile | 482.2 | (X Bridge C 18 OBD Prep Column 100Å, 10 µm, 19 mm x 250 mm; Mobile Phase A: water (20 mmol/L $NH_4HCO_3$), Mobile Phase B: ACN; Flow rate: 20 mL/min; Gradient: 39% B to 40% B in 9 min); 1 | (CHIRALPA K IE, 2 x 25 cm, 5 µm, 50% EtOH in Hex); 2 |

(continued)

The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation.

| Ex | Structure | Name | LCMS $[M+1]^+$ | Prep-HPLC Column; Peak # | Prep CHIRAL HPLC Column; Peak # |
|---|---|---|---|---|---|
| 55 | 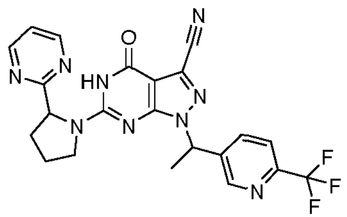 | 4-oxo-6-(2-pyrimidin-2-ylpyrrolidin-1-yl)-1-[1-[6-(trifluoromethy l)-3-pyridyl]ethyl]-5H-pyrazolo[3,4-d] pyrimidine-3-carbonitrile | 482.2 | (X Bridge C 18 OBD Prep Column 100Å, 10 μm, 19 mm x 250 mm; Mobile Phase A: water (20 mmol/L $NH_4HCO_3$), Mobile Phase B: ACN; Flow rate: 20 mL/min; Gradient: 39% B to 40% B in 9 min); 2 | (CHIRALPA K IE, 2 x 25 cm, 5 μm, 40% EtOH in Hex); 1 |
| 56 | 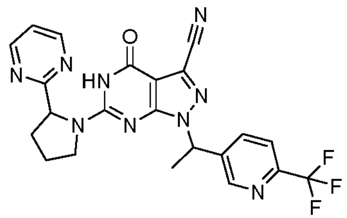 | 4-oxo-6-(2-pyrimidin-2-ylpyrrolidin-1-yl)-1-[1-[6-(trifluoromethy l)-3-pyridyl]ethyl]-5H-pyrazolo[3,4-d] pyrimidine-3-carbonitrile | 482.2 | (XBridge C18 OBD Prep Column 100Å, 10 μm, 19 mm x 250 mm; Mobile Phase A: water (20 mmol/L $NH_4HCO_3$), Mobile Phase B: ACN; Flow rate: 20 mL/min; Gradient: 39% B to 40% B in 9 min); 2 | (CHIRALPA K IE, 2 x 25 cm, 5 μm, 40% EtOH in Hex); 2 |
| 57 | 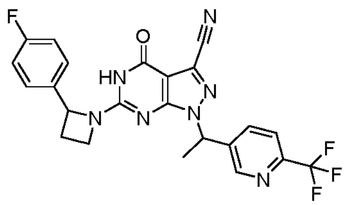 | 6-[2-(4-fluorophenyl)a zetidin-1-yl]-4-oxo-1-[1-[6-(trifluoromethy l)-3-pyridyl]ethyl]-5H-pyrazolo[3,4-d] pyrimidine-3-carbonitrile | 484.2 | (X Bridge C 18 OBD Prep Column 100Å, 10 μm, 19 mm × 250 mm; Mobile Phase A: water (20 mmol/L $NH_4HCO_3$), Mobile Phase B: ACN; Flow rate: 20 mL/min; Gradient: 20% B to 80% B in 9 min); 1 | |

(continued)

The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation.

| Ex | Structure | Name | LCMS $[M+1]^+$ | Prep-HPLC Column; Peak # | Prep CHIRAL HPLC Column; Peak # |
|---|---|---|---|---|---|
| 58 | 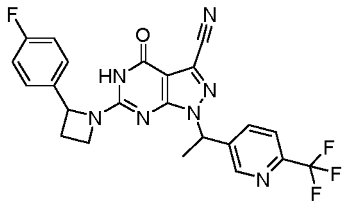 | 6-[2-(4-fluorophenyl)a zetidin-1-yl]-4-oxo-1-[1-[6-(trifluoromethy l)-3-pyridyl]ethyl]-5H-pyrazolo[3,4-d] pyrimidine-3-carbonitrile | 484.2 | (X Bridge C 18 OBD Prep Column 100Å, 10 µm, 19 mm x 250 mm; Mobile Phase A: water (20 mmol/L $NH_4HCO_3$), Mobile Phase B: ACN; Flow rate: 20 mL/min; Gradient: 20% B to 80% B in 9 min); 2 | |
| 59 | 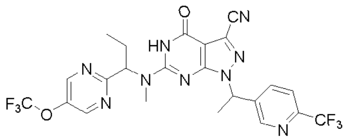 | 6-[methyl-[1-[5-(trifluoro-methoxy)pyrim idin-2-yl] propyl]amin o]-4-oxo-1-[1-[6-(trifluoromethyl)-3-pyridyl] ethyl]- 5H-pyrazolo[3,4-d] pyrimidine-3-carbonitrile | $[M-1]^-$: 566.2 | (Column: SunFire C18 OBD Prep Column , 100Å, 5 µm, 19 mm x 250 mm; Mobile Phase A:Water (0.05%TFA), Mobile Phase B: ACN; Flow rate: 25 mL/min; Gradient: 75% B to 76% B in 7 min); 1 | (Chiralpak IC, 2*25cm, 5um, 30% EtOH in Hex); 1 |
| 60 | 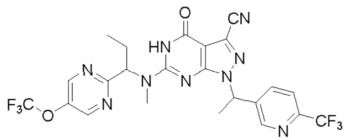 | 6-[methyl-[1-[5-(trifluoro-methoxy)pyrim idin-2-yl] propyl]amin o]-4-oxo-1-[1-[6-(trifluoromethyl)-3-pyridyl] ethyl]- 5H-pyrazolo[3,4-d] pyrimidine-3-carbonitrile | $[M-1]^-$: 566.2 | (Column: SunFire C18 OBD Prep Column , 100Å, 5 µm, 19 mm x 250 mm; Mobile Phase A:Water (0.05%TFA), Mobile Phase B: ACN; Flow rate: 25 mL/min; Gradient: 75% B to 76% B in 7 min); 1 | (Chiralpak IC, 2 x 25cm, 5um, 30% EtOH in Hex); 2 |

(continued)

The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation.

| Ex | Structure | Name | LCMS $[M+1]^+$ | Prep-HPLC Column; Peak # | Prep CHIRAL HPLC Column; Peak # |
|---|---|---|---|---|---|
| 61 | 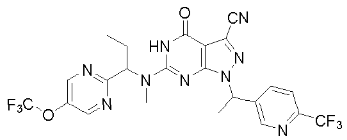 | 6-[methyl-[1-[5-(trifluoro-methoxy)pyrim idin-2-yl] propyl]-amino]-4-oxo-1-[1-[6-(trifluoromethyl)-3-pyridyl]ethyl]-5H-pyrazolo-[3,4-d] pyrimidine-3-carbonitrile | $[M-1]^-$: 566.2 | (Column: SunFire C18 OBD Prep Column , 100Å, 5 µm, 19 mm x 250 mm; Mobile Phase A:Water (0.05%TFA), Mobile Phase B: ACN; Flow rate: 25 mL/min; Gradient: 75% B to 76% B in 7 min); 2 | (Chiralpak IC, 2 x 25cm, 5um, 30% IPA in (HEX:DCM = 3:1)); 1 |
| 62 | 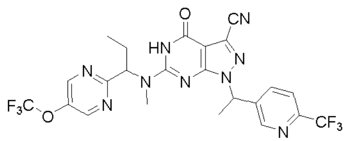 | 6-[methyl-[1-[5-(trifluoro-methoxy)pyrim idin-2-yl] propyl]amin o]-4-oxo-1-[1-[6-(trifluoromethyl)-3-pyridyl]ethyl]-5H-pyrazolo[3,4-d] pyrimidine-3-carbonitrile | $[M-1]^-$: 566.2 | (Column: SunFire C18 OBD Prep Column , 100Å, 5 µm, 19 mm x 250 mm; Mobile Phase A:Water (0.05%TFA), Mobile Phase B: ACN; Flow rate: 25 mL/min; Gradient: 75% B to 76% B in 7 min); 2 | (Chiralpak IC, 2 x 25cm, 5um, 30% IPA in (HEX:DCM = 3:1)); 2 |
| 63 | 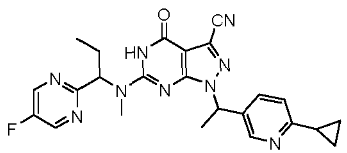 | 1-[1-(6-cyclopropyl-3-pyridyl)ethyl]-6-[1-(5-fluoropyrimidi n-2-yl) propylmethyl-amino]-4-oxo-5H-pyrazolo[3,4-d] pyrimidine-3-carbonitrile | 474.2 | | From final reaction mixture the first eluted peak on (Column: Lux 5u Amylose-1, AXIA Packed, 2.12 x 25 cm (5 um); Mobile Phase A: $CO_2$: 60%, Mobile Phase B: EtOH: 40%); is further separated by (Column: CHIRALPAK IC, 2 x 25 cm, 5 um; Mobile Phase A: $CO_2$ : 60%, Mobile Phase B: EtOH--HPLC: 40%); 1 |

(continued)

The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation.

| Ex | Structure | Name | LCMS $[M+1]^+$ | Prep-HPLC Column; Peak # | Prep CHIRAL HPLC Column; Peak # |
|---|---|---|---|---|---|
| 64 | 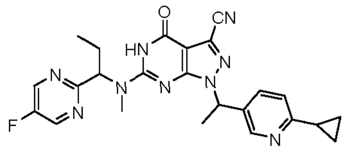 | 1-[1-(6-cyclopropyl-3-pyridyl)ethyl]-6-[1-(5-fluoropyrimidi n-2-yl) propylmethyl-amino]-4-oxo-5H-pyrazolo[3,4-d] pyrimidine-3-carbonitrile | 474.2 | | From final reaction mixture the first eluted peak on (Column: Lux 5u Amylose-1, AXIA Packed, 2.12 x 25 cm (5 um); Mobile Phase A: $CO_2$: 60%, Mobile Phase B: EtOH: 40%); is further separated by (Column: CHIRALPAK IC, 2 x 25 cm, 5 um; Mobile Phase A: $CO_2$ : 60%, Mobile Phase B: EtOH--HPLC: 40%); 2 |
| 65 | 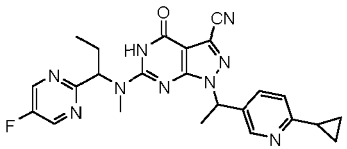 | 1-[1-(6-cyclopropyl-3-pyridyl)ethyl]-6-[1-(5-fluoropyrimidi n-2-yl) propylmethyl-amino]-4-oxo-5H-pyrazolo[3,4-d] pyrimidine-3-carbonitrile | 474.2 | | (Column: Lux 5u Amylose-1, AXIA Packed, 2.12 x 25 cm (5 um); Mobile Phase A: $CO_2$ : 60%, Mobile Phase B: EtOH: 40%); 2 |
| 66 | 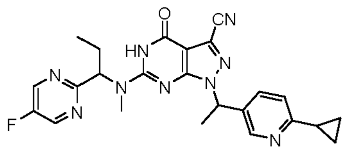 | 1-[1-(6-cyclopropyl-3-pyridyl)ethyl]-6-[1-(5-fluoropyrimidi n-2-yl) propylmethyl-amino]-4-oxo-5H-pyrazolo[3,4-d] pyrimidine-3-carbonitrile | 474.2 | | (Column: Lux 5u Amylose-1, AXIA Packed, 2.12 x 25 cm (5 um); Mobile Phase A: $CO_2$: 60%, Mobile Phase B: EtOH: 40%); 3 |
| 67 | 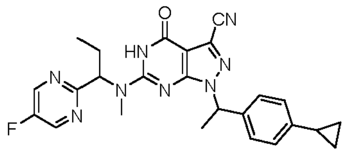 | 1-[1-(4-cyclopropylphe nyl)ethyl]-6-[1-(5-fluoro-pyrimidin-2-yl) propylmethyl-amino]-4-oxo-5H-pyrazolo[3,4-d] pyrimidine-3-carbonitrile | 473.2 | | From final reaction mixture the first eluted peak on (Chiralpak AD-H, 2 x 25 cm, 5 um; 30% IPA in Hex); 1 is further separated by (Chiralpak OJ-H, 2 x 25 cm, 5 um; Mobile Phase A: $CO_2$: 55%, Mobile Phase B: MeOH: MeCN (2:1): 45%); 1 |

(continued)

The following compounds were prepared according to the general procedure provided in the examples and procedures herein using known or prepared starting materials, as described in the reaction schemes and examples herein using appropriate starting materials and purification conditions. The requisite starting materials are either prepared as described in the intermediates section, commercially available, or may be prepared from commercially available reagents using conventional reactions well known in the art without undue experimentation.

| Ex | Structure | Name | LCMS $[M+1]^+$ | Prep-HPLC Column; Peak # | Prep CHIRAL HPLC Column; Peak # |
|---|---|---|---|---|---|
| 68 | 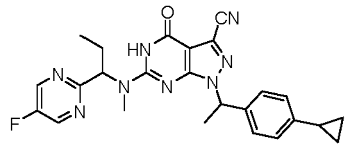 | 1-[1-(4-cyclopropylphe nyl)ethyl]-6-[1-(5-fluoro-pyrimidin-2-yl) propylmethyl-amino]-4-oxo-5H-pyrazolo[3,4-d] pyrimidine-3-carbonitrile | 473.2 | | From final reaction mixture the first eluted peak on (Chiralpak AD-H, 2 x 25 cm, 5 um; 30% IPA in Hex); 1 is further separated by (Chiralpak OJ-H, 2 x 25 cm, 5 um; Mobile Phase A: $CO_2$: 55%, Mobile Phase B: MeOH: MeCN (2:1): 45%); 2 |
| 69 | 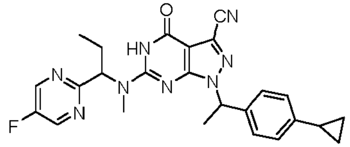 | 1-[1-(4-cyclopropylphe nyl)ethyl]-6-[1-(5-fluoro-pyrimidin-2-yl) propylmethyl-amino]-4-oxo-5H-pyrazolo[3,4-d] pyrimidine-3-carbonitrile | 473.2 | | From final reaction mixture the first eluted peak on (Chiralpak AD-H, 2 x 25 cm, 5 um; 30% IPA in Hex); 1 is further separated by (Chiralpak OJ-H, 2 x 25 cm, 5 um; Mobile Phase A: $CO_2$: 55%, Mobile Phase B: MeOH: MeCN (2:1): 45%); 3 |
| 70 | 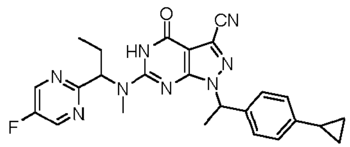 | 1-[1-(4-cyclo-propylphenyl)e thyl]-6-[1-(5-fluoropyrimidi n-2-yl)propyl-methyl-amino]-4-oxo-5H-pyrazolo[3,4-d] pyrimidine-3-carbonitrile | 473.2 | | (Chiralpak AD-H, 2 x 25 cm, 5 um; 30% IPA in Hex); 2 |

EXAMPLES 71-74

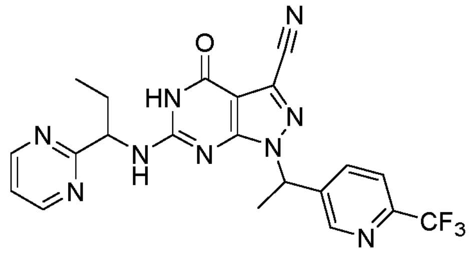

4-Oxo-6-((1-(pyrimidin-2-yl)propyl)amino)-1-(1-(6-(trifluoromethyl)pyridin-3-yl)ethyl)-4,5-dihydro-1*H*-pyrazolo[3,4-*d*]pyrimidine-3-carbonitrile

Step A: 5-(4-Methoxybenzyl)-4-oxo-6-((1-(pyrimidin-2-yl)propyl)amino)-1-(1-(6-(trifluoromethyl)pyridin-3-yl)ethyl)-4,5-dihydro-1*H*-pyrazolo[3,4-*d*]pyrimidine-3-carbonitrile. To a solution of 6-iodo-5-(4-methoxybenzyl)-4-oxo-1-(1-(6-(trifluoromethyl)pyridine -3-yl)ethyl)-4,5-dihydro-1*H*-pyrazolo[3,4-*d*]pyrimidine-3-carbonitrile (0.20 g, 0.35 mmol) in toluene (8 mL) were added 1-(pyrimidin-2-yl)propan-1-amine hydrochloride (47.30 mg, 0.35 mmol), $Pd_2(dba)_3$ $CHCl_3$ (63.10 mg, 0.069 mmol), XantPhos (80.00 mg, 0.14 mmol) and $Cs_2CO_3$ (0.45 g, 1.38 mmol) at room temperature. The reaction mixture was degassed with $N_2$ 3 times. The mixture was stirred at 90 °C for 16 h under $N_2$. The resulting mixture was diluted with water (20 mL) and extracted with EtOAc (2 × 30 mL). The combined organic layers were washed with brine (2 × 30 mL), dried over anhydrous $Na_2SO_4$ and filtered. The filtrate was concentrated under reduced pressure. The residue was purified by Cis phase reversal column with gradient 20%-40% ACN in water as eluent. The collected fractions were combined and concentrated under reduced pressure to afford 5-(4-methoxybenzyl)-4-oxo-6- ((1-(pyrimidin-2-yl)propyl)amino)-1-(1-(6-(trifluoromethyl)pyridin-3-yl)ethyl)-4,5-dihydro-1*H*-pyrazolo[3,4-*d*]pyrimidine-3-carbonitrile. LCMS (ES, *m*/*z*): 590.4 $[M+H]^+$.

Step B: 4-Oxo-6-((1-(pyrimidin-2-yl)propyl)amino)-1-(1-(6-(trifluoromethyl)pyridin- 3-yl)ethyl)-4,5-dihydro-1*H*-pyrazolo[3,4-*d*]pyrimidine-3-carbonitrile. To a solution of 5-(4-methoxybenzyl)-4-oxo-6-((1-(pyrimidin-2-yl)propyl)amino) -1 -(1 -(6-(trifluoromethyl)pyridin-3 - yl)ethyl)-4,5-dihydro-1*H*-pyrazolo[3,4-*d*]pyrimidine-3-carbonitrile (0.10 g, 0.17 mmol) in TFA (4 mL) was added methanesulfonic acid (0.02 mL, 0.25 mmol). The reaction mixture was stirred at 50 °C for 16 h. The resulting mixture was filtered and the filtrate was purified by Prep-HPLC with the following conditions: column: X Bridge Cis OBD Prep Column 100 Å, 10 μm, 19 mm × 250 mm; Mobile Phase A: water with 10 mmol/L $NH_4HCO_3$, Mobile Phase B: ACN; Flow rate: 25 mL/min; Gradient: 40% B to 70% B in 6.5 min; 254/210 nm; Rt: 5.73 min. The fractions containing the desired product were combined and concentrated under reduced pressure to afford 4-oxo-6-((1-(pyrimidin-2-yl)propyl)amino)-1-(1-(6-(trifluoromethyl)pyridin-3-yl)ethyl)-4,5-dihydro-1*H*-pyrazolo[3,4-d]pyrimidine-3-carbonitrile. The product was purified by Prep-Chiral-HPLC with the following conditions: column: CHIRALPAK IC, 2 × 25 cm, 5 μm; Mobile Phase A: Hex: DCM = 3 : 1, Mobile Phase B: IPA; Flow rate: 16 mL/min; Gradient: 50% B to 50% B in 23 min; 254/220 nm; $Rt_1$: 8.23 min; $Rt_2$: 11.84 min; $Rt_3$: 18.12 min. The first peak was obtained at 8.23 min. The collected fractions were combined and concentrated under vacuum to afford mixture A. The second peak was obtained at 11.84 min. The collected fractions were combined and concentrated under vacuum to afford Example 71: $^1$H NMR (400 MHz, $CD_3OD$) δ 8.71 (d, *J*= 5.2 Hz, 2H), 8.53 (d, *J* = 2.4 Hz, 1H), 7.83 (dd, *J*= 8.4 Hz, 2.0 Hz, 1H), 7.71 (d, *J*= 8.4 Hz, 1H), 7.26 (t, *J*= 4.8 Hz, 1H), 6.00 (q, *J* = 7.2 Hz, 1H), 5.13-5.09 (m, 1H), 2.10-1.93 (m, 2H), 1.90 (d, *J* = 7.2 Hz, 3H). 0.96 (t, *J*= 7.2 Hz, 3H). LCMS (ES, *m/z):* 470.2 $[M+H]^+$. The third peak was obtained at 18.12 min. The collected fractions were combined and concentrated under reduced pressure to afford Example 72: $^1$H NMR (400 MHz, $CD_3OD$) δ 8.80 (d, *J*= 4.8 Hz, 2H), 8.75 (d, *J*= 1.6 Hz, 1H), 8.00 (dd, *J*= 8.0 Hz, 2.0 Hz, 1H), 7.81 (d, *J* = 8.4 Hz, 1H), 7.39 (t, *J*= 4.8 Hz, 1H), 5.99 (q, *J* = 7.2 Hz, 1H), 5.18 (t, *J*= 6.4 Hz, 1H), 2.06-1.90 (m, 2H), 1.88 (d, *J*= 7.2 Hz, 3H). 0.83 (t, *J*= 7.2 Hz, 3H). LCMS (ES, *m/z):* 470.2 $[M+H]^+$. The mixture A from above was purified by Prep-Chiral-HPLC with the following conditions: Column: (R,R)Whelk-O 1, 21.1 × 250 mm, 5 μm; Mobile Phase A: Hex, Mobile Phase B: EtOH; Flow rate: 20 mL/min; Gradient: 30% B to 30% B in 25 min; 254/220 nm; $Rt_1$: 16.30 min; $Rt_2$:20.71 min. The faster-eluting enantiomer was obtained at 16.30 min. The collected fractions were combined and concentrated under reduced pressure to afford Example 73: $^1$H NMR (400 MHz, $CD_3OD$) δ 8.71 (d, *J*= 4.8 Hz, 2H), 8.52 (d, *J*= 1.6 Hz, 1H), 7.83 (d, *J* = 8.4 Hz, 1H), 7.71 (d, *J* = 8.4 Hz, 1H), 7.27 (t, *J*= 4.8 Hz, 1H), 5.99 (q, *J* = 7.2 Hz, 1H), 5.18 (dd, *J*= 7.2 Hz, 6.0 Hz, 1H), 2.08-1.93 (m, 2H), 1.90 (d, *J*= 7.2 Hz, 3H). 0.96 (t, *J*= 7.2 Hz, 3H). LCMS (ES,

*m*/*z):* 470.2 $[M+H]^+$. The slower-eluting enantiomer was obtained at 20.71 min. The collected fractions were combined and concentrated under reduced pressure to afford enantiomer Example 74: Enantiomer AB: $^1$H NMR (400

MHz, $CD_3OD$) δ 8.80 (d, *J*= 4.8 Hz, 2H), 8.75 (d, *J*= 1.6 Hz, 1H), 8.00 (dd, *J*= 8.0 Hz, 1.6 Hz, 1H), 7.79 (d, *J*= 8.0 Hz, 1H), 7.39 (t, *J*= 4.8 Hz, 1H), 5.99 (q, *J*= 6.8 Hz, 1H), 5.18 (t, *J* = 6.4 Hz, 1H), 2.06-1.88 (m, 2H), 1.86 (d, *J* = 7.2 Hz, 3H). 0.85 (t, *J*= 7.2 Hz, 3H). LCMS (ES, *m*/*z*): 470.2$[M+H]^+$.

EXAMPLES 75-78

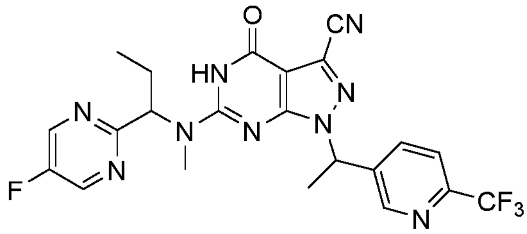

6-((1-(5-fluoropyrimidin-2-yl)propyl)(methyl)amino)-4-oxo-1-(1-(6-(trifluoromethyl)pyridin-3-yl)ethyl)-4,5-dihydro-1*H*-pyrazolo[3,4-*d*]pyrimidine-3-carbonitrile

Step A : 6-((1-(5-fluoropyrimidin-2-yl)propyl)(methyl)amino)-5-(4-methoxybenzyl)-4- oxo-1-(1-(6-(trifluoromethyl)pyridin-3-yl)ethyl)-4,5-dihydro-1*H*-pyrazolo[3,4-*d*]pyrimidine-3-carbonitrile. The title compound was prepared as described for Example 71-74 using *N*-methyl-1-(pyrimidin-2-yl)propan-1-amine hydrochloride. LCMS (ES, *m*/*z*): 622.2$[M+H]^+$. Step B: 6-((1-(5-fluoropyrimidin-2-yl)propyl)(methyl)amino)-4-oxo-1-(1-(6- (trifluoromethyl)pyridin-3-yl)ethyl)-4,5-dihydro-1*H*-pyrazolo[3,4-*d*]pyrimidine-3-carbonitrile. 6-((1-(5-fluoropyrimidin-2-yl)propyl)(methyl)amino)-5-(4-methoxybenzyl)-4-oxo-1-(1-(6-(trifluoromethyl)pyridin-3-yl)ethyl)-4,5-dihydro-1*H*-pyrazolo[3,4-*d*]pyrimidine-3-carbonitrile (110 mg, 0.177 mmol) was dissolved in TFA (3 ml) at room temperature. The reaction solution was stirred overnight at room temperature. The residue was purified by PREP-HPLC with the following conditions: Column: X Bridge C18 OBD Prep Column 100Å, 10 μm, 19 mm × 250 mm; Mobile Phase A: Water (20 mmol/L $NH_4HCO_3$), Mobile Phase B: ACN; Flow rate: 20 mL/min; Gradient: 50% B to 51% B in 16 min; 254/210 nm; $RT_1$: 10.12 min, $RT_2$: 11.10 min. The fractions containing desired product were combined and concentrated under reduced pressure to afford mixture A and mixture B. The mixture A was separated by Prep-Chiral-HPLC with the following conditions: Column: CHIRALPAK IC, 2*25 cm, 5 um; Mobile Phase A: HEX:DCM = 3:1--HPLC, Mobile Phase B: EtOH--HPLC; Flow rate: 16 mL/min; Gradient: 50 B to 50 B in 15 min; 254/220 nm; $RT_1$: 6.94 min; $RT_2$:10.56 min. The faster-eluting enantiomer was obtained at 6.94 min. The collected fractions were combined and concentrated under reduced pressure to afford Example 75: $^1H$ NMR (300 MHz, $CD_3OD$) δ 8.75-8.68 (m, 3H), 8.04-7.94 (m, 1H), 7.79 (d, *J*= 8.1 Hz, 1H), 6.04 (q, *J*= 7.2 Hz, 1H), 5.86-5.79 (m, 1H), 3.08 (s, 3H), 2.45-2.29 (m, 1H), 2.14-1.97 (m, 1H), 1.96 (d, *J* = 7.2 Hz, 3H), 0.95 (t, *J*= 7.4 Hz, 3H). LCMS (ES, *m*/*z):* 502.3 $[M+H]^+$. The slower-eluting enantiomer was obtained at 10.56 min. The collected fractions were combined and concentrated under reduced pressure to afford Example 76: $^1H$ NMR (300 MHz, $CD_3OD$) δ 8.81-8.57 (m, 3H), 8.03-7.88 (m, 1H), 7.79 (d, *J*= 8.4 Hz, 1H), 6.04 (q, *J*= 7.2 Hz, 1H), 5.93-5.76 (m, 1H), 3.07 (s, 3H), 2.48-2.26 (m, 1H), 2.17-2.02 (m, 1H), 1.96 (d, *J*= 7.2 Hz, 3H), 0.95 (t, *J*= 7.4 Hz, 3H). LCMS (ES, m/z): 502.3$[M+H]^+$. The mixture B was separated by Prep-Chiral-HPLC with the following conditions: Column: CHIRALPAK IC, 2*25 cm, 5 um; Mobile Phase A: Hex--HPLC, Mobile Phase B: EtOH-HPLC; Flow rate: 20 mL/min; Gradient: 30 B to 30 B in 18 min; 254/220 nm; $RT_1$: 11.68 min; $RT_2$: 14.81 min. The faster-eluting enantiomer was obtained at 11.68 min. The collected fractions were combined and concentrated under reduced pressure to afford Example 77: EnantiomerBA: $^1H$ NMR (300 MHz, $CD_3OD$) δ 8.70-8.59 (m, 3H), 7.96-7.87 (m, 1H), 7.73 (d, *J*= 8.1 Hz, 1H), 6.04 (q, *J*= 7.2 Hz, 1H), 5.87-5.80 (m, 1H), 3.15 (s, 3H), 2.42-2.27 (m, 1H), 2.15-1.97 (m, 1H), 1.95 (d, J= 7.2 Hz, 3H), 1.05 (t, *J*= 7.4 Hz, 3H). LCMS (ES, *m*/*z):* 502.3 $[M+H]^+$. The slower-eluting isomer was obtained at 14.81 min. The collected fractions were combined and concentrated under reduced pressure to afford Example 78: $^1H$ NMR (300 MHz, $CD_3OD$) δ 8.70-8.59 (m, 3H), 7.96-7.87 (m, 1H), 7.73 (d, *J* = 8.1 Hz, 1H), 6.04 (q, *J* = 7.2 Hz, 1H), 5.87-5.81 (m, 1H), 3.14 (s, 3H), 2.42-2.27 (m, 1H), 2.15-2.00 (m, 1H), 1.95 (d, *J*= 7.2 Hz, 3H), 1.05 (t, *J*= 7.4 Hz, 3H). LCMS (ES, m/z): 502.2 $[M+H]^+$.

INTERMEDIATE 20

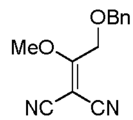

2-(2-(Benzyloxy)-1-methoxyethylidene)malononitrile

Step A: 2-(Benzyloxy)acetyl chloride. Into a 5000 mL 4-necked round-bottom flask purged and maintained with an inert atmosphere of nitrogen, was placed 2-(benzyloxy)-acetic acid (500 g, 3.01 mol), dichloromethane (2500 mL), and N,N-dimethylformamide (5 mL). This was followed by the addition of oxalic dichloride (458.3 g, 3.61 mol) at room temperature. The resulting solution was stirred overnight at room temperature. The resulting mixture was concentrated under vacuum. The residue was diluted with 200 mL of DCM. The resulting mixture was concentrated under vacuum. The residue was diluted with 200 mL of DCM. The resulting mixture was concentrated under vacuum. The product was used in next step without further purification.

Step B: 2-[2-(Benzyloxy)-1-hydroxyethylidene]propanedinitrile. Into a 3000 mL 4-necked round-bottom flask purged and maintained with an inert atmosphere of nitrogen, was placed tetrahydrofuran (1100 mL), propanedinitrile (195 g, 2.95 mol). This was followed by the addition of sodium hydride (236.16 g, 5.90 mol, 60%) in several batches at 0°C over 20 min. To this was added 2-(benzyloxy)acetyl chloride (545 g, 2.95 mol) dropwise with stirring at 0°C. The resulting solution was stirred for 2 h at 0°C. The reaction was then quenched by the addition of 500 mL of water at 0°C. The resulting solution was diluted with 2000 mL of hydrogen chloride (1mol/L). The resulting solution was extracted with 3 × 1000 mL of ethyl acetate and the organic layers combined. The resulting mixture was washed with 2 × 1000 mL of brine. The mixture was dried over anhydrous magnesium sulfate and concentrated under vacuum. The residue was applied onto a silica gel column with ethyl acetate/petroleum ether (1:10) to afford the title compound. LCMS (ES, m/z): 215.2 $[M+H]^+$.

Step C: 2-[2-(benzyloxy)-1-methoxyethylidene]propanedinitrile. Into a 20L 4-necked round-bottom flask purged and maintained with an inert atmosphere of nitrogen, was placed 2-[2-(benzyloxy)-1-hydroxyethylidene]propanedinitrile (457 g, 2.13 mol), dioxane (7000 mL), sodium bicarbonate (609.3 g, 7.25 mol), dimethyl sulfate (376.7 g, 2.99 mol). The resulting solution was stirred overnight at 85°C. The solids were filtered out. The filtrate was concentrated under vacuum. The resulting solution was diluted with 2000 mL of water and then extracted with 3 × 2000 mL of ethyl acetate. The organic layers were combined and washed with 2 × 1000 mL of brine. The mixture was dried over anhydrous magnesium sulfate and concentrated under vacuum. The residue was applied onto a silica gel column with ethyl acetate/petroleum ether (1:10) and afforded the title compound. LCMS (ES, m/z): 229.0 $[M+H]^+$.

## INTERMEDIATE 21

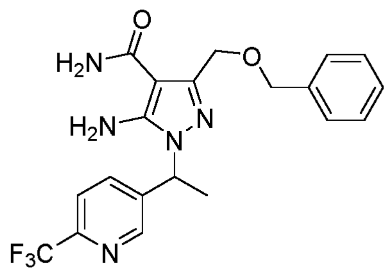

5-amino-3-((benzyloxy)methyl)-1-(1-(6-(trifluoromethyl)pyridin-3-yl)ethyl)-1H-pyrazole-4-carboxamide

Step A: tert-Butyl 2-(1-(6-(trifluoromethyl)pyridin-3-yl)ethyl)hydrazinecarboxylate. Acetic acid (1.589 ml, 27.8 mmol) was added to a stirred mixture of tert-butyl carbazate (11.53 g, 87 mmol) and 1-(6-(trifluoromethyl)pyridine-3-yl)ethanone (15 g, 79 mmol) in ethanol (375 ml) at ambient temperature and the mixture was stirred at 80°C for 2 h. The solvent was removed under reduced pressure and acetonitrile (375 ml) and acetic acid (18.16 ml, 317 mmol) were added at ambient temperature followed by sodium cyanoborohydride (9.97 g, 159 mmol). The reaction was heated at 80°C for 48 hours. The solvent was then was removed under vacuum. The crude residue was dissolved in $Et_2O/H_2O$ (150:50 ml) and pH brought to 13 by carefully adding solid KOH. The organic layer was separated, washed with brine (1 × 100 mL) dried over $Na_2SO_4$ and concentrated under reduced pressure to afford the title compound. LCMS (ES, m/z): 305 $[M+H]^+$.

Step B: 5-(1-Hydrazinylethyl)-2-(trifluoromethyl)pyridine dihydrochloride. Tert-butyl 2-(1-(6-(trifluoromethyl)pyridin-3-yl)ethyl)hydrazine carboxylate (30 g, 63.9 mmol) was dissolved in dioxane (150 ml) and hydrogen chloride (192 ml, 766 mmol) was added. After 48 h a precipitate formed and the mixture was concentrated under reduced pressure to afford the title compound. LCMS (ES, m/z): 205 $[M+H]^+$.

Step C: 5-Amino-3-((benzyloxy)methyl)-1-(1-(6-(trifluoromethyl)pyridin-3-yl)ethyl)- 1H-pyrazole- 4-carbonitrile. To a stirred, 0°C solution of 5-(1-hydrazinylethyl)-2-(trifluoromethyl)pyridine dihydrochloride (20 g, 71.9 mmol) and 2-(2-(benzyloxy)-1-methoxyethylidene)malononitrile (19.70 g, 86 mmol) in methanol (25 ml) was added sodium methoxide solution (0.5M, 302 ml, 151 mmol). The ice bath was removed and the solution was allowed to stir at ambient temperature for 30 minutes. The solvent was removed under vacuum and the crude reaction mixture was diluted with 150 mL water and 500 mL ethyl acetate. The organic layer was separated and the aqueous phase was extracted with ethyl acetate (1 × 150 mL). The combined organic layers were dried over $Na_2SO_4$ and concentrated under vacuum. The residue was purified by silica gel chromatography (10-40% EtOAc in hexanes) to afford the title compound. LCMS (ES, m/z): 402.2 $[M+H]^+$.

Step D: 5-Amino-3-((benzyloxy)methyl)-1-(1-(6-(trifluoromethyl)pyridin-3-yl)ethyl)- 1H-pyrazole- 4-carboxamide. To 5-amino-3-((benzyloxy)methyl)-1-(1-(6- (trifluoromethyl)pyridin-3-yl)ethyl)- 1H-pyrazole-4-carbonitrile (25.4 g, 63.3 mmol) in EtOH (90 ml) was added 30 % aqueous hydrogen peroxide (32.6 ml, 316 mmol) solution followed by addition of ammonium hydroxide (85 ml, 633 mmol) at 0°C. The ice bath was removed and the solution was stirred at ambient temperature for 48 h. The reaction mixture was cooled to 0°C, slowly quenched with enough ( ~ 3 eq.) saturated aqueous sodium thiosulfate solution to quench the peroxide. The solvent was removed under vacuum and to the residue was added 200 mL water, and extracted with ethyl acetate (2x 250 mL). The combined organic layers were dried over $Na_2SO_4$, filtered and evaporated under vacuum to afford the title compound (racemic). LCMS (ES, m/z): 420.4 $[M+H]^+$. This material was resolved using chiral preparative SFC (AS-H, 21 × 250 mm column, 40% MeOH co-solvent) to provide peak 1 (Intermediate 21 - S isomer, stereochemistry determined by VCD), LCMS (ES, m/z): 420.2 $[M+H]^+$ and peak 2 (Intermediate 21 - *R* isomer, stereochemistry determined by VCD), LCMS (ES, m/z): 420.2 $[M+H]^+$.

INTERMEDIATE 22

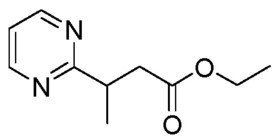

Ethyl 3-(pyrimidin-2-yl)butanoate

Step A: To a solution of ethyl acetate (2.89 ml, 29.5 mmol) in THF (60 ml) was added LiHMDS (22.93 ml, 22.93 mmol, 1M in THF) at -78°C. The reaction mixture was stirred at -78 °C for 1 h. 2-Acetylpyrimidine (2 g, 16.38 mmol) in THF (15ml) was added to reaction mixture at -78 °C. The reaction mixture was stirred at -78°C to room temperature for 4 h. The mixture was cooled, aqueous ammonium chloride (20%, 50 ml) was added and the mixture was extracted with ethyl acetate (3x15 ml). The combined organic fractions were washed with aqueous ammonium chloride (10%, 3 × 50 ml), dried ($Na_2SO_4$), filtered and the solvent was evaporated under reduced pressure. The residue was purified by column chromatography on silica gel eluting with EtOAc/hexanes (0 to 100%) to give ethyl 3-hydroxy-3-(pyrimidin-2-yl)butanoate.

Step B: To a solution of ethyl 3-hydroxy-3-(pyrimidin-2-yl)butanoate (1.8g, 8.56 mmol) in $CH_2Cl_2$ (15 ml), $SOCl_2$ (1.4 ml, 19.18 mmol) was added at 0°C. The reaction mixture was stirred at 0°C for 30 min. $Et_3N$ (2.5 ml, 17.94 mmol) was added to reaction mixture at 0°C. The reaction mixture was stirred at 0 °C and warmed to room temperature over 1 h. The mixture was cooled, aqueous ammonium chloride (20 %, 50ml) was added and the mixture was extracted with ethyl acetate (3x50 ml). The combined organic fractions were washed with aqueous ammonium chloride (saturated, 50 ml), dried ($Na_2SO_4$), filtered and the solvent was evaporated under reduced pressure. The residue was purified by column chromatography on silica gel eluting with EtOAc/hexanes (0 to 70%) to give ethyl 3-(pyrimidin-2-yl)but-2-enoate. LC-MS (m/z): 193.1 $[M+H]^+$

Step C: To a solution of ethyl 3-(pyrimidin-2-yl)but-2-enoate (124 mg, 0.65 mmol) in EtOH (5 ml), was added 10% Pd-C (7 mg) and $Et_3N$ (0.18 ml, 1.29 mmol) at room temperature. The reaction mixture was stirred under an atmosphere of $H_2$ at room temperature, overnight. The reaction mixture was filtered, and the filtrate concentrated under vacuum. To the residue was added aqueous ammonium chloride (20%, 20mL) and extracted with ethyl acetate (2x30 ml). The combined organic fractions were washed with aqueous ammonium chloride (saturated, 20 ml), dried ($Na_2SO_4$), filtered and the solvent was evaporated under reduced pressure. The residue was purified by column chromatography on silica gel eluting with EtOAc/hexanes (0 to 100%) to give ethyl 3-(pyrimidin-2-yl)butanoate. LC-MS (m/z): 195.1 $[M+H]^+$

EXAMPLES 79-80

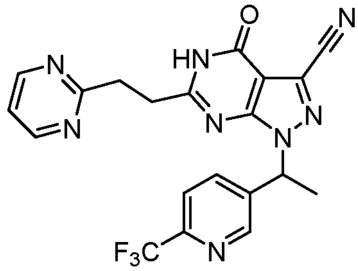

4-oxo-6-(2-(pyrimidin-2-yl)ethyl)-1-(1-(6-(trifluoromethyl)pyridin-3-yl)ethyl)-4,5-dihydro-1H-pyrazolo[3,4-d]pyrimidine-3-carbonitrile

Step 1: To a mixture of (E)-methyl 3-(4,4,5,5-tetramethyl-1,3,2-dioxaborolan-2-yl)acrylate (7.56 g, 35.7 mmol), 2-bromopyrimidine (5.4 g, 34.0 mmol) and $Cs_2CO_3$ (11.07 g, 34.0 mmol) in THF (35 ml) and water (10 ml) at room temperature under nitrogen, was added 1,1'-bis(di-tert-butylphosphino)ferrocene palladium dichloride (0.664 g, 1.019 mmol). The reaction mixture was stirred at 50°C for 4 h. The mixture was then cooled; water (40 mL) was added and extracted with ethyl acetate (3 × 50mL). The combined organic layer was washed with aqueous ammonium chloride (saturated, 2 x30 mL), dried ($Na_2SO_4$), filtered and the solvent was evaporated under reduced pressure. The residue was purified by silica column chromatography eluting with 0-100% EtOAc/hexanes to provide 4.1g of methyl 3-(pyrimidin-2-yl)acrylate.

Step 2: To a well stirred mixture of 5-amino-3-((benzyloxy)methyl)-1-(1-(6-(trifluoromethyl)pyridin-3-yl)ethyl)-1H-pyrazole-4-carboxamide (S-isomer, 300 mg, 0.715 mmol) and NaH (60%, 86 mg, 2.15 mmol) in a microwave vial under $N_2$, at 0°C, was added EtOH (5 ml) slowly. After 15 min of stirring at 0°C, a solution of methyl 3-(pyrimidin-2-yl)acrylate (352 mg, 2.15 mmol) in EtOH (4 ml) was added slowly. The reaction stirred at RT, under $N_2$ for a few minutes. The reaction vial was capped and heated in a microwave reactor at 150°C for 30 min. The reaction mixture was cooled to RT and concentrated. The residue was diluted with $CH_2Cl_2$ (30 ml), and saturated $NH_4Cl$ solution (50 ml). The organic layer was separated, and the aqueous layer was extracted with $CH_2Cl_2$ (30 ml). The combined organic layer was dried ($Na_2SO_4$), filtered and concentrated. The residue was purified by column chromatography (Redisep 24g gold cartridge) eluting with 0-5% of MeOH/CH2Cl2 to provide 190 mg of 3-((benzyloxy)methyl)-6-(2-(pyrimidin-2-yl)vinyl)-1-(1-(6-(trifluoromethyl)pyridin-3-yl)ethyl)-1H-pyrazolo[3,4-d]pyrimidin-4(SH)-one. LCMS (ES, m/z): 534.5 $[M+H]^+$

Step 3: To 3-((benzyloxy)methyl)-6-(2-(pyrimidin-2-yl)vinyl)-1-(1-(6-(trifluoromethyl)pyridin-3-yl)ethyl)-1H-pyrazolo[3,4-d]pyrimidin-4(5H)-one (187 mg, 0.35 mmol) was added MeOH (21 ml)/THF (4 ml)/DCM (4 ml). The slurry was combined with 10% Pd/C (tip of spatula), and hydrogenated using an $H_2$ filled balloon at RT. After 6 hr the reaction was stopped, and the solids filtered off. The filtrate was concentrated and purified by column chromatography (Redisep 24g gold cartridge) eluting with 0-8% of MeOH/$CH_2Cl_2$ to provide 82 mg of 3-((benzyloxy)methyl)-6-(2-(pyrimidin-2-yl)ethyl)-1-(1-(6-(trifluoromethyl)pyridin-3-yl)ethyl)-1H-pyrazolo[3,4-d]pyrimidin-4(5H)-one. LCMS (ES, m/z): 536.5 $[M+H]^+$

Step 4: To 3-((benzyloxy)methyl)-6-(2-(pyrimidin-2-yl)ethyl)-1-(1-(6-(trifluoromethyl)pyridin-3-yl)ethyl)-1H-pyrazolo[3,4-d]pyrimidin-4(5H)-one (81 mg, 0.15 mmol) in DCM (2 ml) at -78°C was added a solution of $BCl_3$ (1M in DCM, 0.605 ml, 0.605 mmol) dropwise. The reaction was maintained at -78°C for 30 min at which point the cold bath was removed and replaced with an ice bath (0°C). The reaction was then maintained at 0°C, with stirring, for 30 min before cooling the reaction mixture to -78°C. The reaction was quenched by dropwise addition of MeOH (1 ml) with vigorous stirring. The reaction then warmed to 0°C, and neutralized with aq $NH_4OH$ (dropwise addition) and diluted with $CH_2Cl_2$ (5-10 ml). $Na_2SO_4$ was added, and the reaction was filtered and concentrated to provide 3-(hydroxymethyl)-6-(2-(pyrimidin-2-yl)ethyl)-1-(1-(6-(trifluoromethyl)pyridin-3-yl)ethyl)-1H-pyrazolo[3,4-d]pyrimidin-4(5H)-one that was taken forward without further purification. LCMS (ES, m/z): 446.4 $[M+H]^+$

Step 5: To 3-(hydroxymethyl)-6-(2-(pyrimidin-2-yl)ethyl)-1-(1-(6-(trifluoromethyl)pyridin- 3-yl)ethyl)-1H-pyrazolo[3,4-d]pyrimidin-4(5H)-one (66 mg, 0.15 mmol) in $CH_2Cl_2$ (5 ml) at RT, under $N_2$ atmosphere, was added Dess-Martin periodinane (63 mg, 0.15 mmol). The reaction was stirred at RT for 10 min and then quenched by addition of aq 10% $Na_2S_2O_3$ solution (15 ml). The reaction was aged for a few minutes, and then saturated aqueous NaHCOs solution (15 ml), and EtOAc (25 ml) were added. The organic layer was separated. The aqueous layer was extracted with EtOAc (25 ml). The combined organic portions were dried ($Na_2SO_4$), filtered and concentrated to provide 4-oxo-6-(2-(pyrimidin-2-yl)ethyl)-1- (1-(6-(trifluoromethyl)pyridin-3-yl)ethyl)-4,5-dihydro-1H-pyrazolo[3,4-d]pyrimidine-3-carbaldehyde that was taken forward as is. LCMS (ES, m/z): 444.4 $[M+H]^+$

Step 6: To a vial containing 4-oxo-6-(2-(pyrimidin-2-yl)ethyl)-1-(1-(6-(trifluoromethyl)pyridin-3-yl)ethyl)-4,5-dihydro-1H-pyrazolo[3,4-d]pyrimidine-3-carbaldehyde (31 mg, 0.07 mmol) was added MeCN (92 ml). To this was added hydroxylamine hydrochloride (7.3 mg, 0.105 mmol) and DIPEA (0.024 ml, 0.14 mmol) dropwise. The reaction was stirred for a minute before 2,4,6-tripropyl-1,3,5,2,4,6-trioxatriphosphinane 2,4,6-trioxide (50 wt% in EtOAc, 0.083 ml, 0.14 mmol)

was added dropwise. The vial was capped and heated to 60°C for 10 min at which point it was then cooled to RT, concentrated, and placed under vacuum overnight. The reaction residue was diluted with in MeCN (3 ml) and 2,4,6-tripropyl-1,3,5,2,4,6-trioxatriphosphinane 2,4,6-trioxide (50 wt% in EtOAc, ~0.1 ml, ~0.1 mmol) was added. The reaction was heated to 60°C for 3 hrs and then cooled to RT, concentrated, and purified by column chromatography (redisep 12g gold cartridge) eluting with 0-7% of $MeOH/CH_2Cl_2$. The fractions containing the product were combined and concentrated. The material was diluted with $CH_2Cl_2$ (25 ml), cooled to 0°C and washed with satd $NaHCO_3$ (2 × 25 ml). The $CH_2Cl_2$ layer was dried (Na2SO4) filtered and concentrated to provide 4-oxo-6-(2-(pyrimidin-2-yl)ethyl)-1-(1-(6-(trifluoromethyl)pyridin-3-yl)ethyl)-4,5-dihydro-1H-pyrazolo[3,4-d]pyrimidine-3-carbonitrile. LCMS (ES, m/z): 441.4 $[M+H]^+$

The title compounds were obtained by chiral preparative SFC (OJ-H, 21x250 mm column, 50% MeOH). The faster-eluting isomer (peak 1/isomer 1) provided Example 79. 1H NMR (400 MHz, Chloroform-d) δ 12.01 (br.s, 1H), 8.78 - 8.76 (m, 3H), 7.95 (d, J = 7.3 Hz, 1H), 7.67 (d, J = 8.1 Hz, 1H), 7.29-7.27 (m, 1H), 6.17 (q, J = 7.0 Hz, 1H), 3.55 (br.d, 2H), 3.31 (br.s, 2H), 2.00 (d, J = 7.1 Hz, 3H). LCMS (ES, m/z): 441.4 $[M+H]^+$.

The slower-eluting isomer (peak 2/isomer 2) provided Example **80.** 1H NMR (400 MHz, Chloroform-d) δ 12.03 (br.s, 1H), 8.78-8.75 (m, 3H), 7.95 (d, J = 7.3 Hz, 1H), 7.66 (d, J = 7.7 Hz, 1H), 7.30-7.26 (br.s, 1H), 6.17 (br.d, J = 6.9 Hz, 1H), 3.56 (br.s, 2H), 3.33 (br.s, 2H), 2.00 (d, J = 7.1 Hz, 3H). LCMS (ES, m/z): 441.4 $[M+H]^+$.

EXAMPLES 81-82

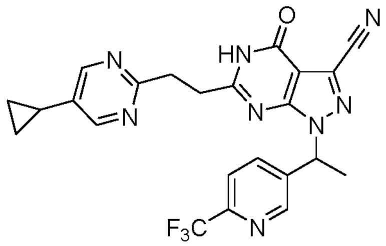

6-(2-(5-cyclopropylpyrimidin-2-yl)ethyl)-4-oxo-1-(1-(6-(trifluoromethyl)pyridin-3-yl)ethyl)-4,5-dihydro-1H-pyrazolo[3,4-d]pyrimidine-3-carbonitrile

Step 1: To a mixture of 2-chloro-5-cyclopropylpyrimidine (464 mg, 3 mmol) and (E)-ethyl 3-(4,4,5,5-tetramethyl-1,3,2-dioxaborolan-2-yl)acrylate (1.02g, 4.5 mmol) in a vial was added XPHOS G2 biaryl Pd catalyst (236 mg, 0.1 mmol). The tube was purged with $N_2$ before adding dioxane (12 ml). A solution of potassium phosphate monohydrate (2.07 g, 3 mmol) in water (3 ml) was added and the reaction was purged again with $N_2$. The reaction was heated to 72°C and aged overnight before it was cooled to RT. The reaction aged at room temperature before removing the water layer. The reaction mixture was filtered to remove the solids. The filtrate was concentrated and purified by column chromatography on silica gel (redisep gold 40g silica cartridge) eluting with 0-60% of EtOAc/hexanes to provide ethyl 3-(5-cyclopropylpyrimidin-2-yl)acrylate. LCMS (ES, m/z): 219.2 $[M+H]^+$

Step 2: To a solution of ethyl 3-(5-cyclopropylpyrimidin-2-yl)acrylate (460 mg, 2.1 mmol) in EtOH (20 ml) was added $Et_3N$ (0.88 ml, 6.3 mmol) and Pd/C (10%, tip of spatula). The material was hydrogenated at RT, using balloon filled with $H_2$ gas. After 2 hrs the catalyst was filtered off, and rinsed with EtOH (10 ml). The combined filtrate was concentrated and purified by column chromatography on silica gel (redisep gold 40g silica cartridge) eluting with 0-70% of EtOAc/hexanes to provide ethyl 3-(5-cyclopropylpyrimidin-2-yl)propanoate. LCMS (ES, m/z): 221.1 $[M+H]^+$

Step 3: To a stirred solution of ethyl 3-(5-cyclopropylpyrimidin-2-yl)pentanoate (370 mg, 1.68 mmol) and 5-amino-3-((benzyloxy)methyl)-1-(1-(6-(trifluoromethyl)pyridin- 3-yl)ethyl)-1H-pyrazole-4-carboxamide (Intermediate 2, S-isomer, 250 mg, 0.596 mmol) in EtOH (11 ml) in a microwave vial under $N_2$, was added NaOEt in EtOH (21 wt%, 2.8 ml, 1.79 mmol). The vial was capped, stirred for a few minutes and heated in a microwave reactor to 100°C for 60 min. The reaction was cooled to RT, and concentrated. The residue was diluted in aq $NH_4Cl$ (40 ml) and extracted with $CH_2Cl_2$ (2 × 40 ml). The combined organic portions were dried ($Na_2SO_4$), filtered and concentrated. The crude material was purified by column chromatography on silica gel (redisep gold 24g silica cartridge) eluting with 0-80% of 9:1 $CH_2Cl_2$ : MeOH / $CH_2Cl_2$ to provide 3-((benzyloxy)methyl)-6-(2-(5-cyclopropylpyrimidin-2-yl)ethyl)-1- (1-(6-(trifluoromethyl)pyridin-3-yl)ethyl)-1H-pyrazolo[3,4-d]pyrimidin-4(5H)-one. LCMS (ES, m/z): 576.4 $[M+H]^+$

Step 4: To 3-((benzyloxy)methyl)-6-(2-(5-cyclopropylpyrimidin-2-yl)ethyl)- 1-(1-(6-(trifluoromethyl)pyridin-3-yl)ethyl)-1H-pyrazolo[3,4-d]pyrimidin-4(5H)-one (100 mg, 0.174 mmol) in DCM at -78°C was added a solution of $BCl_3$ (1M in DCM, 0.7 ml, 0.7 mmol) dropwise. The reaction was maintained at -78°C for 30 min. Then cold bath was removed, and replaced with an ice bath (0°C). The reaction was then maintained at 0°C, with stirring, for 30-45 min before it was

cooled to -78°C, diluted with $CH_2Cl_2$ (5 ml) and quenched by dropwise addition of MeOH (~1 ml) with vigorous stirring. The reaction mixture was warmed to 0°C, and neutralized with aq $NH_4OH$ (dropwise addition). It was then diluted with $CH_2Cl_2$ (5-10 ml), dried with $Na_2SO_4$, and filtered. The filtrate was concentrated to provide 6-(2-(5-cyclopropylpyrimidin-2-yl)ethyl)-3-(hydroxymethyl)-1-(1-(6-(trifluoromethyl)pyridin-3-yl)ethyl)-1H-pyrazolo[3,4-d]pyrimidin-4(5H)-one that was used without further purification. LCMS (ES, m/z): 486.3 $[M+H]^+$

Step 5: To 6-(2-(5-cyclopropylpyrimidin-2-yl)ethyl)-3-(hydroxymethyl)-1- (1-(6-(trifluoromethyl)pyridin-3-yl)ethyl)-1H-pyrazolo[3,4-d]pyrimidin-4(5H)-one (all from Step 4, 0.174 mmol) in $CH_2Cl_2$ (5 ml) at RT, under $N_2$ atmosphere, was added Dess-Martin periodinane (74 mg, 0.174 mmol). The reaction stirred at RT for 15 min before it was quenched by addition of aq 10% $Na_2S_2O_3$ solution (15 ml). The reaction was aged for a few minutes, before saturated aqueous $NaHCO_3$ solution (15 ml) and EtOAc (25 ml) were added. The reaction was aged for a few minutes, and the organic layer was separated. The aqueous layer was extracted with EtOAc (25 ml) and the combined organic portions were dried ($Na_2SO_4$), filtered and concentrated to provide 6-(2-(5-cyclopropylpyrimidin-2-yl)ethyl)-4-oxo-1-(1-(6-(trifluoromethyl)pyridin-3-yl)ethyl)-4,5-dihydro-1H-pyrazolo[3,4-d]pyrimidine-3-carbaldehyde that was used without further purification. LCMS (ES, m/z): 484.3 $[M+H]^+$

Step 6: To a vial containing 6-(2-(5-cyclopropylpyrimidin-2-yl)ethyl)-4-oxo-1-(1-(6-(trifluoromethyl)pyridin-3-yl)ethyl)-4,5-dihydro-1H-pyrazolo[3,4-d]pyrimidine-3-carbaldehyde (from Step 5, 74 mg, 0.153 mmol) in MeCN (4 ml) was added hydroxylamine HCl (25 mg, 0.36 mmol) and then DIPEA (0.1 ml, 0.573 mmol) dropwise. The reaction was stirred for a minute before 2,4,6-tripropyl-1,3,5,2,4,6-trioxatriphosphinane 2,4,6-trioxide (50 wt% in EtOAc, 0.3 ml, 0.504 mmol) was added dropwise. The vial was capped and heated to 60°C for ~6 hrs. It was then cooled to RT and concentrated. The residue was diluted with $CH_2Cl_2$ (25 ml), washed with saturated aqueous $NH_4Cl$ (25 ml) and saturated aqueous NaHCOs (2 × 25 ml). The $CH_2Cl_2$ layer was dried ($Na_2SO_4$) filtered and concentrated. The residue was purified by reverse phase HPLC. Conditions: Sunfire Prep C-18, 10 uM, 30x150 mm OBD column, flow rate 30 ml/min, gradient = 10 - 90 % $CH_3CN$(0.05% TFA) in water (0.05% TFA) to provide 6-(2-(5-cyclopropylpyrimidin-2-yl)ethyl)-4-oxo-1-(1-(6-(trifluoromethyl)pyridin-3-yl)ethyl)-4,5-dihydro-1H-pyrazolo[3,4-d]pyrimidine-3-carbonitrile.

The title compounds were obtained by chiral preparative SFC (AD-H, 21x250 mm column, 35% EtOH). The faster-eluting isomer (peak 1/isomer 1) provided Example **81.** $^1$H NMR (500 MHz, Chloroform-d) δ 12.16 (s, 1H), 8.79 (s, 1H), 8.51 (s, 2H), 7.98 (d, J = 8.2 Hz, 1H), 7.69 (d, J = 8.2 Hz, 1H), 6.19 (q, J = 7.2 Hz, 1H), 3.47 (br.d, J = 6.9 Hz, 2H), 3.26 (t, J = 6.1 Hz, 2H), 2.02 (d, J = 7.1 Hz, 3H), 1.15 (app.q, J = 5.7 Hz, 2H), 0.82 (app.q, J = 5.6 Hz, 2H). LCMS (ES, m/z): 481.3 $[M+H]^+$.

The slower-eluting isomer (peak 2/isomer 2) provided Example **82.** $^1$H NMR (500 MHz, Chloroform-d) δ 12.22 (s, 1H), 8.79 (s, 1H), 8.51 (s, 2H), 7.98 (d, J = 7.6 Hz, 1H), 7.69 (d, J = 8.2 Hz, 1H), 6.19 (q, J = 7.2 Hz, 1H), 3.47 (br.d, J = 6.7 Hz, 2H), 3.26 (t, J = 6.1 Hz, 2H), 2.02 (d, J = 7.2 Hz, 3H), 1.14 (app.q, J = 5.8 Hz, 2H), 0.81 (app.q, J = 5.5 Hz, 2H). LCMS (ES, m/z): 481.3 $[M+H]^+$.

TABLE 4

| The following compounds were prepared according to the above procedures herein using known or prepared starting materials, as described in the reaction schemes and examples herein. The requisite starting materials are either prepared as described in the intermediates section, commercially available, or may be prepared from commercially available reagents using conventional reactions well known in the art without undue experimentation. | | | | |
|---|---|---|---|---|
| Example | Structure | Name | LCMS $(M+H)^+$ | SFC / Peak# |
| 83 | 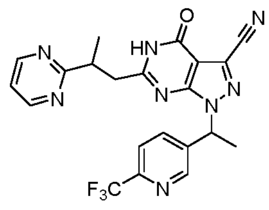 Isomer 1A | 4-oxo-6-(2-(pyrimidin-2-yl) propyl)-1-(1-(6-(trifluoromethyl)pyridin-3-yl)ethyl)-4,5-dihydro-1H-pyrazolo[3,4-d] pyrimidine-3-carbonitrile | 455.3 | AD-H, 21x250 mm column, 30% EtOH - Peak 1 was further separated by OJ-H, 21x250 mm column, 40% MeOH / Peak 1 |

(continued)

| The following compounds were prepared according to the above procedures herein using known or prepared starting materials, as described in the reaction schemes and examples herein. The requisite starting materials are either prepared as described in the intermediates section, commercially available, or may be prepared from commercially available reagents using conventional reactions well known in the art without undue experimentation. | | | | |
|---|---|---|---|---|
| Example | Structure | Name | LCMS $(M+H)^+$ | SFC / Peak# |
| 84 | 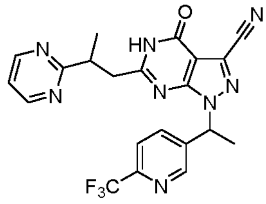 Isomer 1B | 4-oxo-6-(2-(pyrimidin-2-yl) propyl)-1-(1-(6-(trifluoromethyl)pyridin-3-yl)ethyl)-4,5-dihydro-1H-pyrazolo[3,4-d] pyrimidine-3-carbonitrile | 455.2 | AD-H, 21x250 mm column, 30% EtOH - Peak 1 was further separated by OJ-H, 21x250 mm column, 40% MeOH / Peak 2 |
| 85 | 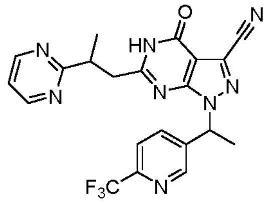 Isomer 2 | 4-oxo-6-(2-(pyrimidin-2-yl) propyl)-1-(1-(6-(trifluoromethyl)pyridin-3-yl)ethyl)-4,5-dihydro-1H-pyrazolo[3,4-d] pyrimidine-3-carbonitrile | 455.2 | AD-H, 21x250 mm column, 30% EtOH / Peak 2 |
| 86 | 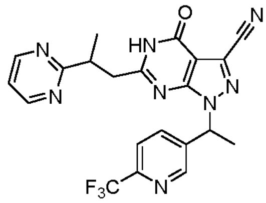 Isomer 3 | 4-oxo-6-(2-(pyrimidin-2-yl) propyl)-1-(1-(6-(trifluoromethyl)pyridin-3-yl)ethyl)-4,5-dihydro-1H-pyrazolo[3,4-d] pyrimidine-3-carbonitrile | 455.2 | AD-H, 21x250 mm column, 30% EtOH / Peak 3 |
| 87 | 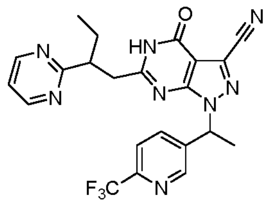 Isomer 1 | 4-oxo-6-(2-(pyrimidin-2-yl) butyl)-1-(1-(6-(trifluoromethyl)pyridin-3-yl)ethyl)-4,5-dihydro-1H-pyrazolo[3,4-d] pyrimidine-3-carbonitrile | 469.3 | 2 × AD-H, 21x250 mm column, 20% EtOH + 0.2% DIPA / Peak 1 |
| 88 | 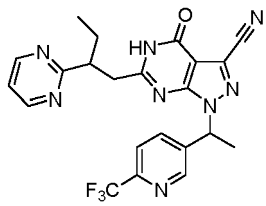 Isomer 2 | 4-oxo-6-(2-(pyrimidin-2-yl) butyl)-1-(1-(6-(trifluoromethyl)pyridin-3-yl)ethyl)-4,5-dihydro-1H-pyrazolo[3,4-d] pyrimidine-3-carbonitrile | 469.3 | 2 × AD-H, 21x250 mm column, 20% EtOH + 0.2% DIPA / Peak 2 |

(continued)

| The following compounds were prepared according to the above procedures herein using known or prepared starting materials, as described in the reaction schemes and examples herein. The requisite starting materials are either prepared as described in the intermediates section, commercially available, or may be prepared from commercially available reagents using conventional reactions well known in the art without undue experimentation. | | | | |
|---|---|---|---|---|
| Example | Structure | Name | LCMS $(M+H)^+$ | SFC / Peak# |
| 89 | 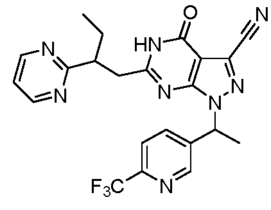 Isomer 3 | 4-oxo-6-(2-(pyrimidin-2-yl) butyl)- 1-(1-(6-(trifluoromethyl)p yridin-3-yl)ethyl)-4,5-dihydro-1H-pyrazolo[3,4-d] pyrimidine-3-carbonitrile | 469.3 | 2 × AD-H, 21x250 mm column, 20% EtOH + 0.2% DIPA / Peak 3 |
| 90 | 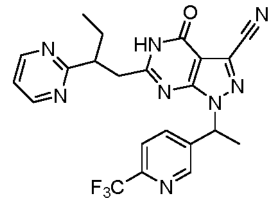 Isomer 4 | 4-oxo-6-(2-(pyrimidin-2-yl) butyl)- 1-(1-(6-(trifluoromethyl)p yridin-3-yl)ethyl)-4,5-dihydro-1H-pyrazolo[3,4-d] pyrimidine-3-carbonitrile | 469.3 | 2 × AD-H, 21x250 mm column, 20% EtOH + 0.2% DIPA / Peak 4 |

## TABLE 5

The following table shows representative data for the compounds of the Examples as PDE9 inhibitors as determined by the assays described herein. In this table, the PDE9 $K_i$ is a measure of the ability of the test compound to inhibit the action of the PDE9 enzyme. Such results are indicative of the intrinsic activity of the compounds for use as inhibitors of the PDE9 enzyme.

| Example | PDE9 IMAP Ki (nM) |
|---|---|
| 1 | 3.20 |
| 2 | 5.24 |
| 3 | 1.82 |
| 4 | 0.03 |
| 5 | 2.30 |
| 6 | 6.21 |

| Example | PDE9 IMAP Ki (nM) |
|---|---|
| 46 | 21.40 |
| 47 | 22.70 |
| 48 | 17.41 |
| 49 | 22.68 |
| 50 | 0.60 |
| 51 | 7.41 |

| | | | |
|---|---|---|---|
| 7 | 20.81 | 52 | 6.70 |
| 8 | 243.20 | 53 | 207.40 |
| 9 | 0.14 | 54 | 6.53 |
| 10 | 9.98 | 55 | 123.60 |
| 11 | 24.46 | 56 | 289.20 |
| 12 | 38.96 | 57 | 1.05 |
| 13 | 5.20 | 58 | 7.80 |
| 14 | 0.01 | 59 | 12.08 |
| 15 | 2.82 | 60 | 1.06 |
| 16 | 11.09 | 61 | 0.17 |
| 17 | 0.01 | 62 | 50.74 |
| 18 | 2.07 | 63 | 14.75 |
| 19 | 7.36 | 64 | 11.94 |
| 20 | 1.57 | 65 | 0.03 |
| 21 | 15.24 | 66 | 0.60 |
| 22 | 6.50 | 67 | 0.71 |
| 23 | 0.60 | 68 | 0.73 |
| 24 | 69.25 | 69 | 14.90 |
| 25 | 4.08 | 70 | 0.02 |
| 26 | 52.88 | 71 | 11.85 |
| 27 | 6.67 | 72 | 21.85 |
| 28 | 20.28 | 73 | 0.25 |
| 29 | 8.96 | 74 | 11.41 |
| 30 | 16.38 | 75 | 3.31 |
| 31 | 1.20 | 76 | 55.09 |
| 32 | 40.53 | 77 | 0.27 |
| 33 | 0.59 | 78 | 34.20 |
| 34 | 0.16 | 79 | 3.96 |
| 35 | 7.12 | 80 | 119.90 |
| 36 | 19.85 | 81 | 0.50 |
| 37 | 2.67 | 82 | 16.20 |
| 38 | 8.35 | 83 | 60.67 |
| 39 | 0.17 | 84 | 111.50 |

| | |
|---|---|
| 40 | 33.19 |
| 41 | 0.42 |
| 42 | 13.22 |
| 43 | 38.81 |
| 44 | 19.65 |
| 45 | 0.23 |

| | |
|---|---|
| 85 | 1.17 |
| 86 | 33.40 |
| 87 | 93.37 |
| 88 | 108.00 |
| 89 | 0.24 |
| 90 | 27.98 |

While the invention has been described and illustrated with reference to certain particular embodiments thereof, those skilled in the art will appreciate that various adaptations, changes, modifications, substitutions, deletions, or additions of procedures and protocols may be made without departing from the scope of the invention.

**Claims**

**1.** A compound of the formula I:

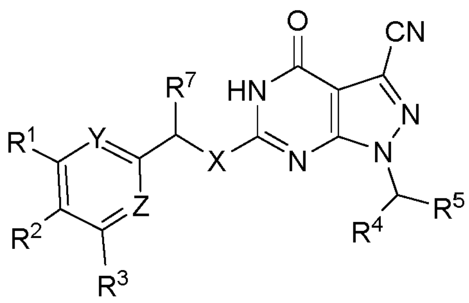

I

wherein:

X is $-N(R^6)-$ or $-C(R^8R^9)-$;
Y is -N= or -CH=;
Z is -N= or -CH=;
$R^1$, $R^2$ and $R^3$ are independently selected from:

(1) hydrogen,
(2) halogen,
(3) hydroxyl,
(4) $C_{1-6}$alkyl, which is unsubstituted or substituted with substituents selected from: hydroxy and fluoro,
(5) $-O-C_{1-6}$alkyl, which is unsubstituted or substituted with substituents selected from fluoro,
(6) $C_{3-6}$cycloalkyl,
(7) $C_{2-6}$alkynyl, and
(8) -CN;

$R^4$ is selected from:

(1) hydrogen,
(2) $-CH_3$;
(3) $-CF_3$,
(4) $-CH_2OH$,
(5) $-CO_2H$, and
(6) $-CH_2CH_3$;

$R^5$ is a phenyl, pyridyl, pyrazinyl, pyrazolyl, pyrimidinyl, pyridazinyl, thiazolyl, cyclohexyl or tetrahydropyranyl ring, wherein the phenyl, pyridyl, pyrazinyl, pyrazolyl, pyrimidinyl, pyridazinyl, thiazolyl, cyclohexyl or tetrahydropyranyl ring is substituted with $R^{1a}$, $R^{1b}$ and $R^{1c}$, wherein $R^{1a}$, $R^{1b}$ and $R^{1c}$ are independently selected from:

(1) hydrogen,
(2) halogen,
(3) hydroxyl,
(4) $C_{1-6}$alkyl, which is unsubstituted or substituted with substituents selected from: hydroxy and fluoro,
(5) -O-$C_{1-6}$alkyl, which is unsubstituted or substituted with substituents selected from fluoro,
(6) $C_{3-6}$cycloalkyl,
(7) azetidine, morpholine, piperidine, or pyrrolidine, and
(8) -CN;

$R^6$ is selected from:

(1) hydrogen, and
(2) $C_{1-6}$alkyl;

$R^7$ is selected from:

(1) hydrogen,
(2) $C_{1-6}$alkyl, which is unsubstituted or substituted with substituents selected from: hydroxy and fluoro,
(3) $C_{2-6}$alkenyl,
(4) $C_{3-6}$cycloalkyl, which is unsubstituted or substituted with substituents selected from: hydroxy and fluoro, and
(5) azetidine, morpholine, piperidine, or pyrrolidine;
or $R^6$ and $R^7$ and the -CH-N- to which they are attached are joined to form an azetidine or a pyrrolidine ring;

$R^8$ and $R^9$ are independently selected from:

(1) hydrogen, and
(2) $C_{1-6}$alkyl;

or a pharmaceutically acceptable salt thereof.

**2.** The compound of Claim 1 of the formula Ia:

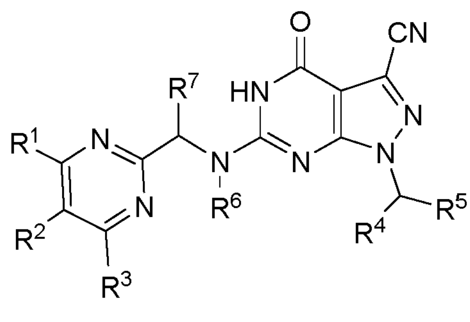

or a pharmaceutically acceptable salt thereof.

**3.** The compound of any of Claims 1-2, or a pharmaceutically acceptable salt thereof, wherein $R^1$, $R^2$ and $R^3$ are independently selected from:

(1) hydrogen,
(2) halogen,
(3) hydroxyl,
(4) $C_{1-6}$alkyl, which is unsubstituted or substituted with substituents selected from: hydroxy and fluoro,
(5) -O-$C_{1-6}$alkyl, which is unsubstituted or substituted with substituents selected from fluoro,

(6) $C_{3-6}$cycloalkyl,
(7) $C_{2-4}$alkynyl, and
(8) -CN.

**4.** The compound of any of Claims 1-3, or a pharmaceutically acceptable salt thereof, wherein $R^1$ is hydrogen, $R^3$ is hydrogen and $R^2$ is selected from:

(1) hydrogen,
(2) fluoro,
(3) chloro,
(4) bromo,
(5) hydroxyl,
(6) $-CH_3$,
(7) $-OCH_3$,
(8) $-CHF_2$,
(9) $-CF_3$,
(10) $-OCHF_2$,
(11) $-OCF_3$,
(12) $-CH_2CH_3$,
(13) $-CH(CH_3)_2$,
(14) $-C(CH_3)_3$,
(15) $-C{\equiv}CH$, and
(16) cyclopropyl.

**5.** The compound of any of Claims 1-4, or a pharmaceutically acceptable salt thereof, wherein $R^4$ is $-CH_3$.

**6.** The compound of any of Claims 1-5, or a pharmaceutically acceptable salt thereof, wherein $R^5$ is a phenyl, pyridyl or tetrahydropyranyl ring.

**7.** The compound of any of Claims 1-6, or a pharmaceutically acceptable salt thereof, wherein $R^{1b}$ is hydrogen and $R^{1c}$ is hydrogen, and $R^{1a}$ is selected from:

(1) hydrogen,
(2) fluoro,
(3) $-CH_3$,
(4) $-CHF_2$,
(5) $-CF_3$,
(6) $-OCHF_2$,
(7) $-OCF_3$, and
(8) -CN.

**8.** A compound of any of Claims 1-7 selected from:

1-(1-(6-cyclopropylpyridin-3-yl)ethyl)-6-(methyl(1-(pyrimidin-2-yl)propyl)amino)-4-oxo-4,5-dihydro-1H-pyrazolo[3,4-d]pyrimidine-3 -carbonitrile;
6-[methyl(1-pyrimidin-2-ylethyl)amino]-4-oxo-1-[1-[6-(trifluoromethyl)-3-pyridyl]ethyl]-5H-pyrazolo[3,4-d]pyrimidine-3-carbonitrile;
6-[methyl(1-pyrimidin-2-ylpropyl)amino]-4-oxo-1-[1 -[6-(trifluoromethyl)-3-pyridyl]ethyl]-5H-pyrazolo[3,4-d]pyrimidine-3-carbonitrile;
6-[methyl(1-pyrimidin-2-ylpropyl)amino]-4-oxo-1-[1 -[4-(trifluoromethyl)phenyl] ethyl]-5H-pyrazolo[3,4-d]pyrimidine-3-carbonitrile;
6-[1-[5-(difluoromethyl)pyrimidin-2-yl]propyl-methyl-amino]-4-oxo-1-[1-[6-(trifluoromethyl)-3-pyridyl]ethyl]-5H-pyrazolo[3,4-d]pyrimidine-3-carbonitrile;
6-[methyl-(2-methyl-1-pyrimidin-2-yl-propyl)amino]-4-oxo-1-[1-[6-(trifluoromethyl)-3-pyridyl]ethyl]-5H-pyrazolo[3,4-d]pyrimidine-3-carbonitrile;
6-[[cyclopropyl(pyrimidin-2-yl)methyl]-methyl-amino]-4-oxo-1-[1-[6-(trifluoromethyl)-3-pyridyl]ethyl]-5H-pyrazolo[3,4-d]pyrimidine-3-carbonitrile;
6-[[cyclobutyl(pyrimidin-2-yl)methyl]-methyl-amino]-4-oxo-1-[1-[6-(trifluoromethyl)-3-pyridyl]ethyl]-5H-pyrazolo[3,4-d]pyrimidine-3-carbonitrile;
6-[1-(5-fluoro-2-pyridyl)propyl-methyl-amino]-4-oxo-1-[1-[6-(trifluoromethyl)-3-pyridyl]ethyl]-SH-pyrazolo[3,4-d]pyrimidine-3-carbonitrile;
6-[1-(5-fluoropyrimidin-2-yl)propyl-methyl-amino]-4-oxo-1-(1-tetrahydropyran-4-ylethyl)-5H-pyrazolo[3,4-d]pyrimidine-3-carbonitrile;
6-[methyl(1-pyrimidin-2-ylpent-4-enyl)amino]-4-oxo-1-[1-[6-(trifluoromethyl)-3-pyridyl]ethyl]-5H-pyrazolo[3,4-d]pyrimidine-3-carbonitrile;
4-oxo-6-(2-pyrimidin-2-ylazetidin-1-yl)-1-[1-[6-(trifluoromethyl)-3-pyridyl]ethyl]-5H-pyrazolo[3,4-d]pyrimidine-3-carbonitrile;
6-[2-(4-fluorophenyl)azetidin-1-yl]-4-oxo-1-[1-[6-(trifluoromethyl)-3-pyridyl]ethyl]-5H-pyrazolo[3,4-d]pyrimidine-3-carbonitrile;
4-oxo-6-(2-pyrimidin-2-ylpyrrolidin-1-yl)-1-[1-[6-(trifluoromethyl)-3-pyridyl]ethyl]-5H-pyrazolo[3,4-d]pyrimidine-3-carbonitrile;
6-[2-(4-fluorophenyl)azetidin-1-yl]-4-oxo-1-[1-[6-(trifluoromethyl)-3-pyridyl]ethyl]-5H-pyrazolo[3,4-d]pyrimidine-3-carbonitrile;
6-[methyl-[1-[5-(trifluoromethoxy)pyrimidin-2-yl]propyl]amino]-4-oxo-1-[1-[6-(trifluoromethyl)-3-pyridyl]ethyl]-5H-pyrazolo[3,4-d]pyrimidine-3-carbonitrile;
1-[1-(6-cyclopropyl-3-pyridyl)ethyl]-6-[1-(5-fluoropyrimidin-2-yl)propyl-methyl-amino]-4-oxo-5H-pyrazolo[3,4-d]pyrimidine-3-carbonitrile;
1-[1-(4-cyclopropylphenyl)ethyl]-6-[1-(5-fluoropyrimidin-2-yl)propyl-methyl-amino]-4-oxo-5H-pyrazolo[3,4-d]pyrimidine-3-carbonitrile;
4-oxo-6-((1-(pyrimidin-2-yl)propyl)amino)-1-(1-(6-(trifluoromethyl)pyridin-3-yl)ethyl)-4,5-dihydro-1H-pyrazolo[3,4-d]pyrimidine-3-carbonitrile;
6-((1-(5-fluoropyrimidin-2-yl)propyl)(methyl)amino)-4-oxo-1-(1-(6-(trifluoromethyl)pyridin-3-yl)ethyl)-4,5-dihydro-1H-pyrazolo[3,4-d]pyrimidine-3-carbonitrile;
4-oxo-6-(2-(pyrimidin-2-yl)ethyl)-1-(1-(6-(trifluoromethyl)pyridin-3-yl)ethyl)-4,5-dihydro-1H-pyrazolo[3,4-d]pyrimidine-3-carbonitrile;
6-(2-(5-cyclopropylpyrimidin-2-yl)ethyl)-4-oxo-1-(1-(6-(trifluoromethyl)pyridin-3-yl)ethyl)-4,5-dihydro-1H-pyrazolo[3,4-d]pyrimidine-3-carbonitrile;
4-oxo-6-(2-(pyrimidin-2-yl)propyl)-1-(1-(6-(trifluoromethyl)pyridin-3-yl)ethyl)-4,5-dihydro-1H-pyrazolo[3,4-d]pyrimidine-3-carbonitrile; and
4-oxo-6-(2-(pyrimidin-2-yl)butyl)-1-(1-(6-(trifluoromethyl)pyridin-3-yl)ethyl)-4,5-dihydro-1H-pyrazolo[3,4-d]pyrimidine-3-carbonitrile;
or a pharmaceutically acceptable salt thereof.

**9.** A pharmaceutical composition which comprises a pharmaceutically acceptable carrier and a compound of any of Claims 1-8 or a pharmaceutically acceptable salt thereof.

**10.** A compound of any of Claims 1-8 or a pharmaceutically acceptable salt thereof for use in therapy.

**11.** A compound of any of Claims 1-8 or a pharmaceutically acceptable salt thereof for use in the treatment of cardiovascular disease, cerebrovascular disease, hypertension, chronic kidney disease, heart failure, psychotic disorders, delusional disorders, drug induced psychosis, anxiety disorders, movement disorders, mood disorders, or neurodegenerative disorders.

**Patentansprüche**

**1.** Eine Verbindung der Formel I:

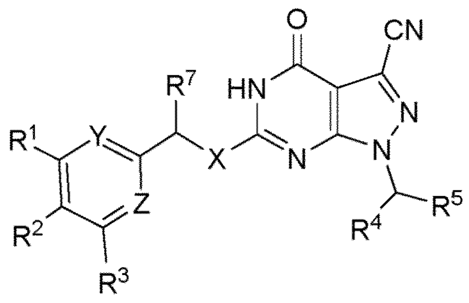

I,

wobei:

X-N($R^6$)- oder -C($R^8R^9$)- ist,
Y -N= oder -CH= ist,
Z -N= oder -CH= ist,
$R^1$, $R^2$ und $R^3$ unabhängig ausgewählt sind aus:

(1) Wasserstoff,
(2) Halogen,
(3) Hydroxyl,
(4) $C_{1-6}$-Alkyl, das unsubstituiert oder substituiert ist mit Substituenten, ausgewählt aus Hydroxy und Fluor,
(5) -O-$C_{1-6}$-Alkyl, das unsubstituiert oder substituiert ist mit Substituenten, ausgewählt aus Fluor,
(6) $C_{3-6}$-Cycloalkyl,
(7) $C_{2-6}$-Alkinyl und
(8) -CN,

$R^4$ ausgewählt ist aus:

(1) Wasserstoff,
(2) $-CH_3$,
(3) $-CF_3$,
(4) $-CH_2OH$,
(5) -COzH und
(6) $-CH_2CH_3$,

$R^5$ ein Phenyl-, Pyridyl-, Pyrazinyl-, Pyrazolyl-, Pyrimidinyl-, Pyridazinyl-, Thiazolyl-, Cyclohexyl-oder Tetrahydropyranylring ist, wobei der Phenyl-, Pyridyl-, Pyrazinyl-, Pyrazolyl-, Pyrimidinyl-, Pyridazinyl-, Thiazolyl-, Cyclohexyl- oder Tetrahydropyranylring substituiert ist mit $R^{1a}$, $R^{1b}$ und $R^{1c}$, wobei $R^{1a}$, $R^{1b}$ und $R^{1c}$ unabhängig ausgewählt sind aus:

(1) Wasserstoff,
(2) Halogen,
(3) Hydroxyl,
(4) $C_{1-6}$-Alkyl, das unsubstituiert oder substituiert ist mit Substituenten, ausgewählt aus: Hydroxy und Fluor,
(5) -O-$C_{1-6}$-Alkyl, das unsubstituiert oder substituiert ist mit Substituenten, ausgewählt aus Fluor,
(6) $C_{3-6}$-Cycloalkyl,
(7) Azetidin, Morpholin, Piperidin oder Pyrrolidin, und
(8) -CN,

$R^6$ ausgewählt ist aus:

(1) Wasserstoff und
(2) $C_{1-6}$-Alkyl,

$R^7$ ausgewählt ist aus:

(1) Wasserstoff,
(2) $C_{1-6}$-Alkyl, das unsubstituiert oder substituiert ist mit Substituenten, ausgewählt aus: Hydroxy und Fluor,
(3) $C_{2-6}$-Alkenyl,
(4) $C_{3-6}$-Cycloalkyl, das unsubstituiert oder substituiert ist mit Substituenten, ausgewählt aus: Hydroxy und Fluor, und
(5) Azetidin, Morpholin, Piperidin oder Pyrrolidin,
oder $R^6$ und $R^7$ und das -CH-N-, an das sie gebunden sind, verbunden sind unter Bildung eines Azetidin- oder eines Pyrrolidinrings,

$R^8$ und $R^9$ unabhängig ausgewählt sind aus:

(1) Wasserstoff und
(2) $C_{1-6}$-Alkyl,

oder ein pharmazeutisch annehmbares Salz davon.

2. Die Verbindung nach Anspruch 1 der Formel Ia:

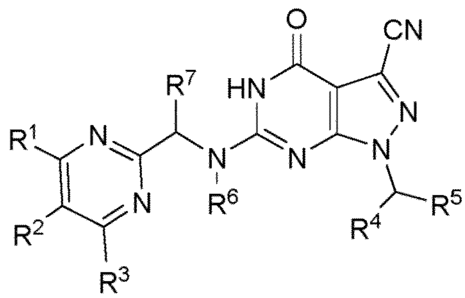

oder ein pharmazeutisch annehmbares Salz davon.

3. Die Verbindung nach einem der Ansprüche 1-2 oder ein pharmazeutisch annehmbares Salz davon, wobei $R^1$, $R^2$ und $R^3$ unabhängig ausgewählt sind aus:

(1) Wasserstoff,
(2) Halogen,
(3) Hydroxyl,
(4) $C_{1-6}$-Alkyl, das unsubstituiert oder substituiert ist mit Substituenten, ausgewählt aus: Hydroxy und Fluor,
(5) -O-$C_{1-6}$-Alkyl, das unsubstituiert oder substituiert ist mit Substituenten, ausgewählt aus Fluor,
(6) $C_{3-6}$-Cycloalkyl,
(7) $C_{2-4}$-Alkinyl und
(8) -CN.

4. Die Verbindung nach einem der Ansprüche 1-3 oder ein pharmazeutisch annehmbares Salz davon, wobei $R^1$ Wasserstoff ist, $R^3$ Wasserstoff ist und $R^2$ ausgewählt ist aus:

(1) Wasserstoff,
(2) Fluor,
(3) Chlor,
(4) Brom,
(5) Hydroxyl
(6) $-CH_3$,
(7) $-OCH_3$,
(8) $-CHF_2$,
(9) $-CF_3$,
(10) $-OCHF_2$,
(11) $-OCF_3$,
(12) $-CH_2CH_3$,

(13) $-CH(CH_3)_2$,
(14) $-C(CH_3)_3$,
(15) -C=CH und
(16) Cyclopropyl.

**5.** Die Verbindung nach einem der Ansprüche 1-4 oder ein pharmazeutisch annehmbares Salz davon, wobei $R^4$ $-CH_3$ ist.

**6.** Die Verbindung nach einem der Ansprüche 1-5 oder ein pharmazeutisch annehmbares Salz davon, wobei $R^5$ ein Phenyl-, Pyridyl- oder Tetrahydropyranylring ist.

**7.** Die Verbindung nach einem der Ansprüche 1-6 oder ein pharmazeutisch annehmbares Salz davon, wobei $R^{1b}$ Wasserstoff ist und $R^{1c}$ Wasserstoff ist, und $R^{1a}$ ausgewählt ist aus:

(1) Wasserstoff,
(2) Fluor,
(3) $-CH_3$,
(4) $-CHF_2$,
(5) $-CF_3$,
(6) $-OCHF_2$,
(7) $-OCF_3$ und
(8) -CN.

**8.** Eine Verbindung nach einem der Ansprüche 1-7, ausgewählt aus:

1-(1-(6-Cyclopropylpyridin-3-yl)ethyl)-6-(methyl(1-(pyrimidin-2-yl)propyl)amino)-4-oxo-4,5-dihydro-1H-pyrazolo[3,4-d]pyrimidin-3-carbonitril,
6-[Methyl(1-pyrimidin-2-ylethyl)amino]-4-oxo-1-[1-[6-(trifluormethyl)-3-pyridyl]ethyl]-5H-pyrazolo[3,4-d]pyrimidin-3-carbonitril,
6-[Methyl(1-pyrimidin-2-ylpropyl)amino]-4-oxo-1-[1-[6-(trifluormethyl)-3-pyridyl]ethyl]-5H-pyrazolo[3,4-d]pyrimidin-3-carbonitril,
6-[Methyl(1-pyrimidin-2-ylpropyl)amino]-4-oxo-1-[1-[4-(trifluormethyl)phenyl]ethyl]-5H-pyrazolo[3,4-d]pyrimidin-3-carbonitril,
6-[1-[5-(Difluormethyl)pyrimidin-2-yl]propylmethylamino]-4-oxo-1-[1-[6-(trifluormethyl)-3-pyridyl]ethyl]-5H-pyrazolo[3,4-d]pyrimidin-3-carbonitril,
6-[Methyl-(2-methyl-1-pyrimidin-2-ylpropyl)amino]-4-oxo-1-[1-[6-(trifluormethyl)-3-pyridyl]ethyl]-5H-pyrazolo[3,4-d]pyrimidin-3-carbonitril,
6-[[Cyclopropyl(pyrimidin-2-yl)methyl]methylamino]-4-oxo-1-[1-[6-(trifluormethyl)-3-pyridyl]ethyl]-5H-pyrazolo[3,4-d]pyrimidin-3-carbonitril,
6-[[Cyclobutyl(pyrimidin-2-yl)methyl]methylamino]-4-oxo-1-[1-[6-(trifluormethyl)-3-pyridyl]ethyl]-5H-pyrazolo[3,4-d]pyrimidin-3-carbonitril,
6-[1-(5-Fluor-2-pyridyl)propylmethylamino]-4-oxo-1-[1-[6-(trifluormethyl)-3-pyridyl]ethyl]-5H-pyrazolo[3,4-d]pyrimidin-3-carbonitril,
6-[1-(5-Fluorpyrimidin-2-yl)propylmethylamino]-4-oxo-1-(1-tetrahydropyran-4-ylethyl)-5H-pyrazolo[3,4-d]pyrimidin-3-carbonitril,
6-[Methyl(1-pyrimidin-2-ylpent-4-enyl)amino]-4-oxo-1-[1-[6-(trifluormethyl)-3-pyridyl]ethyl]-5H-pyrazolo[3,4-d]pyrimidin-3-carbonitril,
4-Oxo-6-(2-pyrimidin-2-ylazetidin-1-yl)-1-[1-[6-(trifluormethyl)-3-pyridyl]ethyl]-5H-pyrazolo[3,4-d]pyrimidin-3-carbonitril,
6-[2-(4-Fluorphenyl)azetidin-1-yl]-4-oxo-1-[1-[6-(trifluormethyl)-3-pyridyl]ethyl]-5H-pyrazolo[3,4-d]pyrimidin-3-carbonitril,
4-Oxo-6-(2-pyrimidin-2-ylpyrrolidin-1-yl)-1-[1-[6-(trifluormethyl)-3-pyridyl]ethyl]-5H-pyrazolo[3,4-d]pyrimidin-3-carbonitril,
6-[2-(4-Fluorphenyl)azetidin-1-yl]-4-oxo-1-[1-[6-(trifluormethyl)-3-pyridyl]ethyl]-5H-pyrazolo[3,4-d]pyrimidin-3-carbonitril,
6-[Methyl-[1-[5-(trifluormethoxy)pyrimidin-2-yl]propyl]amino]-4-oxo-1-[1-[6-(trifluormethyl)-3-pyridyl]ethyl]-5H-pyrazolo[3,4-d]pyrimidin-3-carbonitril,
1-[1-(6-Cyclopropyl-3-pyridyl)ethyl]-6-[1-(5-fluorpyrimidin-2-yl)propylmethylamino]-4-oxo-5H-pyrazolo[3,4-d]pyrimidin-3-carbonitril,

1-[1-(4-Cyclopropylphenyl)ethyl]-6-[1-(5-fluorpyrimidin-2-yl)propylmethylamino]-4-oxo-5H-pyrazolo[3,4-d]pyrimidin-3-carbonitril,
4-Oxo-6-((1-(pyrimidin-2-yl)propyl)amino)-1-(1-(6-(trifluormethyl)pyridin-3-yl)ethyl)-4,5-dihydro-1H-pyrazolo[3,4-d]pyrimidin-3-carbonitril,
6-((1-(5-Fluorpyrimidin-2-yl)propyl)(methyl)amino)-4-oxo-1-(1-(6-(trifluormethyl)pyridin-3-yl)ethyl)-4,5-dihydro-1H-pyrazolo[3,4-d]pyrimidin-3-carbonitril,
4-Oxo-6-(2-(pyrimidin-2-yl)ethyl)-1-(1-(6-(trifluormethyl)pyridin-3-yl)ethyl)-4,5-dihydro-1H-pyrazolo[3,4-d]pyrimidin-3-carbonitril,
6-(2-(5-Cyclopropylpyrimidin-2-yl)ethyl)-4-oxo-1-(1-(6-(trifluormethyl)pyridin-3-yl)ethyl)-4,5-dihydro-1H-pyrazolo[3,4-d]pyrimidin-3-carbonitril,
4-Oxo-6-(2-(pyrimidin-2-yl)propyl)-1-(1-(6-(trifluormethyl)pyridin-3-yl)ethyl)-4,5-dihydro-1H-pyrazolo[3,4-d]pyrimidin-3-carbonitril, und
4-Oxo-6-(2-(pyrimidin-2-yl)butyl)-1-(1-(6-(trifluormethyl)pyridin-3-yl)ethyl)-4,5-dihydro-1H-pyrazolo[3,4-d]pyrimidin-3-carbonitril,
oder ein pharmazeutisch annehmbares Salz davon.

**9.** Eine pharmazeutische Zusammensetzung, die einen pharmazeutisch annehmbaren Träger und eine Verbindung nach einem der Ansprüche 1-8 oder ein pharmazeutisch annehmbares Salz davon umfasst.

**10.** Eine Verbindung nach einem der Ansprüche 1-8 oder ein pharmazeutisch annehmbares Salz davon zur Verwendung in der Therapie.

**11.** Eine Verbindung nach einem der Ansprüche 1-8 oder ein pharmazeutisch annehmbares Salz davon zur Verwendung bei der Behandlung einer kardiovaskulären Erkrankung, zerebrovaskulären Erkrankung, Hypertension, chronischen Nierenerkrankung, von Herzversagen, psychotischen Störungen, wahnhaften Störungen, medikamenteninduzierter Psychose, Angststörungen, Bewegungsstörungen, Gemütsstörungen oder neurodegenerativen Störungen.

## Revendications

**1.** Composé de formule I :

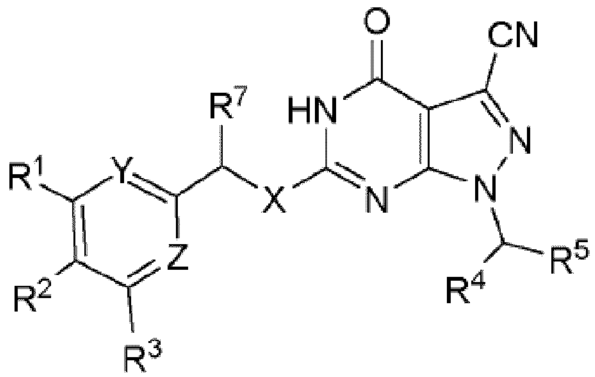

I

dans lequel :

X est -N($R^6$)- ou -C($R^8R^9$)- ;
Y est -N= ou -CH= ;
Z est -N= ou -CH= ;
$R^1$, $R^2$ et $R^3$ sont indépendamment choisis parmi :

(1) hydrogène,
(2) halogène,
(3) hydroxyle,
(4) $C_{1-6}$ alkyle, qui est non substitué ou substitué par des substituants choisis parmi : hydroxy et fluoro,

(5) -O-$C_{1-6}$ alkyle, qui est non substitué ou substitué par des substituants choisis parmi : fluoro,
(6) $C_{3-6}$ cycloalkyle,
(7) $C_{2-6}$ alcynyle, et
(8) -CN ;

$R^4$ est choisi parmi :

(1) hydrogène,
(2) $-CH_3$,
(3) $-CF_3$,
(4) $-CH_2OH$,
(5) $-CO_2H$, et
(6) $-CH_2CH_3$ ;

$R^5$ est un cycle phényle, pyridyle, pyrazinyle, pyrazolyle, pyrimidinyle, pyridazinyle, thiazolyle, cyclohexyle ou tétrahydropyranyle, dans lequel le cycle phényle, pyridyle, pyrazinyle, pyrazolyle, pyrimidinyle, pyridazinyle, thiazolyle, cyclohexyle ou tétrahydropyranyle est substitué par $R^{1a}$, $R^{1b}$ et $R^{1c}$, dans lequel $R^{1a}$, $R^{1b}$ et $R^{1c}$ sont indépendamment choisis parmi :

(1) hydrogène,
(2) halogène,
(3) hydroxyle,
(4) $C_{1-6}$ alkyle, qui est non substitué ou substitué par des substituants choisis parmi : hydroxy et fluoro,
(5) -O-$C_{1-6}$ alkyle, qui est non substitué ou substitué par des substituants choisis parmi : fluoro,
(6) $C_{3-6}$ cycloalkyle,
(7) azétidine, morpholine, pipéridine ou pyrrolidine, et
(8) -CN ;

$R^6$ est choisi parmi :

(1) hydrogène, et
(2) $C_{1-6}$ alkyle ;

$R^7$ est choisi parmi :

(1) hydrogène,
(2) $C_{1-6}$ alkyle, qui est non substitué ou substitué par des substituants choisis parmi : hydroxy et fluoro,
(3) $C_{2-6}$ alcényle,
(4) $C_{3-6}$ cycloalkyle, qui est non substitué ou substitué par des substituants choisis parmi : hydroxy et fluoro, et
(5) azétidine, morpholine, pipéridine ou pyrrolidine ;

ou $R^6$ et $R^7$ et -CH-N- auquel ils sont attachés sont joints pour former un cycle azétidine ou pyrrolidine ;
$R^8$ et $R^9$ sont indépendamment choisis parmi :

(1) hydrogène, et
(2) $C_{1-6}$ alkyle ;

ou un sel pharmaceutiquement acceptable de celui-ci.

**2.** Composé selon la revendication 1 de formule Ia :

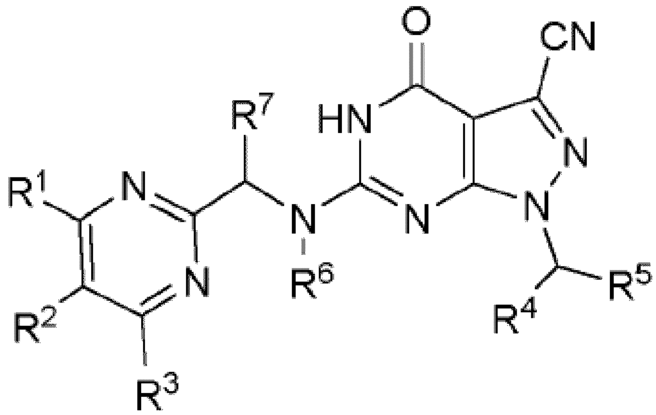

ou sel pharmaceutiquement acceptable de celui-ci.

**3.** Composé selon l'une quelconque des revendications 1-2, ou sel pharmaceutiquement acceptable de celui-ci, dans lequel $R^1$, $R^2$ et $R^3$ sont indépendamment choisis parmi :

(1) hydrogène,
(2) halogène,
(3) hydroxyle,
(4) $C_{1-6}$ alkyle, qui est non substitué ou substitué par des substituants choisis parmi : hydroxy et fluoro,
(5) $-O-C_{1-6}$ alkyle, qui est non substitué ou substitué par des substituants choisis parmi : fluoro,
(6) $C_{3-6}$ cycloalkyle,
(7) $C_{2-4}$ alcynyle, et
(8) -CN.

**4.** Composé selon l'une quelconque des revendications 1-3, ou sel pharmaceutiquement acceptable de celui-ci, dans lequel $R^1$ est un hydrogène, $R^3$ est un hydrogène et $R^2$ est choisi parmi :

(1) hydrogène,
(2) fluoro,
(3) chloro,
(4) bromo,
(5) hydroxyle,
(6) $-CH_3$,
(7) $-OCH_3$,
(8) $-CHF_2$,
(9) $-CF_3$,
(10) $-OCHF_2$,
(11) $-OCF_3$,
(12) $-CH_2CH_3$,
(13) $-CH(CH_3)_2$,
(14) $-C(CH_3)_3$,
(15) $-C{\equiv}CH$, et
(16) cyclopropyle.

**5.** Composé selon l'une quelconque des revendications 1-4, ou sel pharmaceutiquement acceptable de celui-ci, dans lequel $R^4$ est $-CH_3$.

**6.** Composé selon l'une quelconque des revendications 1-5, ou sel pharmaceutiquement acceptable de celui-ci, dans lequel $R^5$ est un cycle phényle, pyridyle ou tétrahydropyranyle.

**7.** Composé selon l'une quelconque des revendications 1-6, ou sel pharmaceutiquement acceptable de celui-ci, dans lequel $R^{1b}$ est un hydrogène et $R^{1c}$ est un hydrogène et $R^{1a}$ est choisi parmi :

(1) hydrogène,
(2) fluoro,
(3) $-CH_3$,
(4) $-CHF_2$,
(5) $-CF_3$,
(6) $-OCHF_2$,
(7) $-OCF_3$, et
(8) -CN.

**8.** Composé selon l'une quelconque des revendications 1-7 choisi parmi :

1-(1-(6-cyclopropylpyridin-3-yl)éthyl)-6-(méthyl(1-(pyrimidin-2-yl)propyl)amino)-4-oxo-4,5-dihydro-1H-pyrazolo[3,4-d]pyrimidine-3-carbonitrile ;
6-[méthyl(1-pyrimidin-2-yléthyl)amino]-4-oxo-1-[1-[6-(trifluorométhyl)-3-pyridyl]éthyl]-5H-pyrazolo[3,4-d]pyrimidine-3-carbonitrile ;
6-[méthyl(1-pyrimidin-2-ylpropyl)amino]-4-oxo-1-[1-[6-(trifluorométhyl)-3-pyridyl]éthyl]-5H-pyrazolo[3,4-d]pyrimidine-3-carbonitrile ;
6-[méthyl(1-pyrimidin-2-ylpropyl)amino]-4-oxo-1-[1-[4-(trifluorométhyl)phényl]éthyl]-5H-pyrazolo[3,4-d]pyrimidine-3-carbonitrile ;
6-[1-[5-(difluorométhyl)pyrimidin-2-yl]propyl-méthyl-amino]-4-oxo-1-[1-[6-(trifluorométhyl)-3-pyridyl]éthyl]-5H-pyrazolo[3,4-d]pyrimidine-3-carbonitrile ;
6-[méthyl(2-méthyl-1-pyrimidin-2-yl-propyl)amino]-4-oxo-1-[1-[6-(trifluorométhyl)-3-pyridyl]éthyl]-5H-pyrazolo[3,4-d]pyrimidine-3-carbonitrile ;
6-[[cyclopropyl(pyrimidin-2-yl)méthyl]-méthyl-amino]-4-oxo-1-[1-[6-(trifluorométhyl)-3-pyridyl]éthyl]-5H-pyrazolo[3,4-d]pyrimidine-3-carbonitrile ;
6-[[cyclobutyl(pyrimidin-2-yl)méthyl]-méthyl-amino]-4-oxo-1-[1-[6-(trifluorométhyl)-3-pyridyl]éthyl]-5H-pyrazolo[3,4-d]pyrimidine-3-carbonitrile ;
6-[1-(5-fluoro-2-pyridyl)propyl-méthyl-amino]-4-oxo-1-[1-[6-(trifluorométhyl)-3-pyridyl]éthyl]-5H-pyrazolo[3,4-d]pyrimidine-3-carbonitrile ;
6-[1-(5-fluoropyrimidin-2-yl)propyl-méthyl-amino]-4-oxo-1-(1-tétrahydropyran-4-yléthyl)-5H-pyrazolo[3,4-d]pyrimidine-3-carbonitrile ;
6-[méthyl(1-pyrimidin-2-ylpent-4-ényl)amino]-4-oxo-1-[1-[6-(trifluorométhyl)-3-pyridyl]éthyl]-5H-pyrazolo[3,4-d]pyrimidine-3-carbonitrile ;
4-oxo-6-(2-pyrimidin-2-ylazétidin-1-yl)-1-[1-[6-(trifluorométhyl)-3-pyridyl]éthyl]-5H-pyrazolo[3,4-d]pyrimidine-3-carbonitrile ;
6-[2-(4-fluorophényl)azétidin-1-yl]-4-oxo-1-[1-[6-(trifluorométhyl)-3-pyridyl]éthyl]-5H-pyrazolo[3,4-d]pyrimidine-3-carbonitrile ;
4-oxo-6-(2-pyrimidin-2-ylpyrrolidin-1-yl)-1-[1-[6-(trifluorométhyl)-3-pyridyl]éthyl]-5H-pyrazolo[3,4-d]pyrimidine-3-carbonitrile ;
6-[2-(4-fluorophényl)azétidin-1-yl]-4-oxo-1-[1-[6-(trifluorométhyl)-3-pyridyl]éthyl]-5H-pyrazolo[3,4-d]pyrimidine-3-carbonitrile ;
6-[méthyl-[1-[5-(trifluorométhoxy)pyrimidin-2-yl]propyl]amino]-4-oxo-1-[1-[6-(trifluorométhyl)-3-pyridyl]éthyl]-5H-pyrazolo[3,4-d]pyrimidine-3-carbonitrile ;
1-[1-(6-cyclopropyl-3-pyridyl)éthyl]-6-[1-(5-fluoropyrimidin-2-yl)propyl-méthyl-amino]-4-oxo-5H-pyrazolo[3,4-d]pyrimidine-3-carbonitrile ;
1-[1-(4-cyclopropylphényl)éthyl]-6-[1-(5-fluoropyrimidin-2-yl)propyl-méthyl-amino]-4-oxo-5H-pyrazolo[3,4-d]pyrimidine-3-carbonitrile ;
4-oxo-6-((1-pyrimidin-2-yl)propyl)amino)-1-(1-(6-(trifluorométhyl)pyridin-3-yl)éthyl)-4,5-dihydro-1H-pyrazolo[3,4-d]pyrimidine-3-carbonitrile ;
6-((1-(5-fluoropyrimidin-2-yl)propyl)(méthyl)amino)-4-oxo-1-(1-(6-(trifluorométhyl)pyridin-3-yl)éthyl)-4,5-dihydro-1H-pyrazolo[3,4-d]pyrimidine-3-carbonitrile ;
4-oxo-6-(2-(pyrimidin-2-yl)éthyl)-1-(1-(6-(trifluorométhyl)pyridin-3-yl)éthyl)-4,5-dihydro-1H-pyrazolo[3,4-d]pyrimidine-3-carbonitrile ;
6-(2-(5-cyclopropylpyrimidin-2-yl)éthyl)-4-oxo-1-(1-(6-(trifluorométhyl)pyridin-3-yl)éthyl)-4,5-dihydro-1H-pyrazolo[3,4-d]pyrimidine-3-carbonitrile ;
4-oxo-6-(2-(pyrimidin-2-yl)propyl)-1-(1-(6-(trifluorométhyl)pyridin-3-yl)éthyl)-4,5-dihydro-1H-pyrazolo[3,4-d]pyrimidine-3-carbonitrile ; et
4-oxo-6-(2-(pyrimidin-2-yl)butyl)-1-(1-(6-(trifluorométhyl)pyridin-3-yl)éthyl)-4,5-dihydro-1H-pyrazolo[3,4-d]pyrimidine-3-carbonitrile ;
ou un sel pharmaceutiquement acceptable de ceux-ci.

**9.** Composition pharmaceutique qui comprend un véhicule pharmaceutiquement acceptable et un composé selon l'une quelconque des revendications 1-8 ou un sel pharmaceutiquement acceptable de celui-ci.

**10.** Composé selon l'une quelconque des revendications 1-8 ou sel pharmaceutiquement acceptable de celui-ci pour utilisation en thérapie.

**11.** Composé selon l'une quelconque des revendications 1-8 ou sel pharmaceutiquement acceptable de celui-ci pour utilisation dans le traitement d'une maladie cardiovasculaire, d'une maladie cérébrovasculaire, d'une hypertension, d'une maladie rénale chronique, d'une insuffisance cardiaque, de troubles psychotiques, de troubles délirants, d'une psychose induite par des médicaments, de troubles d'anxiété, de troubles moteurs, de troubles de l'humeur ou de troubles neurodégénératifs.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2018134712 A1
- WO 2016196071 A1
- WO 2016116752 A
- WO 2014120800 A
- US 20150284341 A


### Non-patent literature cited in the description


- **HUANG, W. et al.** *J. Biomol Screen,* 2002, vol. 7, 215
- **WUNDER et al.** *Mol. Pharmacol.,* 2005, vol. 68 (6), 1775-81
- **WAGER et al.** *ACS Chemical Neuroscience,* 2010, vol. 1, 435-449
- **MOSSER et al.** *JALA,* 2003, vol. 8, 54-63
- *Russian Journal of Organic Chemistry,* 2016, vol. 52 (4), 503-512